(19) Europäisches Patentamt
European Patent Office
Office européen des brevets

(11) EP 3 033 433 B1

(12) # EUROPEAN PATENT SPECIFICATION

(45) Date of publication and mention of the grant of the patent:
**10.10.2018 Bulletin 2018/41**

(21) Application number: **14752600.8**

(22) Date of filing: **12.08.2014**

(51) Int Cl.:
***C12Q 1/6888*** *(2018.01)*

(86) International application number:
**PCT/EP2014/067224**

(87) International publication number:
**WO 2015/022314 (19.02.2015 Gazette 2015/07)**

(54) **DETECTION OF ORGANOPHOSPHATE RESISTANCE IN CRUSTACEANS**

NACHWEIS VON PHOSPHORHALTIGEM ORGANISCHEM WIDERSTAND IN SCHALENTIEREN

DÉTECTION D'UNE RÉSISTANCE À UN ORGANOPHOSPHATE CHEZ LES CRUSTACÉS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.08.2013 NO 20131092**

(43) Date of publication of application:
**22.06.2016 Bulletin 2016/25**

(73) Proprietors:
- **PATOGEN AS**
  **6001 Ålesund (NO)**
- **Norges Miljø - OG Biovitenskapelige Universitet (NMBU)**
  **1433 Ås (NO)**

(72) Inventors:
- **KAUR, Kiranpreet**
  **0670 Oslo (NO)**
- **HORSBERG, Tor Einar**
  **1279 Oslo (NO)**

(74) Representative: **Onsagers AS**
**Munkedamsveien 35**
**P.O. Box 1813 Vika**
**0123 Oslo (NO)**

(56) References cited:
**WO-A1-2013/173598 WO-A2-03/085110**

- **ANDERS FALLANG ET AL: "Evidence for occurrence of an organophosphate-resistant type of acetylcholinesterase in strains of sea lice (Lepeophtheirus salmonis Kr&#xFFFD;yer)", PEST MANAGEMENT SCIENCE, vol. 60, no. 12, 1 January 2004 (2004-01-01), pages 1163-1170, XP055153288, ISSN: 1526-498X, DOI: 10.1002/ps.932**
- **FOURNIER D ET AL: "Drosophila acetylcholinesterase: Mechanisms of resistance to organophosphates", CHEMICO-BIOLOGICAL INTERACTIONS, ELSEVIER SCIENCE IRLAND, IR, vol. 87, no. 1-3, 1 June 1993 (1993-06-01) , pages 233-238, XP025554813, ISSN: 0009-2797, DOI: 10.1016/0009-2797(93)90047-3 [retrieved on 1993-06-01]**
- **STEPHEN N CARMICHAEL ET AL: "Salmon lice (Lepeophtheirus salmonis) showing varying emamectin benzoate susceptibilities differ in neuronal acetylcholine receptor and GABA-gated chloride channel mRNA expression", BMC GENOMICS, BIOMED CENTRAL LTD, LONDON, UK, vol. 14, no. 1, 18 June 2013 (2013-06-18), page 408, XP021152939, ISSN: 1471-2164, DOI: 10.1186/1471-2164-14-408**
- **MIKE A OSTA ET AL: "Insecticide resistance to organophosphates in Culex pipiens complex from Lebanon", PARASITES & VECTORS, BIOMED CENTRAL LTD, LONDON UK, vol. 5, no. 1, 3 July 2012 (2012-07-03), page 132, XP021133721, ISSN: 1756-3305, DOI: 10.1186/1756-3305-5-132**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

SEQUENCE LISTING

<110> PatoGen AS og Norges veterinzerhogskole

<120> Detection of organophosphate resistance in crustaceans

<130> P23478PC00

<160> 15

<170> PatentIn version 3.5

<210> 1
<211> 1908
<212> DNA
<213> Lepeophteirus salmonis

<400> 1

```
atgtggattc aagtcagaaa acaaaacttt ggtctttact ttaataagat attagtgtat      60
ttgctcactt tgtcatggag cgtgggtgct atcgtacaag acaatttggt gatcaccaca     120
aaaaaaggaa agatccgagg tgttactctt aaatctgcaa caaataggga agtagatgca     180
tggtatggga ttccatacgc acaacctcca gtgggtaatc ttcgatttcg tcaccctaga     240
cacattgatg cctgggaggg gattaaagaa acgacccgac ctccaaattc ttgtattcaa     300
gtagttgata cattatttcc aggctttgaa ggcgcagaaa tgtggaatgt gaatacagag     360
cccagcgagg actgtcttta tttaagtgtc catgtcccta aaccccgtcc tacaggatca     420
gctgttctgg tatggatcta cggtggagga ttttattctg gaacttctac tctggaagtc     480
tatgatccac gagttcttgt gtcagaagaa aacataatct tcgttgccat gcaatatcgt     540
gttgcaagtt tagggttcct tttctttgat acggaggatg ttcctggaaa tgcgggattg     600
tatgatcaaa tgatggctct ccaatgggta aagaacaata tagaggaatt tggtggtgat     660
cctgataaaa tcaccatttt cggagagtcc gccggtggtt gctccgtagc ccttcacctc     720
ctttccccac tctcaaggaa cctgttttct caagccatca tgcagagtgc ttcagctctt     780
gttccatggg gagtcatcac aaaaaaagaa agtattattc gtggtcggag gcttgcggaa     840
atgatgagct gtccttacga tgaaaaaaat acaaaggcca tgattgaatg cctgcggcaa     900
aaggatgcaa cagagatggt taatcaggag tggattggta tcatttctgg gattgcagag     960
ttccctttttg ttcctattgt ggatggaagt ttcttggatg agagccctgg aaaatctctt    1020
acaacaaaga actataaaaa aaccaacatt ttaattggag cgaataagga agaagggaat    1080
tatttcatca tgtactatct tacagatctc tttaaaaata cggagagcgt ctatgttgat    1140
cgaacagatt tcattcgaag tgtttcagaa ttgaaccact atgtaaaaaa aatgggaaga    1200
gaggcaataa cttttgagta cacagattgg ctcaatccaa acgatcccat aaaaaataga    1260
gaagcaattg atcgcatggt cggtgactat caattcattt gcccaactgc tgactttgct    1320
cgtatttatg ctagtacagg aaataatgta tacatgtact atttcactga gcgatcttcc    1380
```

actagcccat ggccaacatg gtctggtgta cttcatggcg atgaaattgc ttttgttttt 1440

ggagaggccc ttaataagtc aaagaattat gataagtcag aaattgccct atcaaaaaga 1500

atgatgggat attgggctaa ttttgctaaa actgggaatc cgagtctttc tgctgatgga 1560

acttggagca caaattattg gcctttacat acgccgacaa aacaggaggt acttgaatta 1620

aacgccaatt attctcgagt tctcgagggt cttcgagtta aaaaatgtgc cttttggaaa 1680

aaatatctac ctaaactttt atcattaact tcaaacaatg gagaccatca gtgtcaaaca 1740

tcctcgacat catctccctc atgctgtaaa gatggaacat gctgcaacag tgggacgtcc 1800

tttgcatcca cgttcttttc agtattcatt gtaacaacgt cggtgcatct attttataat 1860

tttaaattta gtgtaattta ctcaaatatt aataatggaa aactgtga 1908

<210> 2
<211> 1908
<212> DNA
<213> Lepeophteirus salmonis

<400> 2

```
atgtggattc aagtcagaaa acaaaacttt ggtctttact ttaataagat attagtgtat      60
ttgctcactt tgtcatggag cgtgggtgct atcgtacaag acaatttggt gatcaccaca     120
aaaaaaggaa agatccgagg tgttactctt aaatctgcaa caaataggga agtagatgca     180
tggtatggga ttccatacgc acaacctcca gtgggtaatc ttcgatttcg tcaccctagg     240
cacattgatg cctgggaggg gattaaagaa acgacccgac ctccaaattc ttgtattcaa     300
gtagttgata cattatttcc aggctttgaa ggcgcagaaa tgtggaatgt gaatacagag     360
cccagcgagg actgtcttta tttaagtgtc catgtcccta aaccccgtcc tacaggatca     420
gctgttctgg tatggatcta cggtggagga ttttattctg gaacttctac tctggaagtc     480
tatgatccac gagttcttgt gtcagaagaa aacataatct tcgttgccat gcaatatcgt     540
gttgcaagtt tagggttcct tttctttgat acggaggatg ttcctggaaa tgcgggattg     600
tatgatcaaa tgatggctct ccaatgggta aagaacaata tagaggaatt tggtggtgat     660
cctgataaaa tcaccatttt cggagagtcc gccggtggtt gctcggtagc ccttcacctc     720
ctttccccac tctcaaggaa cctgttttct caagccatca tgcagagtgc ttcagctctt     780
gttccatggg gagtcatcac aaaaaaagaa agtattattc gtggtcggag gcttgcggaa     840
atgatgagct gtccttacga tgaaaaaaat acaaaggcca tgattgaatg cctgcggcaa     900
aaggatgcaa cagagatggt taatcaggag tggattggta tcatttctgg gattgcagag     960
ttcccttttg ttcctattgt ggatggaagt ttcttggatg agagccctgg aaaatctctt    1020
acaacaaaga actataaaaa aaccaacatt ttaattggag cgaataagga agaagggaat    1080
tattacatca tgtactatct tacagatctc tttaaaaata cggagagcgt ctatgttgat    1140
cgaacagatt tcattcgaag tgtttcagaa ttgaaccact atgtaaaaaa aatgggaaga    1200
```

gaggcaataa cttttgagta cacagattgg ctcaatccaa acgatcccat aaaaaataga 1260

gaagcaattg atcgcatggt cggtgactat caattcattt gcccaactgc tgactttgct 1320

cgtatttatg ctagtacagg aaataatgta tacatgtact atttcactga gcgatcttcc 1380

actagcccat ggccaacatg gtctggtgta cttcatggcg atgaaattgc ttttgttttt 1440

ggagaggccc ttaataagtc aaagaattat gataagtcag aaattgccct atcaaaaaga 1500

atgatgggat attgggctaa ttttgctaaa actgggaatc cgagtctttc tgctgatgga 1560

acttggagca caaattattg gcctttacat acgccgacaa aacaggaggt acttgaatta 1620

aacgccaatt attctcgagt tctcgagggt cttcgagtta aaaaatgtgc cttttggaaa 1680

aaatatctac ctaaactttt atcattaact tcaaacaatg gagaccatca gtgtcaaaca 1740

tcctcgacat catctccctc atgctgtaaa gatggaacat gctgcaacag tgggacgtcc 1800

tttgcatcca cgttcttttc agtattcatt gtaacaacgt cggtgcatct attttataat 1860

tttaaattta gtgtaattta ctcaaatatt aataatggaa aactgtga 1908

<210> 3
<211> 1752
<212> DNA
<213> Lepeophteirus salmonis

<400> 3

atgtggattc aagtccgaaa acacaactta ggtctttcct ttgaaaggat attagtgtat 60
ttactcacat tgtcatggag tctgggatcc atcgtacaag aagatttggt gatcaccaca 120
agaaaaggaa agatccgagg tgttactctg aaatctgcaa caaataagga agtagatgca 180
tggtatggga taccatacgc acaacctccc gtgggtaatc ttcgatttcg tcaccccaaa 240
gacattaatg cctgggatgg gatgaaagaa acgaccaaac atccaaattc ttgtattcaa 300
gtagttgata cattttttcc gggctttgaa ggctcagaga tgtggaatac aaatactgag 360
caaagcgagg actgccttta cttaagtgtt catgccccta aaccccgtcc tacaaaatca 420
gctgttctgg tatggatcta cggtggagga ttttattctg gaacttcaac tctggaactc 480
tatgatccac gagttcttgt gtcagaagaa aacataatct tcgtcggcat acaatatcgt 540
gttgcaagtt taggattctt attctttgat acggaggatg ttcctggaaa tgcgggattg 600
tatgatcaaa tgatggctct ccaatgggta aagaacaata tagaggcatt tggtggtgat 660
cctgataaaa tcaccatttt tggagagtcc gccggtggtt gctccgtagc ccttcatctc 720
ctttctccac tctcaaggaa cctattctct caagccatta tgcaaagttc ttcagctctt 780
gttccatggg gagtcatatc aaaaaaagaa agtatccgtc gtggtcgaag acttgcagaa 840
gagatgcgtt gtccttatgg tgaaaataat accaatgcta tgattgaatg cctgctgcaa 900
aaggacgcaa cagagttggt caaccaggag tggagtggta ccgtctttgg gatttcagag 960

ttcccatttg ttccaattgt ggatggaaaa ttcatggata aaacccctga aaaatctctt 1020
aaagaaaagg actataaaaa aaccaacatt ttaatgggag tcaataagga cgaagggaac 1080
tttttcatca tgtattatct tccagaactc ttcaaaaaaa acgaaaacgt ttatattaac 1140
cgaacagatt tcatccgcag tgtttcagat ttgaacatct atgtgaacaa tgcaggaaga 1200
gaggcaataa cttttgaata cacagattgg ctcaatccaa acgatcccat aaaaaataga 1260
gaagcaattg atcgcatggt cggtgactat caattcattt gcccaactgc tgactttgct 1320
cgtatttatg ctagtacagg aaataatata tacatgtact atttcactga gcgatcttcc 1380
actagcccat ggccaacatg gtctggtgta cttcatggcg atgaaattgc ttttgttttt 1440
ggagagcccc taaatacgtc aaaaaattat gatgattcag aaattgcctt atcaaaaaga 1500
ataatgagct attgggctaa ttttgcaaaa actgggaacc cgaatgtttt ggctaatgga 1560
aactacagca acaaaatctg gcccttacat acaccaataa aacaggaggt acttgaacta 1620
aatgcaaatt attctcgagt tttgaggggg cttcgagtta gaaaatgtgc tttttggaaa 1680
acatatcttc ctaagctttt atcattaact tcaaacaata caaagtctga agttgtaacc 1740
aatccgtcat aa 1752

<210> 4
<211> 1752

<212> DNA
<213> Lepeophteirus salmonis

<400> 4

```
atgtggattc aagtccgaaa acacaactta ggtctttcct ttgaaaggat attagtgtat      60
ttactcacat tgtcatggag tctgggatcc atcgtacaag aagatttggt gatcaccaca     120
agaaaaggaa agatccgagg tgttactctg aaatctgcaa caaataagga agtagatgca     180
tggtatggga taccatacgc acaacctccc gtgggtaatc ttcgatttcg tcaccccaaa     240
gacattaatg cctgggatgg gatgaaagaa acgaccaaac atccaaattc ttgtattcaa     300
gtagttgata catttttcc gggctttgaa ggctcagaga tgtggaatac aaatactgag     360
caaagcgagg actgccttta cttaagtgtt catgcccta aaccccgtcc tacaaaatca      420
gctgttctgg tatggatcta cggtggagga ttttattctg gaacttcaac tctggaactc     480
tatgatccac gagttcttgt gtcagaagaa aacataatct tcgtcggcat acaatatcgt     540
gttgcaagtt taggattctt attctttgat acggaggatg ttcctggaaa tgcgggattg     600
tatgatcaaa tgatggctct ccaatgggta aagaacaata tagaggcatt tggtggtgat     660
cctgataaaa tcaccatttt tggagagtcc gccggtggtt gctccgtagc ccttcatctc     720
ctttctccac tctcaaggaa cctattctct caagccatta tgcaaagttc ttcagctctt     780
gttccatggg gagtcatatc aaaaaaagaa agtatccgtc gtggtcgaag acttgcagaa     840
gagatgcgtt gtccttatgg tgaaaataat accaatgcta tgattgaatg cctgctgcaa     900
```

aaggacgcaa cagagttggt caaccaggag tggagtggta ccgtctttgg gatttcagag 960

ttcccatttg ttccaattgt ggatggaaaa ttcatggata aaacccctga aaaatctctt 1020

aaagaaaagg actataaaaa aaccaacatt ttaatgggag tcaataagga cgaagggaac 1080

tttttcatca tgtattatct tccagaactc ttcaaaaaaa acgaaaacgt ttatattaac 1140

cgaacagatt tcatccgcag tgtttcagat ttgaacatct atgtgaacaa tgcaggaaga 1200

gaggcaataa cttttgaata cacagattgg ctcaatccaa acgatcccat aaaaaataga 1260

gaagcaattg atcgcatggt cggtgactat caattcactt gcccaactgc tgactttgct 1320

cgtatttatg ctagtacagg aaataatata tacatgtact atttcactga gcgatcttcc 1380

actagcccat ggccaacatg gtctggtgta cttcatggcg atgaaattgc ttttgttttt 1440

ggagagcccc taaatacgtc aaaaaattat gatgattcag aaattgcctt atcaaaaaga 1500

ataatgagct attgggctaa ttttgcaaaa actgggaacc cgaatgtttt ggctaatgga 1560

aactacagca acaaaatctg gcccttacat acaccaataa aacaggaggt acttgaacta 1620

aatgcaaatt attctcgagt tttgaggggg cttcgagtta gaaaatgtgc tttttggaaa 1680

acatatcttc ctaagctttt atcattaact tcaaacaata caaagtctga agttgtaacc 1740

aatccgtcat aa 1752

<210> 5
<211> 635
<212> PRT
<213> Lepeophteirus salmonis

<400> 5

```
Met Trp Ile Gln Val Arg Lys Gln Asn Phe Gly Leu Tyr Phe Asn Lys
1               5                   10                  15

Ile Leu Val Tyr Leu Leu Thr Leu Ser Trp Ser Val Gly Ala Ile Val
            20                  25                  30

Gln Asp Asn Leu Val Ile Thr Thr Lys Lys Gly Lys Ile Arg Gly Val
        35                  40                  45

Thr Leu Lys Ser Ala Thr Asn Arg Glu Val Asp Ala Trp Tyr Gly Ile
    50                  55                  60

Pro Tyr Ala Gln Pro Pro Val Gly Asn Leu Arg Phe Arg His Pro Arg
65                  70                  75                  80

His Ile Asp Ala Trp Glu Gly Ile Lys Glu Thr Thr Arg Pro Pro Asn
                85                  90                  95

Ser Cys Ile Gln Val Val Asp Thr Leu Phe Pro Gly Phe Glu Gly Ala
```

100 105 110

Glu Met Trp Asn Val Asn Thr Glu Pro Ser Glu Asp Cys Leu Tyr Leu
115 120 125

Ser Val His Val Pro Lys Pro Arg Pro Thr Gly Ser Ala Val Leu Val
130 135 140

Trp Ile Tyr Gly Gly Gly Phe Tyr Ser Gly Thr Ser Thr Leu Glu Val
145 150 155 160

Tyr Asp Pro Arg Val Leu Val Ser Glu Glu Asn Ile Ile Phe Val Ala
165 170 175

Met Gln Tyr Arg Val Ala Ser Leu Gly Phe Leu Phe Phe Asp Thr Glu
180 185 190

Asp Val Pro Gly Asn Ala Gly Leu Tyr Asp Gln Met Met Ala Leu Gln
195 200 205

Trp Val Lys Asn Asn Ile Glu Glu Phe Gly Gly Asp Pro Asp Lys Ile
210 215 220

Thr Ile Phe Gly Glu Ser Ala Gly Gly Cys Ser Val Ala Leu His Leu
225 230 235 240

Leu Ser Pro Leu Ser Arg Asn Leu Phe Ser Gln Ala Ile Met Gln Ser
245 250 255

Ala Ser Ala Leu Val Pro Trp Gly Val Ile Thr Lys Lys Glu Ser Ile
260 265 270

Ile Arg Gly Arg Arg Leu Ala Glu Met Met Ser Cys Pro Tyr Asp Glu
275 280 285

Lys Asn Thr Lys Ala Met Ile Glu Cys Leu Arg Gln Lys Asp Ala Thr
290 295 300

Glu Met Val Asn Gln Glu Trp Ile Gly Ile Ile Ser Gly Ile Ala Glu
305 310 315 320

Phe Pro Phe Val Pro Ile Val Asp Gly Ser Phe Leu Asp Glu Ser Pro
325 330 335

Gly Lys Ser Leu Thr Thr Lys Asn Tyr Lys Lys Thr Asn Ile Leu Ile
340 345 350

Gly Ala Asn Lys Glu Glu Gly Asn Tyr Phe Ile Met Tyr Tyr Leu Thr

355 360 365

Asp Leu Phe Lys Asn Thr Glu Ser Val Tyr Val Asp Arg Thr Asp Phe
370 375 380

Ile Arg Ser Val Ser Glu Leu Asn His Tyr Val Lys Lys Met Gly Arg
385 390 395 400

Glu Ala Ile Thr Phe Glu Tyr Thr Asp Trp Leu Asn Pro Asn Asp Pro
405 410 415

Ile Lys Asn Arg Glu Ala Ile Asp Arg Met Val Gly Asp Tyr Gln Phe
420 425 430

Ile Cys Pro Thr Ala Asp Phe Ala Arg Ile Tyr Ala Ser Thr Gly Asn
435 440 445

Asn Val Tyr Met Tyr Tyr Phe Thr Glu Arg Ser Ser Thr Ser Pro Trp
450 455 460

Pro Thr Trp Ser Gly Val Leu His Gly Asp Glu Ile Ala Phe Val Phe
465 470 475 480

Gly Glu Ala Leu Asn Lys Ser Lys Asn Tyr Asp Lys Ser Glu Ile Ala
485 490 495

Leu Ser Lys Arg Met Met Gly Tyr Trp Ala Asn Phe Ala Lys Thr Gly
500 505 510

Asn Pro Ser Leu Ser Ala Asp Gly Thr Trp Ser Thr Asn Tyr Trp Pro
515 520 525

Leu His Thr Pro Thr Lys Gln Glu Val Leu Glu Leu Asn Ala Asn Tyr
530 535 540

Ser Arg Val Leu Glu Gly Leu Arg Val Lys Lys Cys Ala Phe Trp Lys
545 550 555 560

Lys Tyr Leu Pro Lys Leu Leu Ser Leu Thr Ser Asn Asn Gly Asp His
565 570 575

Gln Cys Gln Thr Ser Ser Thr Ser Ser Pro Ser Cys Cys Lys Asp Gly
580 585 590

Thr Cys Cys Asn Ser Gly Thr Ser Phe Ala Ser Thr Phe Phe Ser Val
595 600 605

Phe Ile Val Thr Thr Ser Val His Leu Phe Tyr Asn Phe Lys Phe Ser

610 615 620

Val Ile Tyr Ser Asn Ile Asn Asn Gly Lys Leu
625 630 635

<210> 6
<211> 635
<212> PRT
<213> Lepeophteirus salmonis

<400> 6

Met Trp Ile Gln Val Arg Lys Gln Asn Phe Gly Leu Tyr Phe Asn Lys
1 5 10 15

Ile Leu Val Tyr Leu Leu Thr Leu Ser Trp Ser Val Gly Ala Ile Val
20 25 30

Gln Asp Asn Leu Val Ile Thr Thr Lys Lys Gly Lys Ile Arg Gly Val
35 40 45

Thr Leu Lys Ser Ala Thr Asn Arg Glu Val Asp Ala Trp Tyr Gly Ile
50 55 60

Pro Tyr Ala Gln Pro Pro Val Gly Asn Leu Arg Phe Arg His Pro Arg
65 70 75 80

His Ile Asp Ala Trp Glu Gly Ile Lys Glu Thr Thr Arg Pro Pro Asn
85 90 95

Ser Cys Ile Gln Val Val Asp Thr Leu Phe Pro Gly Phe Glu Gly Ala
100 105 110

Glu Met Trp Asn Val Asn Thr Glu Pro Ser Glu Asp Cys Leu Tyr Leu
115 120 125

Ser Val His Val Pro Lys Pro Arg Pro Thr Gly Ser Ala Val Leu Val
130 135 140

Trp Ile Tyr Gly Gly Gly Phe Tyr Ser Gly Thr Ser Thr Leu Glu Val
145 150 155 160

Tyr Asp Pro Arg Val Leu Val Ser Glu Glu Asn Ile Ile Phe Val Ala
165 170 175

Met Gln Tyr Arg Val Ala Ser Leu Gly Phe Leu Phe Phe Asp Thr Glu
180 185 190

Asp Val Pro Gly Asn Ala Gly Leu Tyr Asp Gln Met Met Ala Leu Gln
195 200 205

Trp Val Lys Asn Asn Ile Glu Glu Phe Gly Gly Asp Pro Asp Lys Ile
    210                 215                 220

Thr Ile Phe Gly Glu Ser Ala Gly Gly Cys Ser Val Ala Leu His Leu
225                 230                 235                 240

Leu Ser Pro Leu Ser Arg Asn Leu Phe Ser Gln Ala Ile Met Gln Ser
                245                 250                 255

Ala Ser Ala Leu Val Pro Trp Gly Val Ile Thr Lys Lys Glu Ser Ile
            260                 265                 270

Ile Arg Gly Arg Arg Leu Ala Glu Met Met Ser Cys Pro Tyr Asp Glu
        275                 280                 285

Lys Asn Thr Lys Ala Met Ile Glu Cys Leu Arg Gln Lys Asp Ala Thr
    290                 295                 300

Glu Met Val Asn Gln Glu Trp Ile Gly Ile Ile Ser Gly Ile Ala Glu
305                 310                 315                 320

Phe Pro Phe Val Pro Ile Val Asp Gly Ser Phe Leu Asp Glu Ser Pro
                325                 330                 335

Gly Lys Ser Leu Thr Thr Lys Asn Tyr Lys Lys Thr Asn Ile Leu Ile
            340                 345                 350

Gly Ala Asn Lys Glu Glu Gly Asn Tyr Tyr Ile Met Tyr Tyr Leu Thr
        355                 360                 365

Asp Leu Phe Lys Asn Thr Glu Ser Val Tyr Val Asp Arg Thr Asp Phe
    370                 375                 380

Ile Arg Ser Val Ser Glu Leu Asn His Tyr Val Lys Lys Met Gly Arg
385                 390                 395                 400

Glu Ala Ile Thr Phe Glu Tyr Thr Asp Trp Leu Asn Pro Asn Asp Pro
                405                 410                 415

Ile Lys Asn Arg Glu Ala Ile Asp Arg Met Val Gly Asp Tyr Gln Phe
            420                 425                 430

Ile Cys Pro Thr Ala Asp Phe Ala Arg Ile Tyr Ala Ser Thr Gly Asn
        435                 440                 445

Asn Val Tyr Met Tyr Tyr Phe Thr Glu Arg Ser Ser Thr Ser Pro Trp
    450                 455                 460

Pro Thr Trp Ser Gly Val Leu His Gly Asp Glu Ile Ala Phe Val Phe
465 470 475 480

Gly Glu Ala Leu Asn Lys Ser Lys Asn Tyr Asp Lys Ser Glu Ile Ala
485 490 495

Leu Ser Lys Arg Met Met Gly Tyr Trp Ala Asn Phe Ala Lys Thr Gly
500 505 510

Asn Pro Ser Leu Ser Ala Asp Gly Thr Trp Ser Thr Asn Tyr Trp Pro
515 520 525

Leu His Thr Pro Thr Lys Gln Glu Val Leu Glu Leu Asn Ala Asn Tyr
530 535 540

Ser Arg Val Leu Glu Gly Leu Arg Val Lys Lys Cys Ala Phe Trp Lys
545 550 555 560

Lys Tyr Leu Pro Lys Leu Leu Ser Leu Thr Ser Asn Asn Gly Asp His
565 570 575

Gln Cys Gln Thr Ser Ser Thr Ser Ser Pro Ser Cys Cys Lys Asp Gly
580 585 590

Thr Cys Cys Asn Ser Gly Thr Ser Phe Ala Ser Thr Phe Phe Ser Val
595 600 605

Phe Ile Val Thr Thr Ser Val His Leu Phe Tyr Asn Phe Lys Phe Ser
610 615 620

Val Ile Tyr Ser Asn Ile Asn Asn Gly Lys Leu
625 630 635

<210> 7
<211> 583
<212> PRT
<213> Lepeophteirus salmonis

<400> 7

Met Trp Ile Gln Val Arg Lys His Asn Leu Gly Leu Ser Phe Glu Arg
1 5 10 15

Ile Leu Val Tyr Leu Leu Thr Leu Ser Trp Ser Leu Gly Ser Ile Val
20 25 30

Gln Glu Asp Leu Val Ile Thr Thr Arg Lys Gly Lys Ile Arg Gly Val
35 40 45

Thr Leu Lys Ser Ala Thr Asn Lys Glu Val Asp Ala Trp Tyr Gly Ile
50 55 60

Pro Tyr Ala Gln Pro Pro Val Gly Asn Leu Arg Phe Arg His Pro Lys
65 70 75 80

Asp Ile Asn Ala Trp Asp Gly Met Lys Glu Thr Thr Lys His Pro Asn
85 90 95

Ser Cys Ile Gln Val Val Asp Thr Phe Phe Pro Gly Phe Glu Gly Ser
100 105 110

Glu Met Trp Asn Thr Asn Thr Glu Gln Ser Glu Asp Cys Leu Tyr Leu
115 120 125

Ser Val His Ala Pro Lys Pro Arg Pro Thr Lys Ser Ala Val Leu Val
130 135 140

Trp Ile Tyr Gly Gly Gly Phe Tyr Ser Gly Thr Ser Thr Leu Glu Leu
145 150 155 160

Tyr Asp Pro Arg Val Leu Val Ser Glu Glu Asn Ile Ile Phe Val Gly
165 170 175

Ile Gln Tyr Arg Val Ala Ser Leu Gly Phe Leu Phe Phe Asp Thr Glu
180 185 190

Asp Val Pro Gly Asn Ala Gly Leu Tyr Asp Gln Met Met Ala Leu Gln
195 200 205

Trp Val Lys Asn Asn Ile Glu Ala Phe Gly Gly Asp Pro Asp Lys Ile
210 215 220

Thr Ile Phe Gly Glu Ser Ala Gly Gly Cys Ser Val Ala Leu His Leu
225 230 235 240

Leu Ser Pro Leu Ser Arg Asn Leu Phe Ser Gln Ala Ile Met Gln Ser
245 250 255

Ser Ser Ala Leu Val Pro Trp Gly Val Ile Ser Lys Lys Glu Ser Ile
260 265 270

Arg Arg Gly Arg Arg Leu Ala Glu Glu Met Arg Cys Pro Tyr Gly Glu
275 280 285

Asn Asn Thr Asn Ala Met Ile Glu Cys Leu Leu Gln Lys Asp Ala Thr
290 295 300

Glu Leu Val Asn Gln Glu Trp Ser Gly Thr Val Phe Gly Ile Ser Glu
305 310 315 320

Phe Pro Phe Val Pro Ile Val Asp Gly Lys Phe Met Asp Lys Thr Pro
325 330 335

Glu Lys Ser Leu Lys Glu Lys Asp Tyr Lys Lys Thr Asn Ile Leu Met
340 345 350

Gly Val Asn Lys Asp Glu Gly Asn Phe Phe Ile Met Tyr Tyr Leu Pro
355 360 365

Glu Leu Phe Lys Lys Asn Glu Asn Val Tyr Ile Asn Arg Thr Asp Phe
370 375 380

Ile Arg Ser Val Ser Asp Leu Asn Ile Tyr Val Asn Asn Ala Gly Arg
385 390 395 400

Glu Ala Ile Thr Phe Glu Tyr Thr Asp Trp Leu Asn Pro Asn Asp Pro
405 410 415

Ile Lys Asn Arg Glu Ala Ile Asp Arg Met Val Gly Asp Tyr Gln Phe
420 425 430

Ile Cys Pro Thr Ala Asp Phe Ala Arg Ile Tyr Ala Ser Thr Gly Asn
435 440 445

Asn Ile Tyr Met Tyr Tyr Phe Thr Glu Arg Ser Ser Thr Ser Pro Trp
450 455 460

Pro Thr Trp Ser Gly Val Leu His Gly Asp Glu Ile Ala Phe Val Phe
465 470 475 480

Gly Glu Pro Leu Asn Thr Ser Lys Asn Tyr Asp Asp Ser Glu Ile Ala
485 490 495

Leu Ser Lys Arg Ile Met Ser Tyr Trp Ala Asn Phe Ala Lys Thr Gly
500 505 510

Asn Pro Asn Val Leu Ala Asn Gly Asn Tyr Ser Asn Lys Ile Trp Pro
515 520 525

Leu His Thr Pro Ile Lys Gln Glu Val Leu Glu Leu Asn Ala Asn Tyr
530 535 540

Ser Arg Val Phe Glu Gly Leu Arg Val Arg Lys Cys Ala Phe Trp Lys
545 550 555 560

Thr Tyr Leu Pro Lys Leu Leu Ser Leu Thr Ser Asn Asn Thr Lys Ser
565 570 575

Glu Val Val Thr Asn Pro Ser
580

<210> 8
<211> 583
<212> PRT
<213> Lepeophteirus salmonis

<400> 8

Met Trp Ile Gln Val Arg Lys His Asn Leu Gly Leu Ser Phe Glu Arg
1 5 10 15

Ile Leu Val Tyr Leu Leu Thr Leu Ser Trp Ser Leu Gly Ser Ile Val
20 25 30

Gln Glu Asp Leu Val Ile Thr Thr Arg Lys Gly Lys Ile Arg Gly Val
35 40 45

Thr Leu Lys Ser Ala Thr Asn Lys Glu Val Asp Ala Trp Tyr Gly Ile
50 55 60

Pro Tyr Ala Gln Pro Pro Val Gly Asn Leu Arg Phe Arg His Pro Lys
65 70 75 80

Asp Ile Asn Ala Trp Asp Gly Met Lys Glu Thr Thr Lys His Pro Asn
85 90 95

Ser Cys Ile Gln Val Val Asp Thr Phe Phe Pro Gly Phe Glu Gly Ser
100 105 110

Glu Met Trp Asn Thr Asn Thr Glu Gln Ser Glu Asp Cys Leu Tyr Leu
115 120 125

Ser Val His Ala Pro Lys Pro Arg Pro Thr Lys Ser Ala Val Leu Val
130 135 140

Trp Ile Tyr Gly Gly Gly Phe Tyr Ser Gly Thr Ser Thr Leu Glu Leu
145 150 155 160

Tyr Asp Pro Arg Val Leu Val Ser Glu Glu Asn Ile Ile Phe Val Gly
165 170 175

Ile Gln Tyr Arg Val Ala Ser Leu Gly Phe Leu Phe Phe Asp Thr Glu
180 185 190

```
Asp Val Pro Gly Asn Ala Gly Leu Tyr Asp Gln Met Met Ala Leu Gln
        195                 200                 205

Trp Val Lys Asn Asn Ile Glu Ala Phe Gly Gly Asp Pro Asp Lys Ile
    210                 215                 220

Thr Ile Phe Gly Glu Ser Ala Gly Gly Cys Ser Val Ala Leu His Leu
225                 230                 235                 240

Leu Ser Pro Leu Ser Arg Asn Leu Phe Ser Gln Ala Ile Met Gln Ser
                245                 250                 255

Ser Ser Ala Leu Val Pro Trp Gly Val Ile Ser Lys Lys Glu Ser Ile
            260                 265                 270

Arg Arg Gly Arg Arg Leu Ala Glu Glu Met Arg Cys Pro Tyr Gly Glu
        275                 280                 285

Asn Asn Thr Asn Ala Met Ile Glu Cys Leu Leu Gln Lys Asp Ala Thr
    290                 295                 300

Glu Leu Val Asn Gln Glu Trp Ser Gly Thr Val Phe Gly Ile Ser Glu
305                 310                 315                 320

Phe Pro Phe Val Pro Ile Val Asp Gly Lys Phe Met Asp Lys Thr Pro
                325                 330                 335

Glu Lys Ser Leu Lys Glu Lys Asp Tyr Lys Lys Thr Asn Ile Leu Met
            340                 345                 350

Gly Val Asn Lys Asp Glu Gly Asn Phe Phe Ile Met Tyr Tyr Leu Pro
        355                 360                 365

Glu Leu Phe Lys Lys Asn Glu Asn Val Tyr Ile Asn Arg Thr Asp Phe
    370                 375                 380

Ile Arg Ser Val Ser Asp Leu Asn Ile Tyr Val Asn Asn Ala Gly Arg
385                 390                 395                 400

Glu Ala Ile Thr Phe Glu Tyr Thr Asp Trp Leu Asn Pro Asn Asp Pro
                405                 410                 415

Ile Lys Asn Arg Glu Ala Ile Asp Arg Met Val Gly Asp Tyr Gln Phe
            420                 425                 430

Thr Cys Pro Thr Ala Asp Phe Ala Arg Ile Tyr Ala Ser Thr Gly Asn
        435                 440                 445
```

Asn Ile Tyr Met Tyr Tyr Phe Thr Glu Arg Ser Ser Thr Ser Pro Trp
450 455 460

Pro Thr Trp Ser Gly Val Leu His Gly Asp Glu Ile Ala Phe Val Phe
465 470 475 480

Gly Glu Pro Leu Asn Thr Ser Lys Asn Tyr Asp Asp Ser Glu Ile Ala
485 490 495

Leu Ser Lys Arg Ile Met Ser Tyr Trp Ala Asn Phe Ala Lys Thr Gly
500 505 510

Asn Pro Asn Val Leu Ala Asn Gly Asn Tyr Ser Asn Lys Ile Trp Pro
515 520 525

Leu His Thr Pro Ile Lys Gln Glu Val Leu Glu Leu Asn Ala Asn Tyr
530 535 540

Ser Arg Val Phe Glu Gly Leu Arg Val Arg Lys Cys Ala Phe Trp Lys
545 550 555 560

Thr Tyr Leu Pro Lys Leu Leu Ser Leu Thr Ser Asn Asn Thr Lys Ser
565 570 575

Glu Val Val Thr Asn Pro Ser
580

<210> 9
<211> 22
<212> DNA
<213> Lepeoptheirus salmonis

<400> 9
ttttaattgg agcgaataag ga 22

<210> 10
<211> 21
<212> DNA
<213> Lepeophtheirus salmonis

<400> 10
tctgttcgat caacatagac g 21

<210> 11
<211> 24
<212> DNA
<213> Lepeophtheirus salmonis

<400> 11
attttaattg gagcgaataa ggaa 24

<210> 12

<211> 26
<212> DNA
<213> Lepeophtheirus Salmonis

<400> 12
cgctctccgt atttttaaag agatct 26

<210> 13
<211> 20
<212> DNA
<213> Lepeophtheirus salmonis

<400> 13
aagggaatta ttwcatcatg 20

<210> 14
<211> 20
<212> DNA
<213> Lepeophtheirus salmonis

<400> 14
aagggaatta tttcatcatg 20

<210> 15
<211> 20
<212> DNA
<213> Lepeophtheirus salmonis

<400> 15
aagggaatta ttacatcatg 20

## Description

### Field of the invention

The present invention relates to a method for detection of a mutation, in particular a single polymorphism associated with organophosphate resistant *Lepeophtheirus salmonis,* and oligonucleotide sequences and kits useful in the method of the present invention. The present invention furthermore provides kits and reagents useful for the detection of organophosphate resistance associated mutations.

### Background of the invention

Sea lice (*Lepeophtheirus salmonis* and *Caligus* spp.) are the major pathogens affecting global salmon farming industry and have a significant impact in many areas. The annual loss has recently been estimated to €300 million (Costello M. J. (2009)) and the aquaculture industry relays heavily on a few chemotherapeutants for lice control. Emerging resistance development to these drugs increase the necessity to develop new treatment methods (biological, prophylactic and drugs) and tools to avoid increased loss due to sea lice and to ensure a sustainable salmon farming industry in the future. Control measures have relied upon a limited number of chemotherapeutants since the 1970s. Parasite resistance and reduced efficacy have now been reported for the majority of these compounds (Sevatdal S., et al., (2005)).

Although various chemotherapeutants have been in use in the aquaculture industry for more than 30 years, it is only during the last 15 years that such use has been part of some kind of integrated pest management system. Management practices include coordinated salmon production within a defined area, use of single year class of fish, limited production period, fallowing, coordinated restocking, use of wrasse, synchronized treatments during the winter and targeting female lice to reduce the impact of settlement during the spring (Pike A., Wadsworth S. L., (2000)).

Organophosphates belong to a group of anti-parasitic agents that acts by inhibiting a vital enzyme, acetyl choline esterase, in cholinergic synapses. The inhibition blocks the cleavage of the transmitter substance acetyl choline and results in elevated levels of it in the synaptic cleft thereby causing exitation, paralysis and death of the organism.

Organophosphates have been used against salmon lice *(Lepeophtheirus salmonis),* a species within a group of ectoparasitic copepods on fish called sea lice, in Norwegian salmonid aquaculture since the late 1970s. The first agent used was metrifonate (Neguvon™), followed by dichlorvos in 1986 (Nuvan™) and azamethiphos (Salmosan™) in 1994 (*Torrissen et al. 2013*). In 1991, the first cases of reduced efficacy of organophosphate treatments were noted in Mid-Norway (*Denholm et al. 2002*). When the use of azamethiphos was terminated during 1999, the problem of reduced sensitivity in salmon lice against azamethiphos was wide-spread. At the time, the cause for resistance in salmon lice against azamethiphos was not determined.

Azamethiphos was re-introduced as a treatment against salmon lice in 2008. In 2009, the first new reports of reduced efficacy of treatments with azamethiphos came from the field. A bioassay to test the sensitivity of the parasites was established by Veso (Sevatdal, unpublished). Since then several treatment failures have been verified as resistance using this bioassay (Sevatdal, unpublished).

Further controls are required to progress towards a true integrated pest management (IPM) system common to other forms of food production. One key to succeeding with an IPM is to develop tools for the management of resistance to the medicines in use (Brook K. (2009). Considerations in developing an integrated pest management program for control of sea lice on farmed salmon in Pacific Canada. Journal of Fish Diseases. 32. 59-73). So far, drug resistance in sea lice has been detected by various types of bioassays as a significant increase in EC50/LC50. Although the bioassays can detect any type of resistance to a given drug, such methods are not very accurate or sensitive.

The genomes of all organisms undergo spontaneous mutations during their continuing evolution, forming variant forms of progenitor genetic sequences. A mutation may results in an evolutionary advantage or disadvantage relative to a progenitor form or may be neutral. A variant that result in an evolutionary advantage may eventually be incorporated in many members of the species and may thus effectively become the progenitor form. Furthermore, often various variant forms survive and coexist in a species population. The coexistence of multiple forms of a genetic sequence gives rise to genetic polymorphism, including single-nucleotide polymorphisms (SNPs).

A single-nucleotide polymorphism (SNP) is a DNA sequence variation occurring when a single nucleotide - A, T, C or G - in the genome (or other shared sequence) differs between members of a biological species or paired chromosomes in an organism. For example, two DNA fragments from different individuals, AAGCCTA to AAGCTTA, contain a difference in a single nucleotide, commonly referred to as two alleles. Almost all common SNPs have only two alleles. The genomic distribution of SNPs is not homogenous; SNPs usually occur in non-coding regions more frequently than in coding regions or, in general, where natural selection is acting and fixating the allele of the SNP that constitutes the most favorable genetic adaptation.

Up to date, no mutations or SNPs consequently associated with organophosphate resistance in sea lice have been reported.

Efficient and sensitive methods for diagnosing resistance are crucial in order to manage and control drug resistance. Early detection of reduced sensitivity to a chemical can enable effective countermeasures to be enforced at a time point when these have a greater probability of being effective. Therefore, accurate and speedy identification of organophosphate resistant *Lepeophtheirus salmonis* is crucial. Detection of organoposphate resistance prior to treatment, and the use of such analyses after treatment to evaluate treatment efficacy constitutes an important determinant for the integrated pest management (IPM) in the aquaculture industry.

FOURNIER D et al., Drosophila acetylcholinesterase: Mechanisms of resistance to organophosphates, CHEMICO-BIOLOGICAL INTEGRATIONS, 1993, vol. 87, no. 1-3, pages 233-238 discloses that four mutations have been identified in resistant strains of Drosophila melanogaster: Phe115 to Ser, Ileu199 to Val, Gly303 to Ala and Phe368 to Tyr.

**Summary of invention**

The present invention is based on the surprising finding that resistance towards organophosphates commonly used to combat sea lice infestation is linked to an identified mutation present in one of two novel acetylcholine esterase protein genes identified in sea lice (*Lepeophtheirus salmonis)* resulting in an amino acid substitution compared with the corresponding gene present in organophosphate sensistive sea lice. More particularly, the present invention is based on the identification of novel single-nucleotide polymorphisms (SNPs) in the gene encoding the acetylcholine esterase protein (LS-ace1) of the sea lice (*Lepeophtheirus salmonis*) shown to be involved in the resistance towards organophosphate-based chemotherapy. Specifically, the present inventors have identified one organophosphate resistance-associated SNPs located in SL-ace1 resulting in the substitution of phenyl with tyrosine in position 362 (Phe362Tyr).

Described herein is an in vitro method provided for detection of organophosphate resistance in one or more crustaceans comprising the steps of detecting a mutation associated with organophosphate resistance in the acetylcholine esterase gene 1 of the crustaceans to be analyzed.

The crustacean may be one or more copepods, e.g. belonging to the family Caligidae. The copepod is selected from the group consisting of *Lepeophtheirus salmonis, Caligus elongatus,* and *Caligus rogercresseyi.*

Said mutation is present in the codon of the acetylcholine esterase gene 1 encoding the amino acid in position 362 of the ace 1 protein, and said codon encodes tyrosine.

The mutation detected is a single nucleotid polymorphism.

According to one embodiment the present method comprise the steps of detecting a single nucleotide polymorphism (SNP) associated with organophosphate resistance in the sea lice to be analyzed, wherein said sea lice is resistant to organophosphate if comprising an acetylcholine esterase gene 1 encoding a acetylcholine esterase protein according to SEQ ID No. 6, wherein the amino acid in position 362 is tyrosine.

According to another embodiment the present method comprise the steps of detecting a single nucleotide polymorphism (SNP) associated with organophosphate resistance in the one or more sea lice to be analyzed, wherein said sea lice is resistant to organophosphate if comprising a SL-ace1 gene wherein the nucleotide in position 1085 of said SL-ace1 is A in the one or more sea lice to be analyzed, and wherein the numbering of said positions is in accordance with the sequence depicted in SEQ ID No. 2.

According to another embodiment the present method comprise the steps of:

a) collecting sea lice from infested fish or water samples;

b) isolating genomic material from the any life stage of collected sea lice;

c) determining the nucleotide polymorphic site at the positions 1085 of the isolated genomic material compared with of the nucleic acid sequence SEQ ID No. 2, wherein said sea lice is resistant to organophosphates if the nucleotide in said position is A, or a complementary oligonucleotide thereof.

According to another embodiment of the present method, said step c) is performed using a primer selected from the group consisting of SEQ ID No. 9-12.

According to another embodiment of the present method, said step c) is performed using at least one probe selected from the group consisting of SEQ ID No. 13-15.

According to another embodiment of the present method, said step c) comprises nucleic acid amplification.

According to another embodiment of the present method, the nucleic acid amplification is performed using polymerase chain reaction.

According to another embodiment of the present method, said step c) is performed by contacting the genomic material of the sea lice to be analyzed with a detection reagent, and determining which nucleotide is present in position 1085.

According to another embodiment of the present method, said step c) is performed using SNP specific probe hybridization, SNP specific primer extension, SPN specific amplification, sequencing, 5' nuclease digestion, molecular beacon assay, oligonucleotide ligation assay, size analysis, single-stranded conformation polymorphism analysis, denaturing gradient gel electrophoresis.

According to another embodiment of the present method, the organophosphate is azamethiphos.

Disclosed herein is also an isolated oligonucleotide sequence comprising single nucleotide polymorphism (SNP) associated with organophosphate resistance in crustaceans, wherein said isolated oligonucleotide sequence comprises nucleotides that distinguishes sea lice which are resistant to organophosphates from non-resistance to organophosphates, and wherein the SNP are present in the genomic DNA of the crustaceans.

The oligonucleotide comprises a single-nucleotide polymorphism (SNP) associated with organophosphate resistance in sea lice, wherein said isolated oligonucleotide sequence comprises nucleotides that distinguishes sea lice which are resistant to organophosphates from non-resistant sea lice, and which enables the detection of the codon of the acel gene encoding amino acid 362 of the acetylcholine esterase protein.

The oligonucleotide sequence of the present invention is identical or has at least 80% sequence identity with a sequence selected from the group consisting of SEQ ID No. 9-12 or a fragment thereof, and complementary sequences of SEQ ID No 9-12 and fragments thereof, wherein said oligonucleotide sequence is an oligonucleotide primer comprising at least 16 consecutive nucleotides of a target nucleic acid molecule of SEQ ID No. 2. According to another embodiment, the oligonucleotide sequence is identical or has at least 80 % sequence identity with a sequence selected from the group consisting of SEQ ID No. 13-14 and complementary sequences of SEQ ID No 13-14. According to another embodiment, the oligonucleotide sequence is SEQ ID No. 15 or the sequence complementary to SEQ ID No 15.

Furthermore, the present invention provides a kit for detection of organophosphate resistance in sea lice comprising at least one oligonucleotide according to the present invention.

Finally, the present invention provides the use of an isolated oligonucleotide sequence according to the present invention for the detection of organophosphate resistance in one or more sea lice, wherein said sequence is capable of detecting a codon encoding tyrosin in position 362 of the acetylcholine esterase protein (SEQ ID No. 6), in particular wherein the oligonucleotide sequence is capable of detecting the SNP T1085A.

The present invention and its various embodiments will be described in more detail in the following.

**Figures**

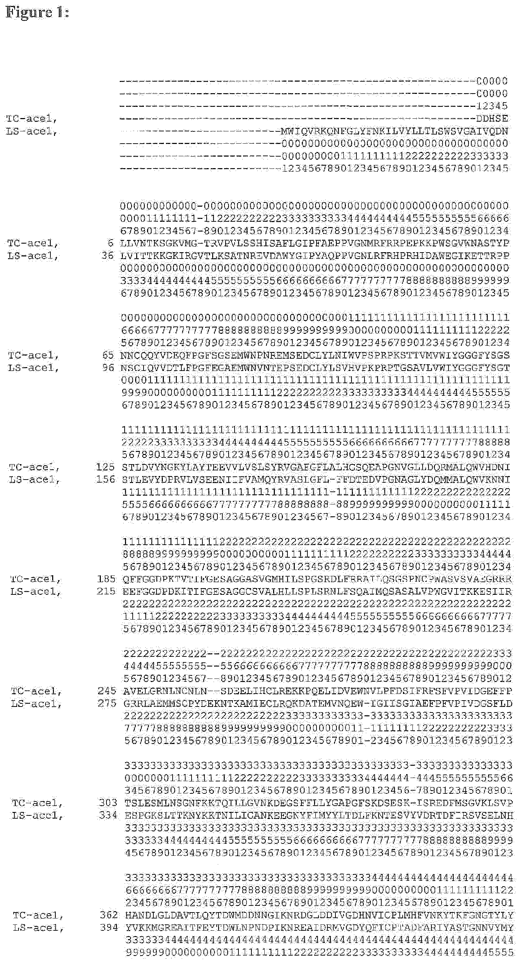

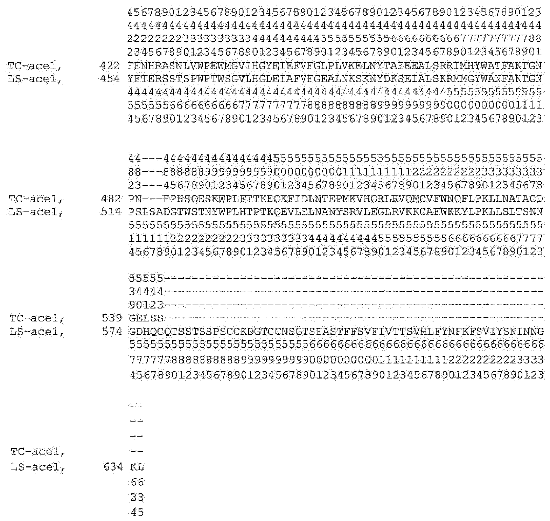

**Figure 1** Alignment of the amino acid sequences for the acetyl cholin esterase from *Torpedo californica* (TC-ace1) and *Lepeophtheirus salmonis* (LS-ace1). The number (vertically displayed) over (*Torpedo californica*) or under *(Lepeophtheirus salmonis*) the amino acid gives the number in their respective amino acid sequence.

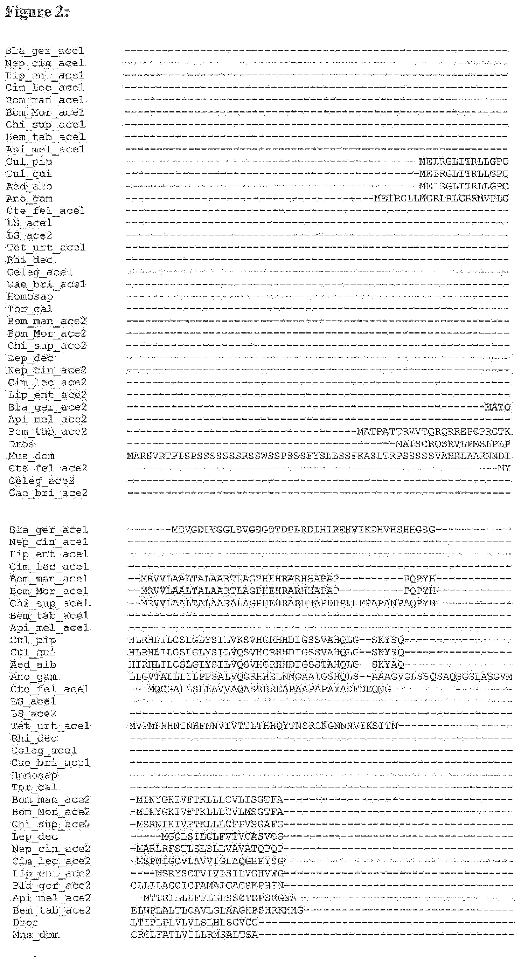

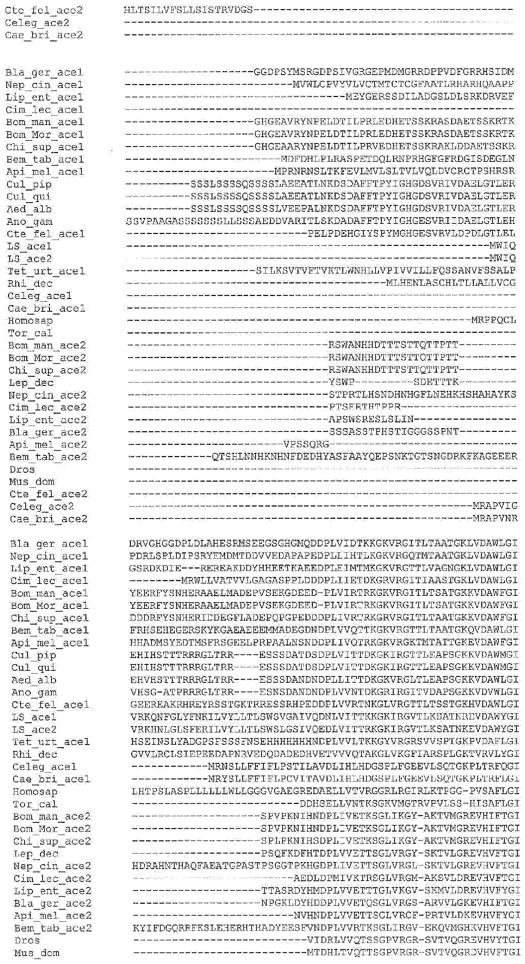

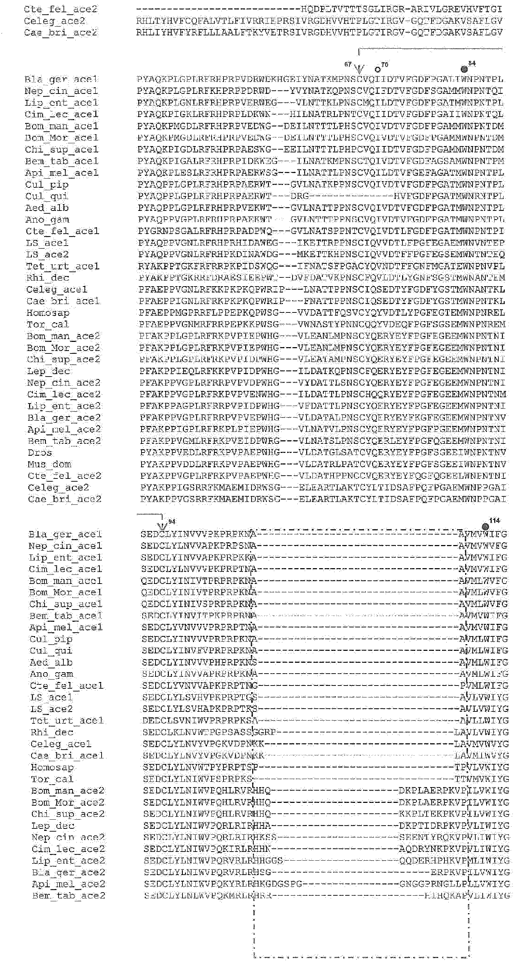

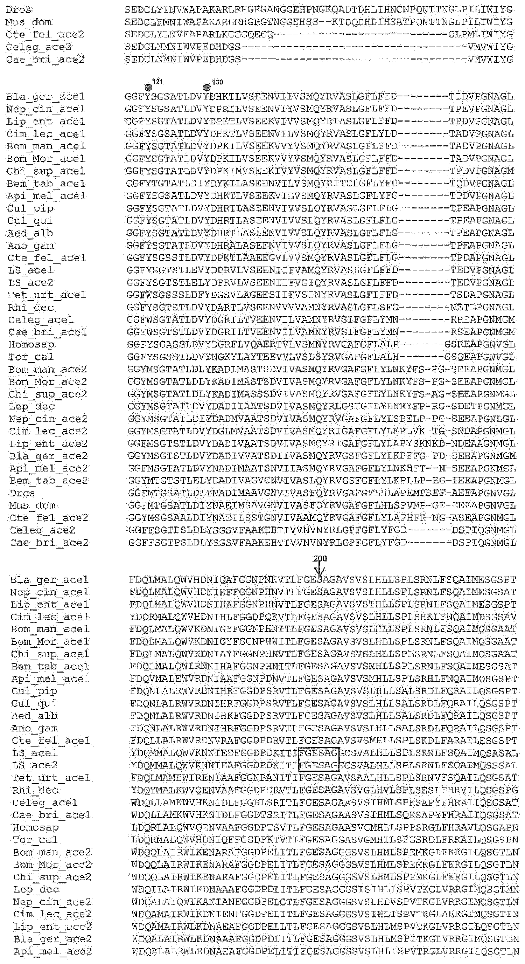

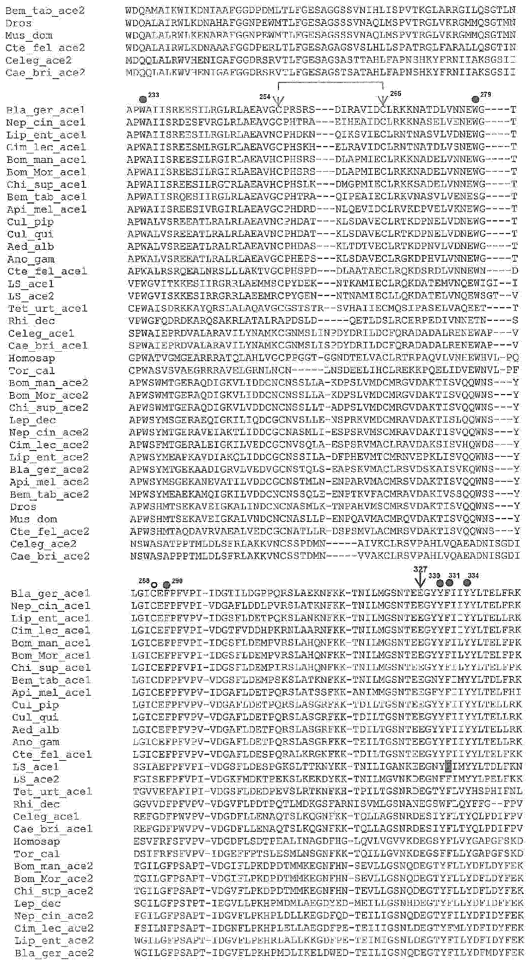

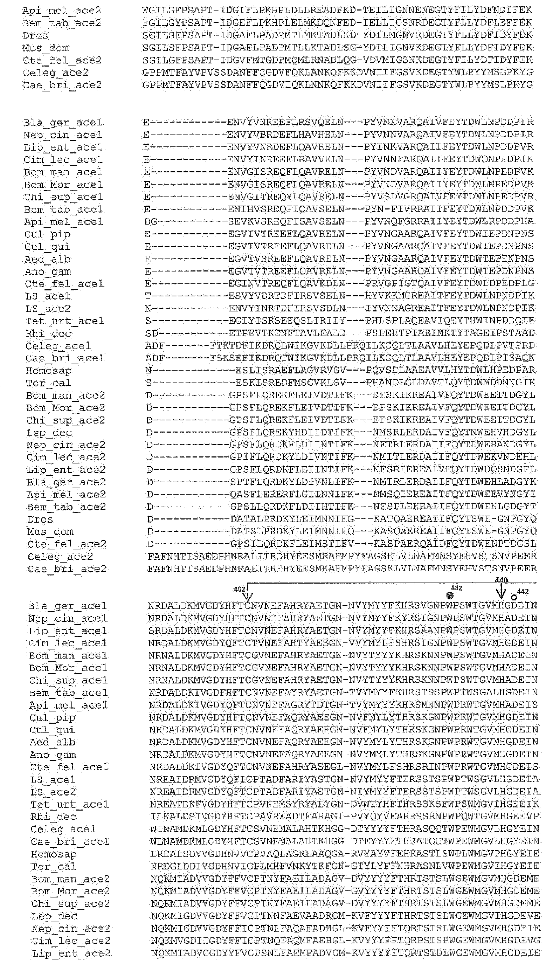

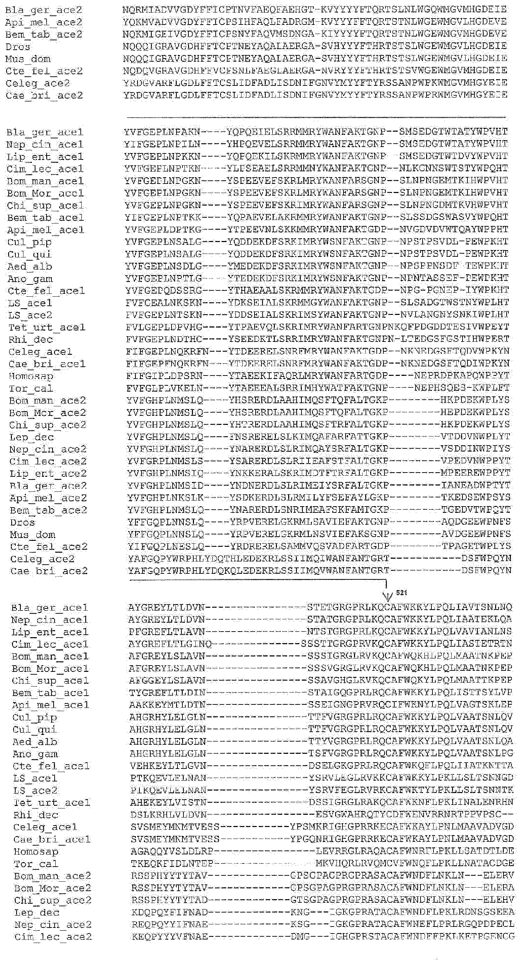

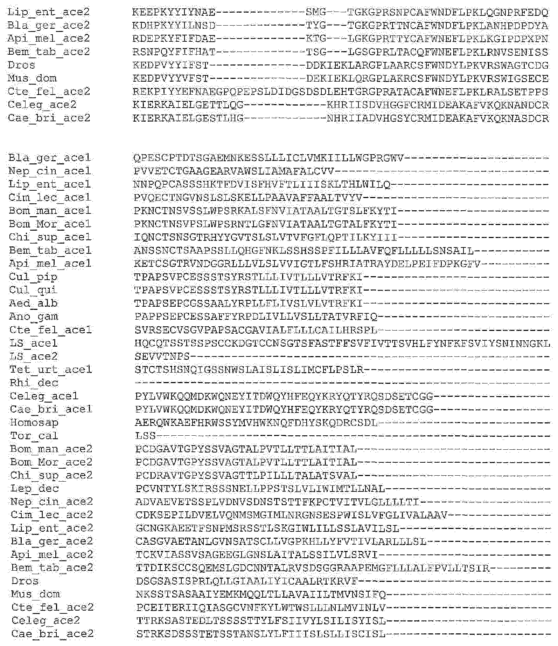

**Figure 2** Alignment of LS-ace1 and LS-ace2 proteins with other typical AChE proteins from other species (Insects, Nematods, Arachnida and vertebrates). By convention, numbering is that of *Torpedo californica.* The three amino acids composing the catalytic triad (Ser200, Glu327 and His440) are indicated by arrows. The 14 conserved aromatic residues lining the active gorge are represented by circles. Out of these 14, 11 residues were present in both LS-ace1 and LS-ace2 (shown by filled circles), whereas the other 3 non conserved residues (shown by open circles) were absent in both the proteins of salmon lice.The choline binding site (Trp at 84) is underlined. Three interchain disulphide bridges are drawn between conserved Cys residues (shown by arrows). The solid box represents the canonical *FGESAG* motif, characteristic of the active site of cholinesterases. The dotted box represents the typical sequence insertion/deletion domain that easily distinguishes acel and ace2 proteins.

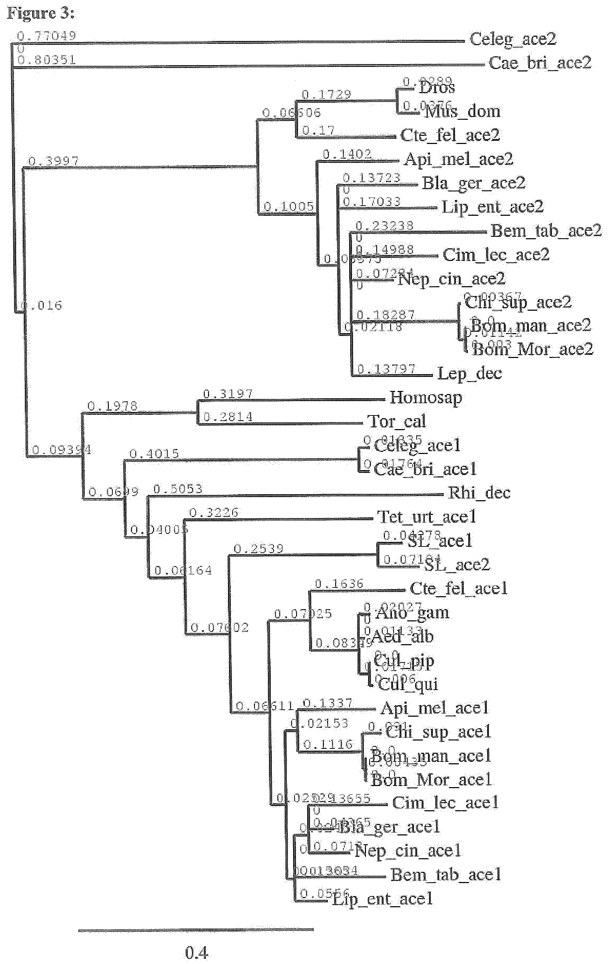

**Figure 3** Phylogenetic relationship of LS-ace1 and LS-ace2 with other acetylcholinesterases from Insecta, Nematoda, Arachnidae and Veterbrata. The phylogenetic tree was constructed using a ClustalW alignment and ----tree (MrBayes). LS-acel (SL_ace1) and LS-ace2 (SL_ace2) are boxed in the phylogenetic tree.

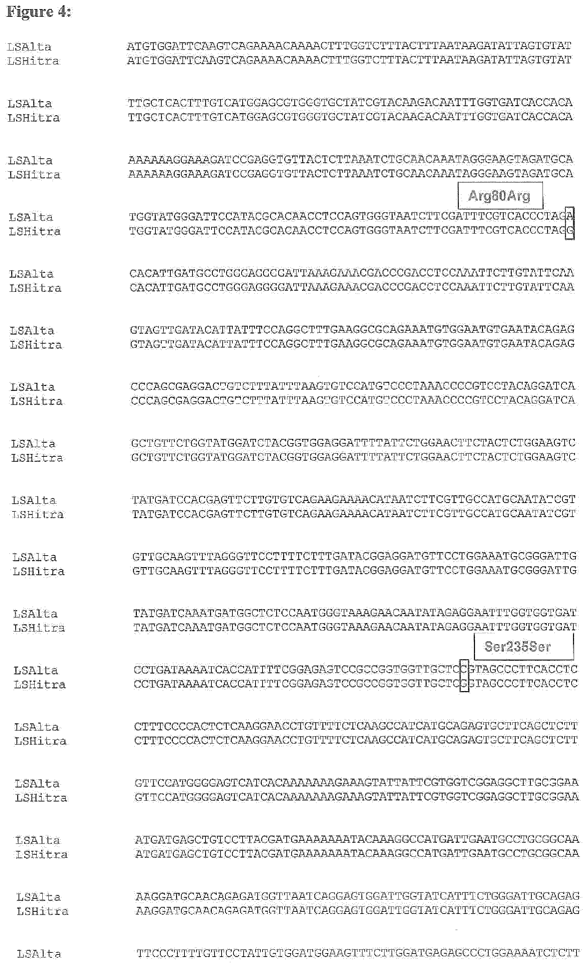

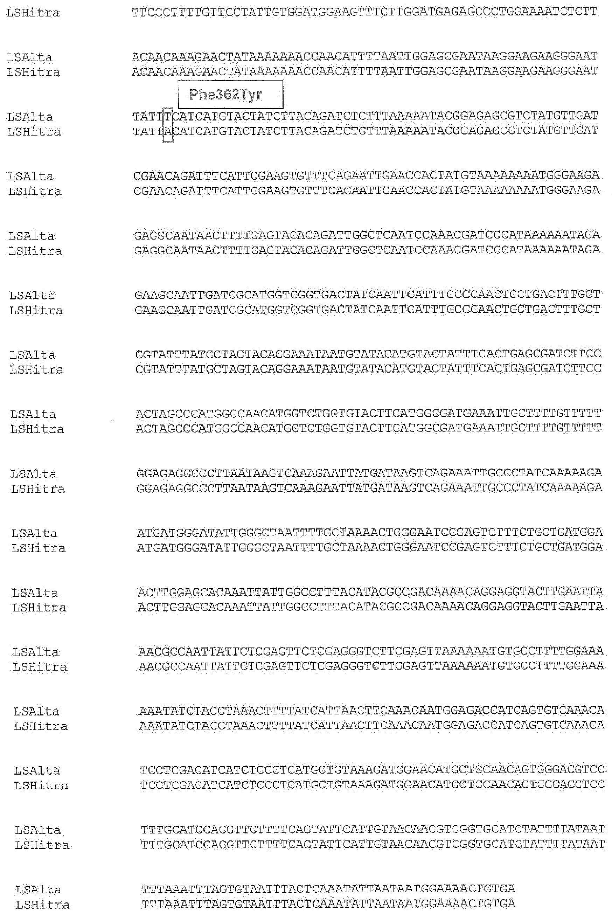

**Figure 4** Nucleotide alignment of LS-ace1 from sensitive (LSAlta) and resistant (LSHitra) sea lice strain. The changes identified are boxed.

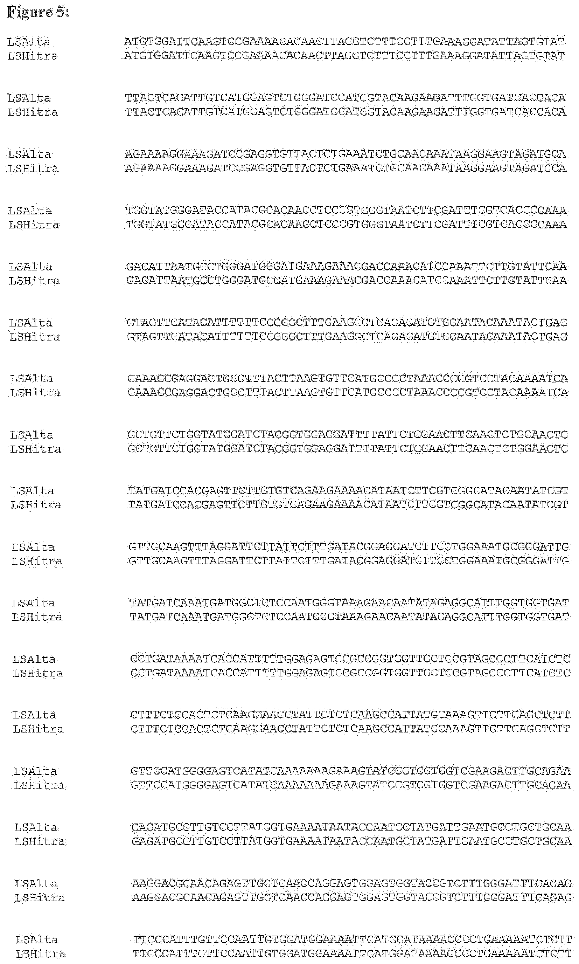

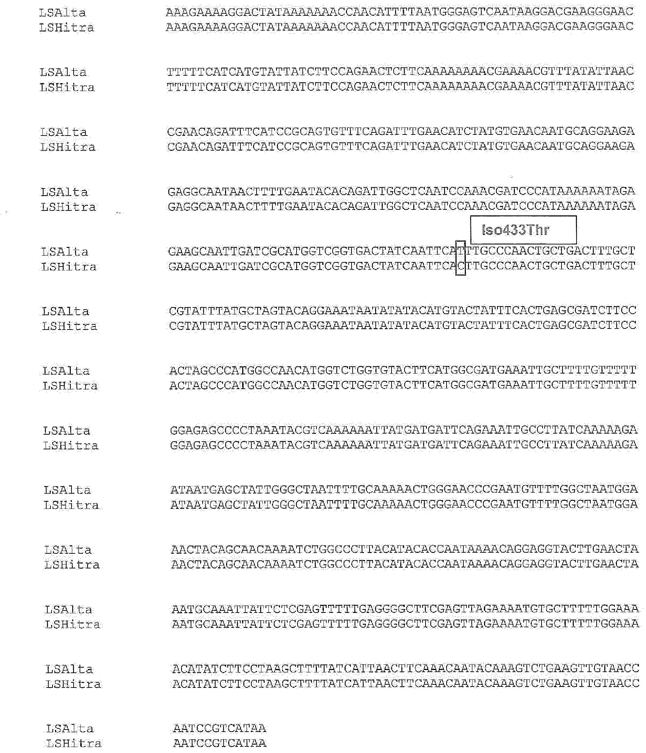

**Figure 5** Nucleotide alignment of LS-ace2 from sensitive (LSAlta) and resistant (LSHitra) sea lice strain. The change identified is boxed.

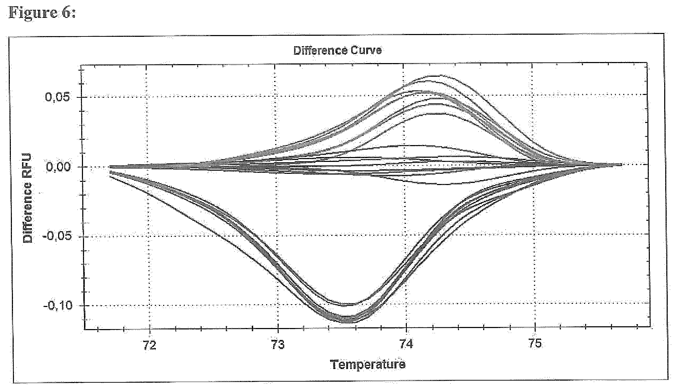

**Figure 6** High Resolution Melt (HRM) Analysis separated the samples based on differences in the sahpes of their melt curve that refelects the differences in their genotypes. The Green cluster represents samples homozygous for *Phe362Tyr* mutation, Blue cluster represents samples heterozygous for *Phe362Tyr* mutation and Red cluster represents the wild type samples without *Phe362Tyr* mutation.

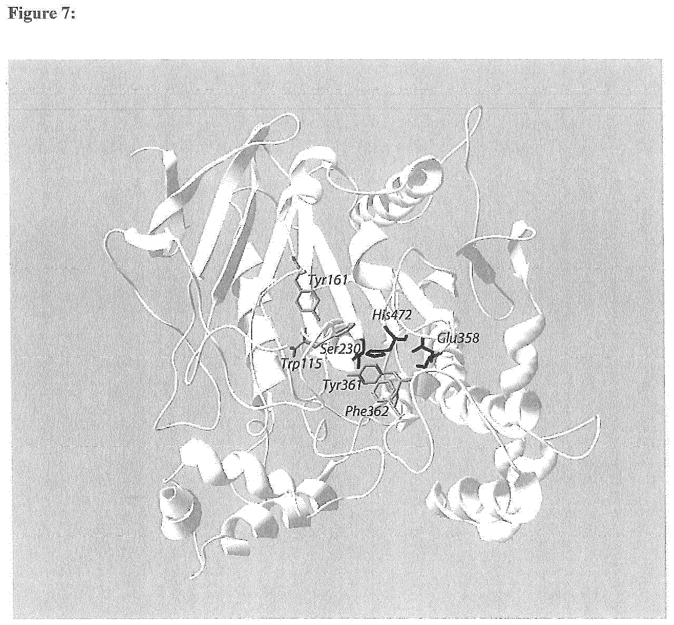

**Figure 7** 3D model of the *Lepeophtheirus salmonis* protein LS-ace1 using the ace protein from *Drosophila melanogaster* as template. The catalytic triade amino acid residues are indicated in black, and some of the "aromatic patch" amino acid residues are indicated in gray. The other amino acids are indicated as helixes, loops and sheets.

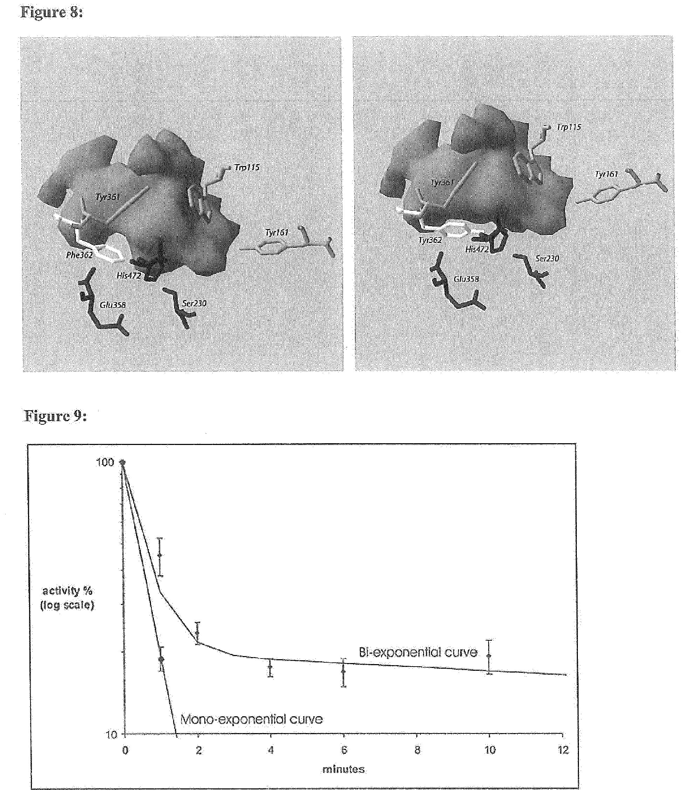

**Figure 8** Positioning of some of the important amino acids in the active gorge (solid gray). The catalytic triade amino acids are displayed in black and some of the "aromatic patch" amino acids are displayed in grey. The position of the wild type *Phe362* is displayed in the left panel (white) and the mutated type *Tyr362* is displayed in the right panel (also white). The other amino acids are omitted.

**Figure 9** Plots of inhibition curves of acetyl cholin esterase from sea lice (L. salmonis) after inhibition with azamethiphos in a bi-molecular rate assay. Protein extracts from adult female sealice were incubated with one azamethiphos concentration, and aliquots were assayed for AChE activity at different time-points. The curves displayed either a mono-exponential (•) or bi-exponential (♦) decline in activity. The standard errors for the observations are indicated.

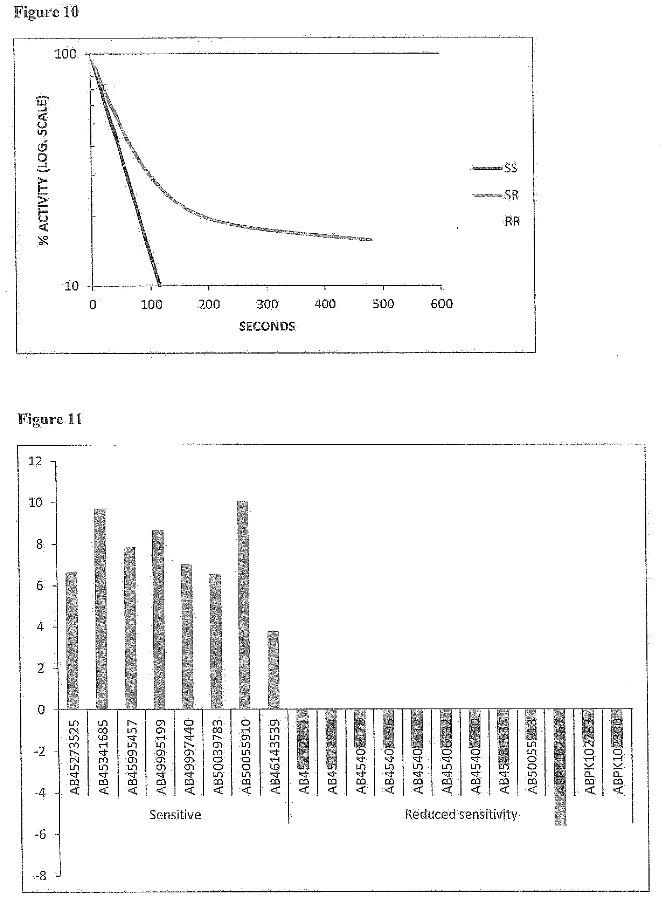

**Figure 10.** Plots of expected inhibition curves of acetyl cholin esterase from sea lice (*L. salmonis*) after inhibition with azamethiphos in a bi-molecular rate assay. SS = homozygote sensitive parasites, SR=heterozygot resistant parasites, RR= homozygote resistant parasites.

**Figure 11.** SNP-analyses correlate well with known resistance status in field strains. This figure shows the average deviation values for populations of field strains of sea lice (*L. salmonis*) analyzed using the Real-Time PCR-assay using RNA as template. Each population is encoded by an AB-number, and had been characterized as sensitive or having reduced sensitivity to organophosphate treatment based on bioassays (as indicated). The study includes a total of 327 samples from individual sea lice, and for each population between 5 and 30 samples were analyzed. The figure shows that the Real-Time PCR-assay is suited for differentiation between sensitive and resistant sea lice populations.

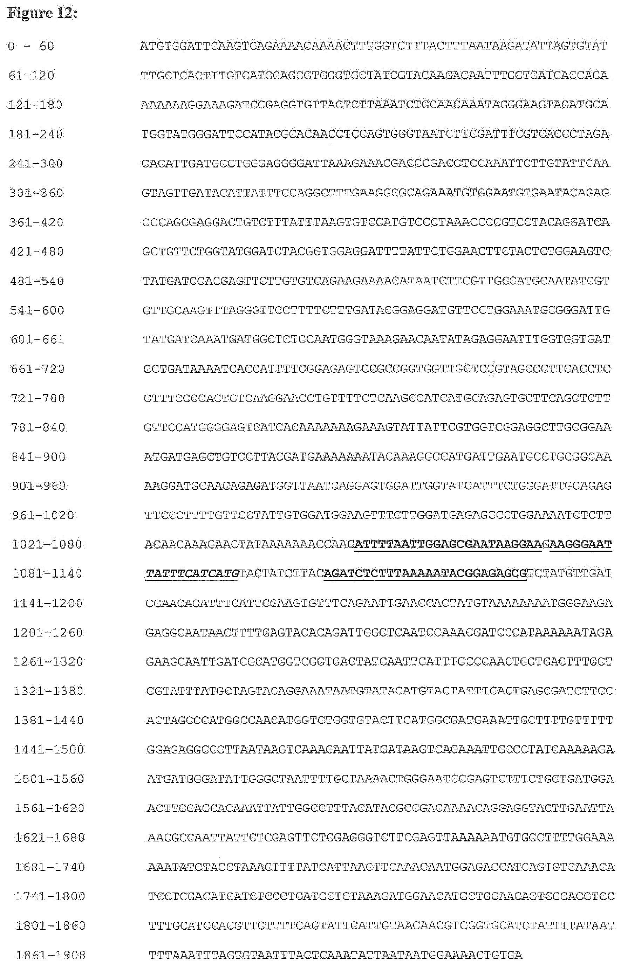

**Figure 12** Nucleotide sequences of the salmon louse (*L. salmonis*) acel gene in the sensitive strain LSAlta. The positioning of primers and probes from Table 5 are underlined and marked in bold in both sequences, and the SNP T1085A is marked in grey within the probe. Other nucleotide differences between the sensitive and resistant strain are marked in grey. The nucleotide numbering in this figure, and the positioning of the SNP T1085A is used for reference throughout the present specification.

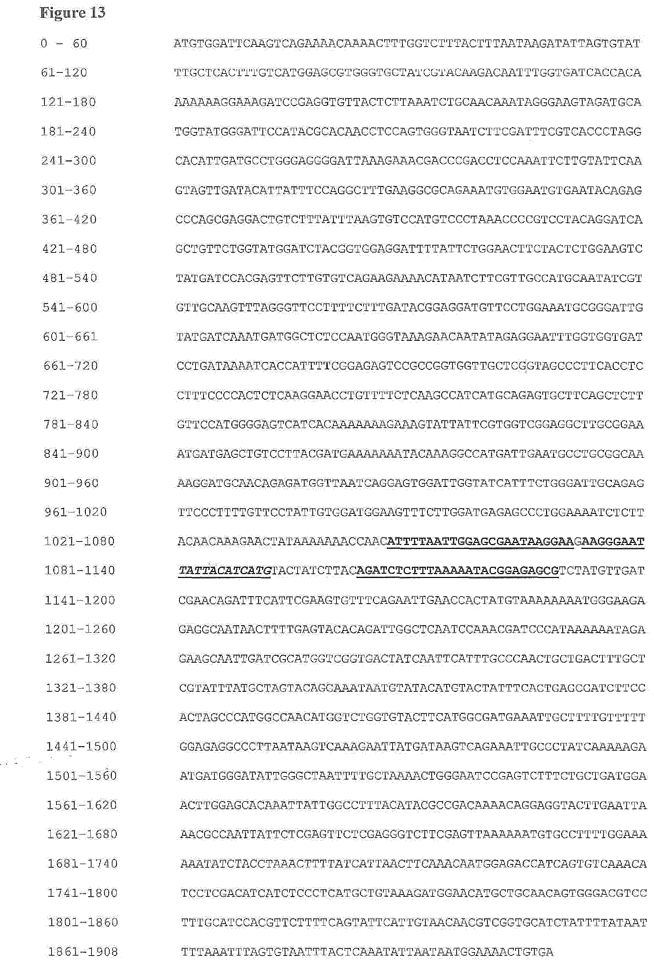

**Figure 13** Nucleotide sequences of the salmon louse (*L. salmonis*) acel gene in the resistant strain LSHitra. The positioning of primers and probes from Table 5 are underlined and marked in bold in both sequences, and the SNP T1085A is marked in grey within the probe. Other nucleotide differences between the sensitive and resistant strain are marked in grey. The nucleotide numbering in this figure, and the positioning of the SNP T1085A is used for reference throughout the present specification.

**Detailed description of the invention**

The present invention provides an in vitro method for determination of organophosphate resistance in crustaceans, including copepods, such as sea lice *(Lepeophtheirus salmonis),* based on the surprising findings that a mutation linked with resistance against organophosphate in sea lice was found in the acethylcholine esterase 1 protein coding sequence (LS-Ace1).

The present inventors have identified and sequenced two surprisingly similar genes coding for two variants of the AChE protein in *Lepeophtheirus salmonis* denoted LS-ace1 and LS-ace2. The screening for mutations revealed one mutation in the LS-ace1-gene resulting in an amino-acid change in the encoded protein which is linked to organophosphate resistance.

Several important challenges with the biological material, techniques and interpretation of the findings were encountered and overcome in the study:

1) Biological material and cultivation

Several different strains of salmon lice had to be sampled, cultivated and tested in search for the mechanism behind the azamethiphos resistance. The initial clue to reduced sensitivity was only that full-scale treatment with the product SalmosanTM comprising azamethiphos as the active ingredient had a poorer effect than expected. Reduced effect of a treatment can have several causes, as incorrect calculation of the treatment volume, incorrect calculation of the dosage, insufficient mixing of the active substance in the holding tank prior to dosing it to the cage, insufficient mixing of the stock solution in the treated cage, and/or too short holding time of the fish in the bath.The verification that the cause was insensitivity by the parasite towards azamethiphos therefore had to be done on salmon lice of that particular strain cultivated on live fish in a wet-lab. Cultivation of salmon lice in the lab can be quite challenging, as unexpected events easily can destroy the culture. As an example, oversaturation of the water with nitrogen gas destroyed some of our other salmon lice strains.

2) Enzymatic assays

The kinetic property of the mutated enzyme versus the wild type enzyme is an important clue to whether or not the enzyme is resistant towards organophosphates. An enzyme-kinetic inhibition assay technique was established in our lab some years ago (Fallang et al. 2004), but was difficult to reproduce in the current study. It is not known why this has been so difficult on these salmon lice strains. Without being bound by theory, one reason might be that the level of unspecific carboxylesterases in salmon lice is higher and more variable in salmon lice sampled during the last 2-3 years than it was when the Fallang et al. (2004) study was conducted. Unspecific esterases can cleave the substrate acetylcholine, causing interference with the assay (but not with the biological function of AChE). As the enzymatic studies on the strains in question have not given conclusive results so far, another approach to verify the correlation between phenotype and genotype had to be taken.

3) Verification of resistance

The parasites had to be tested by a bioassay method, i.e. exposure to increasing concentrations of the agent to see how much they tolerated. As no bioassay method for salmon lice sensitivity towards azamethiphos was published, an unpublished protocol had to be used, with necessary adaptations. To be on the safe side, small scale laboratory treatments with fish infected with parasites containing only the wild-type enzyme versus fish infected with parasites containing the mutation were also successfully performed and confirmed the bioassay results.

4) Technical challenges with the identification and sequencing of ace genes

The cDNA sequences of the ace genes in salmon lice were not known prior to the present invention. In an attempt to identify homologous sequences in salmon lice genome, highly conserved acetylcholinesterase sequences from other published arthropod species was used. These sequences were further used to construct primers to amplify acetylcholinesterase in salmon lice. As small changes in the nucleotide sequences may result in non-working primers, the selection of good salmon lice-specific primers was challenging. In addition, the amplification of the 5'prime and 3'prime ends of the cDNA was challenging due to a high degree of homology (90%) between the two acetylcholinesterase cDNA sequences in salmon lice disclosed herein.

5) The two ace-genes

Similar to most of the other arthropods, salmon lice has two ace genes, but it came as a surprise that the two genes were very similar to each other with an amino acid homology of 84 %. Since most of the coding region of both these genes was very much homologous to each other, it was very challenging to find out whether there were one or two genes coding for ace in salmon lice. This was only discovered when the full-length cDNA was determined. This finding was unusual when comparing to ace-genes in other arthropods. Most arthropods have two ace-genes, but the amino-acid homology is normally only 30 - 50 %. The interaction between these two very similar enzymes in salmon lice is unknown. By using quantitative PCR (qPCR), no difference in the expression in neither of the ace genes between sensitive and resistant parasites have been found.

6) Interpretations

The discovery of two ace genes quite similar to each other, the influence of the Phe362Tyr mutation on the survival of the parasite during treatment and the implication of both homozygote resistant and heterozygote resistant parasites being present in the same azamethiphos-resistant salmon lice strains complicated the interpretation of the results. It was initially belived that the parasite carried two versions of the enzyme, one azamethiphos-resistant and one sensitive version, and that resistance was caused by these being differentially regulated in sensitive and resistant parasites (Fallang et al. 2004). It was thus surpricing that the Phe362Tyr mutation in the acel gene results in organophosphate resistance and at the same time that both versions of ace were equally expressed in heterozygote parasites.

Without being bound by theory, the dynamics of the resistance is believed to be as follows:

a) Ace1 and ace2 are working together in the cholinergic synapses in the parasite's nervous system.

b) The wild-type acel is more effective in cleaving acetyl choline than the mutated ace1. The mutated acel has though a reasonable effect on cleaving acetyl choline.

c) During treatment with azamethiphos, wild-type acel and ace2 are completely inhibited. This results in death of the parasite

d) When the parasite carries the Phe362Tyr mutation, either as homozygotes or as heterozygotes, a treatment with azamethiphos results in an almost complete inhibition of ace2. In heterozygote individuals, the wild-type acel is also inhibited completely, but the mutated acel is not significantly inhibited. The residual activity of mutated acel is sufficient to maintain a basal activity of the enzyme in the nervous system of the parasite and thereby ensuring survival during the treatment. After treatment, the more effective wild-type acel and ace2 are regenerated. In homozygote resistant parasites, only ace2 is inhibited during treatment. Thus, these parasites will survive the treatment more easily.

e) The fitness of heterozygote parasites is probably not influenced significantly, as the activity of the wild-type acel and ace2 are sufficient to maintain the enzyme activity at a normal level. The fitness of homozygote resistant parasites is probably impaired as the AChE activity is reduced.

f) Without a selection pressure of azamethiphos, the homozygote resistant parasites will over time be out-competed by the sensitive parasites. The heterozygote parasites will not be out-competed and the mutated allele(s) will therefore prevail along with the wild type allele in the population for a long time after termination of the use of the substance.

Based on the above findings, the present application provides for a method for determining organophosphate resistance in crustaceans comprising the steps of detecting a mutation associated with organophosphate resistance in the acetylcholine esterase gene 1 of the crustaceans to be analyzed.

Although organophosphate resistance linked SNP presented in the experimental data was identified in the sea lice species *Lepeophtheirus salmonis,* the skilled person will acknowledge, based on the teaching herein, that the present method and the present oligonucleotides may be used to determine organophosphate resistance is crustaceans, in particular copepods, in particular copepods belonging to the family Caligidae. In particular, it is to be understood that the present method and the present oligonucleotides may be used to determine organophosphate resistance in copepods affecting farmed fish, such as fish belonging to the family *Salmonidae.* According to one embodiment, the present method and present oligonucleotides are useful for detection of organophosphate resistance in copepods selected from the group consisting of *Lepeophtheirus salmonis, Caligus elongatus,* and *Caligus rogercresseyi.*

Throughout the application, the term "sea lice" is to be understood to mean any copepod belonging to the family Caligidae. However, whenever the term "sea lice" is used in connection with the experimental data in the present application, sea lice refers to the species *Lepeophtheirus salmonis.* Throughout the present specification, the numbering of amino acids in the protein is by convention from the *Torpedo californica* protein sequence since this was the first AChE sequence to be revealed. The *T. californica* and the corresponding *L. salmonis* amino acid number is shown in **Figure 1.** Furthermore, the *L. salmonis* amino acid number is in the present specification given in *italic* while the *T. californica* amino acid number is given as normal text. In tables and figures, the names of the respective amino acids are given using their one-letter abbrevations, while in the present specification, the amino acids are given using their full name or their three-letter abbrevations. **Figure 2** presents an alignment of the amino acid sequences of the acel and ace2 proteins from a number of organisms. **Figure 3** describes the phylogenetic relationship between the LS-acel, LS-ace2 and acel and ace2 from a number of other organisms. **Figure 4** shows an alignment of the nucleotide sequences of LS-ace1 from an organophosphate-sensitive strain (LSAlta) and an organophosphate-resistant strain (LSHitra) with the mutation leading to the amino acid change indicated. **Figure 5** is a similar alignment for LS-ace2. **Table 1** lists the observed frequencies of the *Phe362Tyr* mutation in LS-ace1 in samples from the salmon lice strains not being under selection pressure by azamethiphos when collected for the study. **Table 2** lists the correponding observed frequencies of the *Phe362Tyr* mutation in samples of the salmon lice strains being under selection pressure by azamethiphos. **Table 3** lists the frequencies of the *Iso433Thr* mutation in LS-ace2 in the sea lice populations not being under selection pressure by azamethiphos. **Figure 6** illustrates clustering of samples with different genotypes based on the differences in melt curve shapes using High Resolution Melt (HRM) analysis. **Figure 7** illustrates the 3D model of the LS-ace1 protein, modeled with ace from *D. melanogaster* as template, as helixes and coils with some of the important amino acids displayed. **Figure 8** illustrates the active gorge of the LS-ace1 protein without and with the *Phe362Tyr* mutation, with some of the important amino acids displayed. **Table 4** lists the association between the frequencies of the different alleles in LS-ace1, the sensitivity of the strain determined by bioassays and the treatment efficacy of azamethiphos on two of these strains. **Figure 9** is a plot of inhibition curves of acetyl cholin esterase from salmon lice after inhibition with azamethiphos in a bi-molecular rate assay. **Table 5** show primers and probes useful in method according to one embodiment of the present application and **table 6** shows the results of using olionucleotide sequences according to the present invention in a Real-Time PCR Assay, see example 5. Finally, **table 7** list the amino acid sequences and nucleotid sequences contained in the sequence listing attached to the present application.

Based on the identification of the a SNP resulting in a mutation in the SL-Ace1 gene linked with organophosphate resistance in sea lice, a method for determination of organophosphate resistance in crustaceans, such as copepods, in particular in sea lice, e.g. *Lepeophtheirus salmonis* have been provided. More particular, a method for detection of organophosphate resistance is provided comprising the steps of detecting a mutation, such as a single nucleotide polymorphism (SNP) in the genomic DNA of a sea lice to be analyzed, in particular a mutation present in the acetylchiline esterase 1 gene (SL-ace1), wherein said sea lice is resistant to organophosphates if the mutation results in an amino acid substitution in posision 362.

The present disclosure provides a method for detection of a single nucleotide polymorphism in the SL-ace 1 gene of a crustaceans, wherein said crustaceans is resistant to organophosphate if comprising a SL-ace1 gene, wherein the nucleotide in position 1085 of SL-ace1 gene is A, wherein the numbering of said position is in accordance with the sequence depicted in SEQ ID No. 1.

It is to be understood that the detection of any mutation in the SL-ace1 gene resulting in organophosphate resistance in crustaceans, such as copepods, such as sea lice, is covered by the present application. The skilled person will e.g. acknowledge that due to the degeneration of the genetic code, different mutations may result in the substitution of the phenylalanine 362 with a tyrosin. For example, as shown in figure 4, the triplet encoding phenylalanine 362 have changed from TTC to TAC. Due to the degeneration of the genetic code, a mutation resulting in a change from TTC to TAC would likewise result in the substitution of phenylalanine with tyrosine.

In addition to the qualitative determining a mutation involved in organophosphate resistance a method for gradation of crustaceans population being susceptible of developing resistance, i.e. taking into account the composition of haplotypes that are determined within a crustaceans population is described herin.

According to the present invention, "single-nucleotide polymorphisms (SNP)" is to be understood to refer to a nucleotide sequence variation occurring when a singe nucleotide, A, T (U), C or G in the genome, or other shared sequences (e.g. RNA) or fragments thereof, differs between members of a biological species, such as between variants of *Lepeophtheirus salmonis.* SNPs may fall within coding sequences of genes, or non-coding regions of genes, or in the regions between the genes in a genome. The SNP identified according to the present invention fall within the genes encoding the acetylcholine esterase 1 gene.

Furthermore, as used herein, an "oligonucleotide sequence" or "nucleic acid sequence" is generally an oligonucleotide sequence or a nucleic acid sequence containing a SNP described herein, or one that hybridizes to such molecule such as a nucleic acid sequence with a complementary sequence.

The skilled person is well aware of the fact that nucleic acid molecules may be double-stranded or single-stranded, and that reference to a particular site of one strand refers, as well, to the corresponding site on a complementary strand. Thus, when defining a SNP position, reference to an adenine (A), a thymine (T) (uridine (U)), a cytosine (C) or a guanine (G) at a particular site on one strand of a nucleic acid is also to be understood to define a thymine (uridine), adenine, guanine, or cytosine, respectively, at the corresponding site on a complementary strand of the nucleic acid molecule. Thus, reference may be made to either strand in order to refer to a particular mutation or SNP position. The oligonucleotide probes and oligonucleotide primers according to the present invention may be designed to hybridize to either strand, and SNP detection methods disclosed herein may thus also in general target either strand.

An "isolated nucleic acid" as used herein is generally one that contains at least one of the SNPs described herein or one that hybridizes to such molecule, e.g. a nucleic acid with a complementary sequence, and is separated from most other nucleic acids present in the natural source of the nucleic acid, and is thus substantially free of other cellular material.

*Oligonucleotide probes and oligonucleotide primers*

Described herein are oligonucleotide probes and oligonucleotide primers that may be used for detection of the presence of the SNPs according to the present invention in DNA or RNA of a crustacean to be tested, and thus for determination of organophosphate resistance. The detection of nucleic acids present in a biological sample is widely applied in both human and veterinary diagnosis, wherein nucleic acids from e.g. pathogens present in biological samples are isolated and hybridized to one or more hybridizing probes or primers are used in order to amplify a target sequence.

One or more oligonucleotide probes may be constructed based on the teaching herein and used in hybridization based detection methods where upon the binding of the oligonucleotides to the target sequence enables detection of the presence of at least one of the SNPs described herein if present in the sample to be tested.

The skilled person will acknowledge that an oligonucleotide probe according to the present invention may be a fragment of DNA or RNA of variable length used herein in order to detect an SNP in a target sequence, e.g. single-stranded DNA or RNA, upon hybridization of the oligonucleotide probe to complementary sequence(s) of the said target sequence to be analyzed. The oligonucleotide probe according to the present invention may furthermore be labeled with a molecular marker in order to easily visualize that hybridization, and thus detection of the SNPs disclosed herein, have been achieved. Molecular markers commonly known to the skilled person may be used, e.g. a radiolabel, and more preferably, a luminescent molecule or a fluorescent molecule enabling the visualisation of the binding of the probe(s) to a target sequence.

A oligonucleotide probe according to the present invention is able to hybridize to another nucleic acid molecule, such as the single strand of DNA or RNA originating from a crustacean to be analysed, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a probe to target nucleic acids depends on inter alia the length of the probe and the degree of complementation, variables well known to the skilled person. A oligonucleotide probe as described herein typically comprises a nucleotide sequence which under stringent conditions hybridize to at least 8, 10, 12, 16, 20, 22, 25, 30, 40, 50 (or any other number in-between) or more consecutive nucleotides in a target nucleic acid molecule, e.g. single-stranded DNA or RNA isolated from the crustacean to be analyzed. Described herein is an oligonucleotide probe comprising about 13 to 25 consecutive nucleotides. It is to be understood that the oligonucleotide probe comprise one of the SNPs described herein or the complement thereof. New technology like specific Locked Nucleic Acid (LNA) hybridization probes allows for the use of extremely short oligonucleotide probes (You Y.; Moreira B.G.; Behlke M.A. and Owczarzy R. (2006). "Design of LNA probes that improve mismatch discrimination". Nucleic Acids Res. 34 (8): e60. doi:10.1093/nar/gkl175. PMC 1456327. PMID 16670427.) According to one embodiment, probes are provided which are selected from the group consisting of SEQ ID No. 13-15.

Described herein are oligonucleotide primers useful for amplification of any given region of a nucleotide sequence, in particular a region containing one of the SNPs described herein. An oligonucleotide primer as described herein typically comprises a nucleotide sequence at least 8, 10, 12, 16, 20, 22, 25, 30, 40, 50 (or any other number in-between) or more consecutive nucleotides. The oligonucleotide primer comprises about 18 - 25 consecutive nucleotides, more preferably about 20 nucleotides.

As used herein, the term "oligonucleotide primer" is to be understood to refer to a nucleic acid sequence suitable for directing an activity to a region of a nucleic acid, e.g. for amplification of a target nucleic acid sequence by polymerase chain reaction (PCR). Described herein are "oligonucleotide primer pairs" suitable for amplification of a region of genome material comprising the SNPs according to the present invention.

The skilled person will acknowledge that an oligonucleotide primer may be a fragment of DNA or RNA of variable length used herein in order to detect an SNP in a target sequence, e.g. single-stranded DNA or RNA, upon alignment of the oligonucleotide probe to complementary sequence(s) of the said target sequence to be analyzed. An oligonucleotide primer may furthermore be labeled with a molecular marker in order to enable visualization of the results obtained. Various molecular markers or labels are available, dependent on the SNP detection method used.

An oligonucleotide primer according to the present invention typically comprises the appropriate number of nucleotides allowing that said primer align with the target sequence to be analyzed. It is to be understood that the oligonucleotide primer according to the present invention according to one embodiment comprises the SNP described herein or the complement thereof. The primers useful in order to determine organophosphate resistant sea lice is selected from the group consisting of SEQ ID No. 9-12.

Oligonucleotide probes and oligonucleotide primers according to the present invention may be synthesizes according to methods well known to the skilled person.

Furthermore described herein is isolated nucleic acid sequences and variants or fragments thereof having at least 70% identity with the nucleic acid sequences depicted in SEQ ID NO. 2, or fragments thereof. The term "% identity" is to be understood to refer to the percentage of nucleotides that two or more sequences or fragments thereof contains, that are the same. A specified percentage of nucleotides can be referred to as e.g. 70% identity, 80% identity, 85% identity, 90% identity, 95% identity, 99% identity or more (or any number in between) over a specified region when compared and aligned for maximum correspondence. The skilled person will acknowledge that various means for comparing sequences are available. For example, one non-limiting example of a useful computer homology or identity program useful for determining the percent homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

*SNP identification methods*

Upon the identification of the SNPs associated with organophosphate resistance in crustacean, the skilled person will acknowledge that various methods commonly used in order to detect polymorphisms within a population of crustacean may be used. Both methods based on genome sequencing, hybridization and enzyme based methods are applicable for determining whether a sea louse is organophosphate resistant in accordance with the present inventions.

Various enzyme based methods are available for the skilled person, of which a number of polymerase chain reaction (PCR) based methods are available.

For example, Perkin Elmer Life Sciences provides SNP detection kit that may be used in order to determine whether a sea louse is organophosphate resistant (AcycloPrime™-FP SNP Detection). In said method, a thermostable polymerase is used which extends an oligonucleotide primer according to the present invention by one base, then ending the oligonucleotide primer one nucleotide immediately upstream of the relevant SNP position by the incorporation of fluorescent dye-labeled terminators. The identity of the base added is then determined by the increase fluorescence polarization of its linked dye (http://shop.perkinelmer.com/Content/Manuals/MAN_AcycloPrimeSNPKit.pdf). Oligonucleotide primers according to the present invention useful in such a method would thus be constructed in order to facilitate the extension of the primer by one base in the position selected from the group 8605, 9035, 13957, 14017, or 14064, relative to SEQ ID No. 1.

Another enzyme based method that may be used is restriction fragment length polymorphism (RLFP), utilizing that various, highly specific endonuclease upon digestion of the target sample, i.e. genome, results in different fragments that may be separated by gel electrophoresis.

Yet another enzyme based method that may be utilized is the flap endocuclease (FEN) method. In said method, a structure-specific endonuclease is used to cleave a three-dimensional complex formed by hybridization with the target DNA, e.g. genomic material from sea lice, and where annealing with a target sequence comprising the SNP of interest triggers cleavage by the endonuclease (Oliver, 2005, Mutat. Res., vol. 573 (1-2), pp. 103-110).

Yet another method applicable is based on the use of TaqMan® Assays (Invitrogen). In said assay the oligonucleotide primers used in order to detect an SNP is labeled in both the 5'- and the 3' end, i.e. with a fluorophore at the 5'end of the oligonucleotide primer, and a quencher at the 3'-end of the oligonucleotide primer. Upon annealing of the oligonucleotide primer with a target sequence, the Taq polymerase will extend the oligonucleotide primer and form a nascent strand, followed by degradation of the oligonucleotide primer being annealed to the target, said degradation eventually resulting in the release of the fluorophore and provide a cleavage close to the quencher. The fluorescence signal produced is proportional to the fluorophore released. Various fluorophore labels may be used, such as e.g. 6-carboxyfluorescein, tetrafluorofluorescein. As quenchers, tetramethylrhodamine or dihydrocyclopyrroloindol may be used.

Several hybridization methods for detection of SNPs are available to the skilled person, and which may be utilized in accordance with the method of the present invention. For example, the SNPs according to the present invention may be detected utilizing molecular beacon technology. Oligonucleotide primers may be synthesized comprising complementary regions at each end allowing the formation of a hairpin loop, and wherein a fluorophore is attached at one end of the oligonucleotide primer, and a quenching agent is attached to the other end, and wherein fluorescence signal is produced upon binding to a DNA target of interest, i.e. genomic material isolated from the sea louse to be analyzed. Yet another method applicable is based on DNA or RNA sequencing, which is the process of determining the precise order of nucleotides within a molecule. It includes any method or technology that is used to determine the order of the four bases (adenine, guanine, cytosine, and thymine) in a strand of DNA. The skilled person is well known with the various commonly known DNA and RNA sequencing methods that may be used, such as e.g. shotgun sequencing or bridge PCR sequencing.

Still another method applicable for detecting SNP is High Resolution Melting Analysis (HRM) enabling rapid detection of SNPs and determination of genetic variation within a population. The first step of a HRM protocol is often amplification of the region of interest, suing standard nucleotide sequence amplification techiches well known to the skilled person, and wherein the amplification is performed in the presense of a specialized double-stranded DNA binding dye being highly fluorescent when bound to dsDNA and poorly fluorescent in unbound state. This difference provides for the monitoring of the DNA amplification. After amplification, the target is gradually denatured by increasing the temperature in small increments, resulting in a characteristic melting profile. As the amplified DNA is denatured gradually, dye is released, thus resulting in a drop in fluorescence. As seen in example 3, HRM may be used to detect a single base change between otherwise identical nucleotide sequence regions. According to one embodiment, probes useful in high resolution melting analysis for the detection of organophosphate resistance in crustaceans, such as copepods, are provided, selected from the group consisting of SEQ ID No. 9-10.

*Isolation of sea lice genomic material*

The method according to the present invention may according to one embodiment involve the isolation of a biological sample from a sea lice and testing for the presence of a SNP associated with organophosphate resistance in the genome. The skilled person will acknowledge that the mutation /SNP identified according to the present invention may be detected by analyzing genomic DNA as well as genomic RNA, dependent upon the detection method used.

In order to determine whether a sea louse is organophosphate resistant in accordance with the present invention, genomic material may be isolated. Various methods for obtaining genomic material well known to the skilled person are available. The skilled person will acknowledge that any tissue (i.e. any part of the sea lice) may be used in order to extract genomic material. Furthermore, the genomic material to be analyzed according to the present invention may be obtained from sea lice of any life stages, e.g. the free swimming stages (nauplius stage I and II), the copepod stage, the pre-adult (chalimus stages 1-4), or the adult stage (adult male or adult female). According to one embodiment, tissue removed from sea lice to be tested is maintained in 70% ethanol or other conservation liquid prior to further isolation of genomic material. DNA may be extracted from the obtained tissue using commonly available DNA extraction/isolation methods, such as e.g. DNeasy DNA Tissue Kit according to the protocol of the manufacturer (http://lycofs01.lycoming.edu/~gcat-seek/protocols/DNeasy_Blood_&_Tissue_Handbook.pdf).

*SNP detection Kits*

Based on the teaching herein, the skilled person will acknowledge that, based on the identified mutation/SNP and associated sequence information disclosed herein, detection reagents can be developed and used to determine any SNP described herein individually or in combination, and that such detection reagents can be readily incorporated into kits used for SNP detection known in the art. The term "kit" as used herein in the context of SNP detection reagents are intended to cover e.g. combinations of multiple SNP detection reagents, or one or more SNP detection reagents, such as oligonucleotide probe(s) and oligonucleotide primer(s) or primer sets, arrays/microarrays of nucleic acid molecules, and beads that contain one ore more oligonucleotide probe(s), oligonucleotide primer(s) or other detection reagents useful in the method of the present invention. It is furthermore to be understood that the SNP detection reagents in a kit according to the present invention may furthermore include other components commonly included in such kits, e.g. such as various types of biochemical reagents (buffers, DNA polymerase, ligase, deoxynucleotide triphosphates for chain extension/amplification, etc.), containers, packages, substrates to which SNP detection reagents are attached., etc. necessary to carry the method according to the present invention. A kit is provided which comprises the necessary reagents to carry out one or more assays in order to detect the SNP disclosed herein according to the method of the present invention. A kit according to the present invention may preferably comprise one or more oligonucleotide probes that hybridize to a nucleic acid target molecule (i.e. genetic material) enabling detection of each target SNP position if present in the material analyzed. Multiple pairs of probes may be included in the kit to simultaneously analyze for the presence of the SNP disclosed herein at the same time. The probes contained in the kit according to the present invention may according to one embodiment be immobilized on a carrier, such as e.g. an array or a bead.

According to one embodiment, the oligonucleotide probes are suitable for the detection of the SNP T1085A.

A kit according to the present invention comprises oligonucleotide primer(s) and optionally further SNP detection reagents useful in SNP detection methods utilizing oligonucleotide primers or primer pair(s). The kit according to the present invention comprises a forward primer and a reverse primer for amplifying a region containing a a mutation in the triplet encoding amino acid 362 of the SL-ace1 gene. Said kit may furthermore optionally comprise further SNP detection reagents (enzymes and nucleotide triphosphates) necessary for conducting PCR or real time PCR. According to one embodiment, the primer pairs are suitable for the detection of the SNP T1085A. According to yet another embodiment, the kit according to the present invention comprises primer pairs suitable for detection of all the SNPs described herein.

Organophosphates as used herein refers to a group of insecticides acting on the enzyme acetylcholine esterase. Described herein is a method for detection of the organophosphate azamethiphos (S-[(6-Chloro-2-oxo[1,3]oxazolo[4,5-b]pyridin-3(2H)-yl)methyl]O,O-dimethyl phosphorothioate, CAS No. 35575-96-3). It is well known that resistance against one type of organophosphate in general provides the possibility of resistance also against all type of organophosphates; see e.g. Sevatdal S, Fallang A, Ingebrigtsen K, Horsberg TE. 2005. Monooxygenase mediated organophosphate detoxification in sea lice (Lepeophtheirus salmonis). Pest Manag Sci. 2005 Aug;61(8):772-8, Du Y, Nomura Y, Satar G, Hu Z, Nauen R, He SY, Zhorov BS, Dong K. 2013. Molecular evidence for dual organophosphate-receptor sites on a mosquito sodium channel. Proc Natl Acad Sci USA. 2013 Jul 2. [Epub ahead of print], and Zhu F, Gujar H, Gordon JR, Haynes KF, Potter MF, Palli SR. 2013. Bed bugs evolved unique adaptive strategy to resist organophosphate insecticides. Sci Rep. 2013;3:1456. doi: 10.1038/srep01456.

In health, agriculture, and government, the word "organophosphates" refers to a group of insecticides or nerve agents acting on the enzyme acetylcholinesterase. The skilled person will thus acknowledge, based on the teaching of the present disclosure, that the method described herein may be used to determine resistance towards various types of organophosphates in crustaceans, such as e.g. azametiphos, parathion, malathion, methyl parathion, chlorpyrifos, diazinon, dichlorvos, phosmet, fenitrothion, tetrachlorvinphos, and azinphos methyl.

## Examples

### Example 1: Identification and sequencing of acetylcholine esterase genes in Lepeophteirus salmonis

*Samples of salmon lice*

Salmon lice samples with different histories of sensitivity / resistance towards the organophosphate azamethiphos were collected in the field and cultivated in the laboratory at the NIVA Marine Research Station at Solbergstrand, Drøbak or at the Institute of Biology, University of Bergen.

The strains were:

| | |
|---|---|
| LSAlta: | Sampled 2010, inbred for approx. 10 generations. No history of insensivity towards azamethiphos. |
| LSBjugn: | Sampled 2008, inbred for approx. 15 generations. History of insensitivity towards azamethiphos. |
| LSGulen: | Sampled in 2004, inbred for approx. 25 generations. No history of insensivity towards azamethiphos. |
| LSHitra: | Sampled in 2011, inbred for approx. 7 generations. History of insensitivity towards azamethiphos. |
| LSHitra-s: | Subset of LSHitra where fish with parasites were treated with 100 ppb azamethiphos (Salmosan™) for 30 minutes in a 200 liter fibre-glass tank. The subset is the surviving parasites after treatment. |
| LSVeso: | Sampled in 2012 in the field (Otterøya) shortly after a full-scale treatment with azamethiphos in the farm. The site had been treated with azamethiphos at several occations prior to sampling. Only surviving parasites were sampled. |

*Total RNA extraction and cDNA synthesis*

Total RNA was extracted using RNeasy Mini kit (Qiagen, CA, USA), from female adult individuals, as per manufactures's protocol. The RNA was quantified and qualified on ND-100 Spectrophotometer (Thermo Fisher Scientific, DE, USA). First strand cDNA was synthesized from total RNA (1ug) using qScript reverse transcriptase (Quanta Biosciences, MD, USA).

*Partial cDNA fragments of LS-ace1 and LS-ace2*

Conserved sequences of AChEs were selected from other species using GenBank database, which were then compared against the sealice genome database (Viroblast; sealouse.imr.no) to detect the homologous sequences in sea lice.

*Rapid amplification of cDNA ends*

5' and 3' ends of partial cDNAs, obtained by homology blast, were amplified using Rapid amplification of cDNA ends (RACE) with sequence specific primers (mentioned below) and SMART RACE kit (Clontech, Palo Alto, CA, USA) as per manufacturer's instructions. RACE PCR was performed under conditions: 5 cycles at 94°C for 30 s, 72°C for 3 min followed by 5 cycles at 94°C for 30 s, 70°C for 30 s, 72°C for 3 min followed by 25 cycles at 94°C for 30 s, 68°C for 30 s and 72°C for 3 min.

*Primers used for 5'RACE*

LS-ace1 primer AATCCCCTCCCAGGCATCAATGTGTCTA
LS-ace 2 primer CATCCCATCCCAGGCATTAATGTCTTTG

*Primers used for 3'RACE*

LS-ace1 primer CCTATTGTGGATGGAAGTTTCTTGGATGAG

LS-ace 2 primer CAACCAGGAGGGGAGTGGTACCGTCTT

Both 5'RACE and 3'RACE PCR products were cloned using TOPO TA Cloning Kit for sequencing (Invitrogen, CA, USA) followed by isolation of plasmid DNA from positive colonies using PureLink Quick Plasmid Miniprep kit (Invitrogen, CA, USA) under manufactures's instructions. Amplicons were obtained using the plasmid DNA and TOPO vector specific primers (mentioned below) under PCR conditions: 94°C for 4 min followed by 30 cycles at 94°C for 1 min, 52°C for 1 min, 72°C for 1 min and followed by final extension at 72°C for 5 min. Amplicons were then subjected to direct sequencing using BIG Dye Terminator v3.1 cycle sequencing kit (Life technologies, Invitrogen, CA, USA) on 3130xl Genetic Analyzer (ABI Prism, Life technologies, Invitrogen, CA, USA) to obtain the full length sequence of cDNAs.

*TOPO vector specific primers used*

M13Forward primer: GTAAAACGACGGCCAG
M13Reverse primer: CAGGAAACAGCTATGAC

*Screening of full length LS-ace1 and LS-ace2*

Full length cDNAs (LS-ace1 and LS-ace2), from 3 sensitive (LSAlta) and 3 resistant (LSHitra) adult female sea lice samples, were amplified using gene specific primers (mentioned below). PCR reactions were performed using Phusion high- fidelity DNA polymerase (New England BioLabs, MA, USA) under the conditions: 98°C for 30 s, followed by 35 cycles at 98°C for 10 s, 55°C for 15 s, 72°C for 2 min followed by a final extension at 72°C for 10 min. Amplicons were then subjected to direct sequencing using BIG Dye Terminator v3.1 cycle sequencing kit (Life technologies, Invitrogen, CA, USA) on 3130x1 Genetic Analyzer (ABI Prism, Life technologies, Invitrogen, CA, USA).

*Primers used to amplify the whole cDNA*

LS-ace1 forward primer: CTCTGCTGCTACACCGACTCCTGTT

LS-ace1 reverse primer: TCGAGGATGTTTGACACTGATGGTC

LS-ace2 forward primer: TGTTTTAGATGTGGATTCAAGTCCGAA

LS-ace2 reverse primer: CGATGGATGGTACGTACGTATGAACATA

The deduced amino acid sequence of both LS-ace1 and LS-ace2 was aligned with previously published acetylcholinesterases (AChE) from other insects, nematodes, arachnida and vertebrates (**Insects**: *Liposcelis entomophila acel (Lip_ent_ace1),Liposcelis entomophila ace2 (Lip_ent_ace2), Bemisia tabaci acel (Bem_tab_ace1), Bemisia tabaci ace2 (Bem_tab_ace2), Blattella germanica ace 1 (Bla_ger_ace1), Blattella germanica ace 2 (Bla_ger_ace2), Nephotettix cincticeps ace1(Nep_cin_ace1), Nephotettix cincticeps ace2(Nep_cin_ace2), Ctenocephalides felis ace1 (Cte_fel_ace1), Ctenocephalides felis ace2 (Cte_fel_ace2), Culex pipiens ace(Cul_pip), Chilo suppressalis acel (Chi_sup_acel), Chilo suppressalis ace2 (Chi_sup_ace2), Apis mellifera acel (Api_mel_ace1), Apis mellifera ace2 (Api_mel_ace2), Cimex lectularius acel (Cim_lec_ace1), Cimex lectularius ace2 (Cim_lec_ace2), Bombyx mandarina acel (Bom_man_ace1), Bombyx mandarina ace2 (Bom_man_ace2), Bombyx mori ace1(Bom_Mor_ace1), Bombyx mori ace2(Bom_Mor_ace2), Leptinotarsa decemlineata (Lep_dec) Drosophila (Dros), Musca domestica (Mus_dom), Anopheles gambiae (Ano_gam), Aedes albopictus (Aed_alb), Culex quinquefasciatus (Cul_qui),* Nematods: *Caenorhabditis elegans acel (Celeg_ace1), Caenorhabditis elegans ace2 (Celeg_ace2), Caenorhabditis briggsae acel(Cae_bri_ace1), Caenorhabditis briggsae ace2 (Cae_bri_ace2),* Arachnida: *Tetranychus urticae ace1 (Tet_urt_ace1), Rhipicephalus decoloratus (Rhi_dec),* **Vertebrates:** *Torpedo californica (Tor_cal), Homo sapiens (Homosap)*), using ClustalW alignment.

Surprisingly, both the LS-ace proteins exhibited high degree of homology with each other (84%). This is very much different from other arthropods, where only a moderate or low level of homology has been reported between acel and ace2 genes. For example, 35.6%, 39% and 53% similarity has been reported between *Cimex lectularius, Chilo suppressalis* and *Anopheles gambiae* acel and ace2, respectively (Seong *et al,* 2012; Jiang *et al,* 2009; Weill *et al,* 2002). Besides, both the LS-ace proteins exhibited the typical feature of lacking 32 amino acids at the sequence insertion/deletion domain, which has been reported to be a common feature of all the insect acel type genes (Kim *et al,* 2006). All these observations indicate the possibility that two different forms of ace1 gene are present in sea lice unlike most of the other arthropods that have two different ace (ace1 and ace2) genes. This could possibly be due to duplication of a single ace gene in sea lice.

Moreover, both of these proteins showed highest homology to acetylcholinesterase-1 proteins from *Blattella germanica* (63%) followed by *Liposcelis entomophila* (62%), *Nephotettix cincticeps* (62%), *Cimex lectularius* (60%), *Bombyx mori* (60%), *Leptinotarsa decemlineata* (60%), *Bemisia tabaci* (59%), *Chilo suppressalis* (59%) and *Bombyx mandarina* (58%). However, only moderate level of cross homology was observed between LS-ace1 and LS-ace2 with acetylcholinesterase-2 from different species, ranging from 45% (*Chilo suppressalis*) to 33% (*Blattella germanica*). In addition, both LS-ace1 and LS-ace2 showed only 44% homology to *Drosophila melanogaster* AChE and 46% to *Torpedo californica* AChE.

The protein alignment also revealed that both LS-ace1 and LS-ace2 have the characteristic features of AChE, including the anionic choline binding site at Trp84, the three residues of the catalytic triad (Ser200, Glu327 and His440), the six cysteines potentially involved in three conserved disulphide bonds (67-94; 254-265; 402-521), the characteristic *FGESAG* motif surrounding the active serine and the 14 aromatic residues lining the active site gorge, 11 of which were well conserved and present in both LS-ace1 and LS-ace2 (Figure 2). This includes the acyl pocket residues (Trp233, Phe290 and Phe331) that accommodate the acyl moiety of the active site. In addition, the residues (Gly118, Gly119 and Ala201) that form the oxyanion hole, that helps to stabilize the tetrahedral molecule during catalysis, was also present in both the proteins. The 3 non conserved amino acids (70, 121 and 442) were absent in both the salmon lice ace proteins.

Interesting, most of the other species (including LS-ace1 and LS-ace2) have aspartic acid at 442 instead of tyrosine that is present in *Torpedo californica* (Figure 2). Moreover, Phe290 is present and Phe288 is absent in both the sequences, a characteristic property of all invertebrate AChE sequences, explaining a wider substrate specificity than vertebrate AChE (Vellom *et al.* 1993).

To construct the phylogenetic tree, the ClustalW alignment of LS-ace1 and LS-ace2 along with AChEs from 35 different species (already deposited in GenBank) was subjected to MrBayes software. The phylogenetic tree suggested that both LS-ace1 and LS-ace2 were clustered with other AChE-type1 proteins, and were clearly separated from AChE-type 2 proteins that form a separate clad in the phylogenetic tree (Figure 3).

**Example 2: Identification of missense changes in LS-ace1 and LS-ace2**

Missense changes in LS-ace1 and LS-ace2 were screened in 50 samples each from 2 sensitive (LSAlta and LSGulen) and 2 resistant populations (LSHitra, LSBjugn), by direct sequencing. These samples were not under any organophosphate treatment pressure when enrolled. In addition, 2 *L. salmonis* populations (24 samples of LSHitra-s and 20 samples of LSVeso) that survived the organophosphate treatment were also screened for these missense changes by direct sequencing. The sensitivity status of all the *L. salmonis* populations has been mentoned in detail above (Samples of salmon lice).

*Primers to amplify missense change in LS-ace 1*

LS-ace1 forward primer: GTGGATGGAAGTTTCTTGGATGAGAG
LS-ace1 reverse primer: CTCAAAAGTTATTGCCTCTCTTCCCATT

*Primers to amplify missense change in LS-ace2*

LS-ace2 forward primer: ACGAGCAAAGTCAGCAGTTG

LS-ace2 reverse primer: TTTCATCCGCAGTGTTTCAG

To determine the role of LS-ace1 and LS-ace2 with resistance, whole cDNA sequence of both the genes was screened in 3 sensitive (LSAlta) and 3 resistant (LSHitra) sea lice organisms. Sequencing revealed three single nucleotide substitutions in LS-ace1. Of these 3 changes, only one led to an amino acid change (Phenylalanine to Tyrosine at *codon 362,* which corresponds to codon 331 in the *Torpedo californica* amino acid sequence), whereas the other 2 substitutions were silent changes (Figure 4). All the three changes were identified only in the resistant salmon lice organisms.

A single change was identified in LS-ace2 that led to Isoleucine to Threonine substitution at *codon 433* (Figure 5).

Both the non-synonymous changes (*Phe362Tyr* in LS-ace1 and *Iso433Thr* in LS-ace2) were screened, by direct sequencing, in lab cultured sea lice populations, including 2 sensitive strains (LSAlta, LSGulen) and 2 resistant strains (LSBjugn and LSHitra) to determine their association with resistance against organophosphates (azamethiphos). 50 samples from each population were enrolled for screening. None of these populations were under any treatment when enrolled. Besides, 20 highly resistant samples that survived higher doses of azametiphos along with 24 first generation samples from a resistant (LSHitra) population that survived the azametiphos treatment were also screened for the two changes.

**Table 1:** Frequencies of *Phe362Tyr* in LS-ace1 in the salmon lice populations without any treatment.

| | **SENSITIVE/ RESISTANCE FOR AZAMETIPHOS** | ***Phe362Tyr* in LS-ace1** |
|---|---|---|
| **LSALTA** | SENSITIVE | 0 % |
| **LSGULEN** | SENSITIVE | 4% |
| **LSBJUGN** | MODERATELY RESISTANT | 28% |
| **LSHITRA** | RESISTANT | 66% |

**Table 2:** Frequencies of *Iso433Thr* change in LS-ace2 in the salmon lice populations without any treatment.

| | *** Phe362Tyr * in LS-ace1** |
|---|---|
| **LSVESO - SELECTED IN THE FIELD WITH AZAMETHIPHOS (N= 20)** | 95 % |
| **LSHITRA - SELECTED IN THE LAB WITH AZAMETHIPHOS (N=24)** | 100 % |

Screening revealed a higher frequency of *Phe362Tyr* in LS-ace1 in the resistant population indicating its association with resistance against azemetiphos in sea lice (Table 1). Moreover, the frequency observed was very much higher in the samples that survived the azametiphos treatment (Table 2). This observation clearly indicates an association between *Phe362Tyr* and survival of the sea lice under azametiphos treatment. Moreover, *Phe362* (Phe331 in *T. californica)* is very much conserved throughout the species as evident from the multiple sequence alignment of LS-acel and LS-ace2 with acetylcholinesterases from other species (Figure 2).

However, no such association was observed with *Iso433Thr* change in LS-ace2 (Table 3). Thus, this change was considered to be a polymorphism.

**Table 3:** Frequencies of the *Iso433Thr* change in LS-ace2 in the salmon lice populations without any treatment.

| | **SENSITIVE /RESISTANCE FOR AZAMETIPHOS** | ***Iso433Thr* in LS-ace2** |
|---|---|---|
| **LSALTA** | SENSITIVE | 80 % |
| **LSGULEN** | SENSITIVE | 86 % |
| **LSBJUGN** | MODERATELY RESISTANCE | 88 % |
| **LSHITRA** | RESISTANT | 98 % |

**Example 3: High Resolution Melt analysis (HRM)**

The *Phe362Tyr* mutation in LS-ace1 was validated by HRM, which is a simple rapid tool to screen single base changes (mutations/polymorphisms) with high sensitivity and accuracy. The methodology included the generation of specific PCR product using gene specific primers (mentioned below) and Precision Melt supermix (Bio Rad, CA, USA), as per manufactures's instructions, with a sensitive fluorescent dye (EvaGreen) that binds specifically only to double stranded DNA, followed by subjecting the amplicon to gradual increase in temperature (65°C to 95°C), which led to the denaturation of double stranded amplicon and decrease in fluorescence. This change in florescence was recorded by the C1000 Touch thermal cycler (Bio-Rad, CA, USA) as a melt curve (fluorescence versus temperature). The samples were assembled into different groups based on difference in the shapes of their melt curves.

*HRM primers for the Phe362Tyr mutation*

Forward primer: TTTTAATTGGAGCGAATAAGGA (SEQ ID No. 9)

Reverse Primer: TCTGTTCGATCAACATAGACG (SEQ ID No. 10)

High Resolution Melt analysis (HRM) was performed to validate the sequencing results and in an attempt to develop a rapid diagnostic tool for the detection of *Phe362Tyr* mutation in LS-ace1. After standardising the technique with samples of known genotypes determined by direct sequencing (wild type, heterozygous and homozygous for *Phe362Tyr* mutation), samples with unknown genotypes were run to confirm the results obtained. These were also confirmed by direct sequencing. HRM analysis could distinguish between the samples of different genotypes with high accuracy. As shown in Figure 6, the samples were very well separated based on their genotypes.

**Example 4: 3D modeling of the enzymes**

The three-dimentional structure of the enzyme was modeled using SWISS MODEL in the automated mode (*Arnold et al. 2006),* http://swissmodel.exoasy.org/. The models were generated using native acetyl choline esterase (AChE or ace) from *Torpedo californica* and *Drosophila melanogaster* as templates. The templates were generated on basis of the crystaline structure of the *T. californica* or *D. melanogaster* ace proteins, determined by X-ray diffraction (Harel et al. 2000, PDB-ID 1qo9; Harel & Sussman (unpublished), PDB-ID 2j3d). Through the process, 3D files (pdb-files) of the salmon louse protein showing the best fit with the templates from *D. melanogaster* and *T. californica* were generated.

The 3D modeling was done using SWISS MODEL (*Arnold et al. 2006),* http://swissmodel.expasy.org/. As templates, the 3D structure of the native enzyme from *Drosophila melanogaster* and *Torpedo californica* were tested. These 3D structures were based on X-ray diffraction. The protein from *L. salmonis* could be fitted to both templates, but the fits were not optimal. The QMEAN4 score (a parameter between 0 and 1 where a higher number indicates a better fit) was 0.541 when fitted against the AChE protein from *Drosophila melanogaster* and 0.614 when fitted against the protein from *Torpedo californica.* Even though the fit was slightly better with the *T. californica* protein, the results here describe the fit against the *Drosphila* protein. This because *Drosophila melanogaster* is another arthropod (insect) while *Torpedo californica* is a vertebrate (fish). The esteratic subsite of the active gorge of the acetylcholine esterase enzyme consists of a triade of three amino acids, serine (Ser), glutamic acid (Glu) and histidine (His). These are in *T. californica* located in positions Ser200, Glu327 and His440, in the LS-ace1 protein in positions *Ser230, Glu358* and *His472.* They are responsible for cleaving the acetylcholine molecule.

The catalytic triade is located almost in the center of the protein. A groove leads from the surface to the triade. Several aromatic amino acid residues (the "aromatic patch") are lining the gorge, binding the choline part of the AChE molecule and providing an exit route for the degradation products after hydrolysis. The residues considered to be of highest importance are Trp84 (*Trp115*), Tyr130 (*Tyr161*), Phe330 (*Tyr361*) and Phe331 (*Phe362*) (Pezzementi et al. 2003). One of these aromatic residues, Phe331 (*Phe362*) is also considered a part of the "histidine trap", fixing the histidine at position 440 (*His472*) in its correct position during hydrolysis of acetyl choline. Several other amino acids are also of importance for binding the substrate in the active gorge. The amino acids at the esteratic subsite, the "aromatic patch" and the anionic subsites of the enzyme (not listed) are highly conserved across species (*Ordentlich et al. 1993*).

In the figure, the positions of the three amino acids forming the catalytic triade of the enzyme, *Ser230, Glu358* and *His472* within the whole protein are indicated in black. The "aromatic patch" amino acid residues (*Trp115, Tyr161, Tyr361* and *Phe362*) are indicated in gray. The other amino acids are indicated as coils, sheets and helixes.

The mutation described here leads to a substitution of the amino acid *Phe362* to *Tyr362.* The aromatic ring of Tyr is turned approximately 50 degrees compared to the aromatic ring of Phe. Tyrosine has a hydroxyl group in the para-position, which enters the groove, decreasing the volume of the pocket from 743 $Å^3$ (wild type) to 714 $Å^3$ (mutated type) according to the model. The substitution of the nonpolar Phe with the more polar Tyr changes the polarity of the active gorge and thereby also the binding site for organophosphates, probably affecting binding of these molecules in the enzyme.

In addition to being a part of the "aromatic patch", the amino acid Phe in position 331 (*Phe362*) participates in aromatic trapping of histidine in position 440 (*His472*) in the catalytic triade. Phe331 is important for maintaining histidine in its correct position for catalysis (*Nabeshima et al. 2004).* The model estimated the shortest distance between *Phe362* and *His472* (wild type) to be 3.52 Å, and the shortest distance between *Tyr362* and *His472* (mutated type) to be 3.20 Å. So possibly, the *Phe362Tyr* mutation interferes directly with the catalytic activity of histidine in the catalytic triade by leaving less room for the acetyl choline molecule. This may affect the normal catalytic function of the enzyme, thus posing a fitness cost associated with the mutation.

The *Phe362Tyr* mutation has not been described as naturally occurring in any other species. It has though been investigated by site-directed mutagenesis in *Drosophila melanogaster* (Phe371Tyr, Drosophila amino acid sequence, *Boublik et al. 2002*). The mutation was demonstrated to decrease the sensitivity of the enzyme to the organophosphate paraoxon by 200-fold.

**Table 4:** Sensitivity tests of different strains of sea lice (*L. salmonis*) and the frequency of alleles, given as S/S (homozygote *Phe362*), R/R. (homozygote *Tyr362*) and S/R (heterozygote *Phe362* / *Tyr362*).

| **Strain** | **EC50 (mg/L)** | | **Mutation (N)** | | | **Treatment efficacy (%)** | |
|---|---|---|---|---|---|---|---|
| | | **95% CI** | **S/S** | **S/R** | **R/R** | **mean** | **95% CI** |
| LSAlta | 2.1 | 1.3-3.5 | 50 | 0 | 0 | 100 | 91-109 |
| LSBjugn | 4.5 | 1.9-11 | 36 | 13 | 1 | - | |
| LSGulen | 1.8 | 1.4-2.5 | 48 | 2 | 0 | - | |
| LSHitra | 60 | 10-350 | 22 | 18 | 10 | 50 | 39-61 |
| LSHitra-s | - | | 0 | 22 | 2 | - | |
| LSVeso | >100* | | 1 | 7 | 12 | - | |
| | | | | | | | |
| - | not tested | | | | | | |
| * | tested at only one concentration | | | | | | |

The sensitivity of the strains analyzed was tested by traditional bioassays and small scale treatments (*Helgesen et al., 2013).* The history of the strains were

LSAlta: Sampled 2010, inbred for approx. 10 generations. Fully sensitive to all agents

LSBjugn: Sampled 2008, inbred for approx. 15 generations. Moderately resistant to azamethiphos, pyrethroids and emamectin benzoate.

LSGulen: Sampled in 2004, inbred for approx. 25 generations. Fully sensitive to all agents

LSHitra: Sampled in 2011. Resistant towards azamethiphos (not tested for other agents). Inbred for approx. 7 generations.

LSHitra-s: Subset of LSHitra where fish with parasites were treated with 100 ppb azamethiphos (Salmosan™) for 30 minutes in a 200 liter fibre-glass tank. The subset is the surviving parasites after treatment.

LSVeso: Sampled in 2012 in the field (Otterøya) shortly after a full-scale treatment with azamethiphos in the farm. Only surviving parasites were sampled.

The table demonstrates a high negative correlation between the sensitivity ($EC_{50}$ values) and the frequency of S/S alleles in the samples. The correlation between $EC_{50}$s and the S/S frequency was

*Person's r (with 95 % confidence interval) = -0.9687 (- 0.9980 - -0.5947).*

Since the confidence interval does not contain the value zero, the correlation is statistically significant ($p<0.05$). As the relationship between dose and effect is non-linear, the data were also correlated using the non-parametric Spearman's p. This was also statistically significant.

$$Spearman's\ \rho = -0.9000\ (p<0.0374)$$

The small scale treatments demonstrated 100 % efficacy of 100 ppb azamethiphos towards the fully sensitive LSAlta strain, and 50 % efficacy towards the resistant LSHitra strain. This demonstrated that when the R allele was present in some samples, the treatment efficacy was lower than when it was not present. Since the mean value from one group was not included in the 95 % confidence interval of the other group, the treatment effects were statistically significantly different ($p<0.05$).

In samples collected in 2000 - 2002 from various parts of Norway, the inhibition of the salmon louse acetylcholin esterase by azamethiphos was determined (Fallang et al. 2004). The study has not yet been repeated for samples collected in 2010 - 2013.

In the previous study, the sea lice were homogenized, centrifuged and filtered. Each well in a column was prepared with aliquots of 30 μl of the sample supernatant, 0.12 mg DTNB and buffer up to 140 μl. Azamethiphos was added to the first well giving a final concentration of 1.6 μM. After 30 min of equilibration at 25°C, azamethiphos giving a final concentration of 0.01 μM, was added to wells 2-7. The same amount of buffer was added to well 8. Aliquots of 0.4 mg acetylthiocholine (substrate) were then added to the first two wells, after which the reading (405 nm, each min for 30 min) was started. The same amount of substrate was added to wells 3-6, respectively, after 1, 3, 5 and 10 min. After 20 min, substrate was added to wells 7 and 8. The final volume in each well was 200 μl. The maximum slope for each well, recorded within 10 min after addition of azamethiphos and substrate, corrected for spontaneous hydrolysis of the substrate, was used in the calculation.

The enzyme activity was calculated for each well. The enzyme activity was followed down to approx. 10% of the activity in the uninhibited sample, after which the readings became too unreliable. The percent remaining AChE activity in each single sample was plotted against the incubation time using a semi-logarithmic plot. When the curves in the semi-logarithmic plot formed a straight line, they were considered being best described by a mono-exponential function. The line for the average value was fitted using the following equation:

$$E=E_1*e^{-k_1^{(obs)}*t}$$

Curves not forming a straight line in the plot were considered being best described by a bi-exponential function, and the line for the average value was fitted using the following equation:

$$E=E_1*e^{-k_1^{(obs)}*t} + E_2*e^{-k_2^{(obs)}*t}$$

where E is the enzyme activity in the well, $E_1$ and $E_2$ are the zero-time intercepts for the exponential terms, $k_1^{(obs)}$ and $k_2^{(obs)}$ are the observed inhibition rate constants, e is the base of the natural logarithm and t is the time after addition of the inhibitor azamethiphos. The concentration-independent bimolecular rate constants, $k_{i1}$ and $k_{i2}$ were calculated by dividing $k_1^{(obs)}$ and $k_2^{(obs)}$ with the concentration of azamethiphos used in the assay.

The figure demonstrates that the enzyme activity in some samples was inhibited completely within 2 minutes (mono-exponential decline rate), most likely as a result of the acetylcholin esterase being of the wild type. In other samples, the acetyl cholin esterase was inhibited approx. 80 % by azamethiphos within 2 minutes, but the activity remained at this level (bi-exponential decline rate), most likely as a result of heterozygot resistant acetylcholin esterase. In homozygot resistant parasites, a very slow decline rate corresponding to the last part of the curve for the heterozygot parasites would have been expected. This was not seen in the samples collected in 2000 - 2002.

## Example 5: Testing of organophosphate resistance

Based on the identification of the SNP responsible for organophosphate resistance in *L. salmonis,* we developed a sensitive Real-Time PCR (TaqMan) 5'-nuclease assays for single nucleotide polymorphism (SNP) detection (Real-Time PCR SNP-assay) (Table 5), and successfully applied it to differentiate between organophosphate resistant and sensitive sea lice. Fluorogenic PCR probes, chemically modified with a minor groove binding agent to increase duplex stability, were used in single and multiplex probe closed tube formats. The probe were tested in a commercially available thermocycling fluorimeter (Applied Biosystems 7500 Real-Time PCR System) at PatoGen Analyse AS laboratory in Ålesund. The assay were used qualitatively to determine the relative amount of the SNPs in individual samples, and samples from different life stages of sea lice were use. Comparison of results obtained using this assay showed no discrepancies from results obtained by genome sequencing. The reported SNPs and the Real-Time PCR SNP-assay is ideal for the differentiating between organophosphate resistant and sensitive sea lice in the aquaculture industry.

*Sampling of sea lice*

Sea lice samples were collected from sea lice with known sensitivity-status to organophosphate. The sea lice collected in fish farms had been tested with respect to sensitivity to organophosphate by bioassays as previously described. Sea lice used for comparison between genome sequencing and the Real-Time PCR-assay had been tested using bioassays and the genome sequence were known from previous genomic sequencing performed as described in example 1 above.

In addition, sea lice were sampled from fish farms on the west coast of Norway. Sea lice were collected using forceps, and approximately 10-50 lice per site were conserved in 70% ethanol and kept at 4°C. Samples were sent refrigerated to PatoGen by express mail carrier.

*Nucleic acid purification*

In PatoGens laboratory, RNA and/or DNA were extracted from samples by methods well known to the skilled person. In short, tissue samples were transferred to Micro Collection Tubes and lysed and homogenized using QIAzol Lysis Reagent, steel beads and vigorous shaking using a TissueLyser system, followed by nucleic acid extraction using an RNAeasy kit (Qiagen) or DNAeasy kit (Qiagen), all according to the manufacturer's instructions, and by methods well known to the skilled person. Chloroform were added to the samples and shaken vigorously. After resting and centrifuging, the relevant liquid phase were collected for further extraction of either RNA or DNA by vacuum technology using a Qiagen robot system, all according to the manufacturer's instructions, and by methods well known to the skilled person. Finally, nucleic acids were eluted in 25 ml of elution buffer and used for PCR by methods well known to the skilled person.

*SNP-detection by Real-Time PCR*

The primers and probes listed in table 5 are TaqMan® MGB Probe SNP Genotyping Assays using TaqMan® 5' nuclease assay chemistry for amplifying and detecting specific SNP alleles in purified genomic DNA or RNA. The primers and probe used to identify the SNPs is listed in table 5, and were ordered from Life Technologies Corporation. One-step amplification (45 cycles) was performed on an Applied Biosystems 7500 Real-Time PCR System performed at PatoGen Analyse AS laboratory in Ålesund, all according to the manufacturer's instructions, and by methods well known to the skilled person. The assay was used qualitatively to determine the relative amount of the relevant SNPs in individual samples.

**Table 5: Primers and probes** The primers and probe used to identify the SNPs in salmon lice. Probes are both listed with IUPAC nucleotide codes for SNPs, and with the alternative nucleotides for each position as indicated. IUPAC codes used are in accordance with Nomenclature for Incompletely Specified Bases in Nucleic Acid Sequences, of which only one is relevant here: W=A or T (Nomenclature Committee of the International Union of Biochemistry (NC-IUB) (1984) http://www.chem.qmul.ac.uk/iubmb/misc/naseq.html).

| Position | Forward primer | Revers primer | Probe with IUPAC nucleotide code | Probe | Sensitive | Resistant |
|---|---|---|---|---|---|---|
| T1085A | ATTTTAATTGGAGCGAATAAGGAA | CGCTCTCCGTATTTTTAAAGAGATCT | AAGGGAATTATT**W**CATCATG | AAGGGAATTATT**T/A**CATCATG | T | A |

**Results**

The result from Real-Time PCR-assay is interpreted by looking at the deviation in Ct-values between the probes detecting the two variants of the SNP. Sensitive sea lice are of the type homozygote SS, and have a deviation higher than zero. Genetically resistant sea lice have a deviation lower than zero. Genetically resistant sea lice can be either heterozygote (RS) or homozygote (RR), and the heterozygote (RS) has a deviation between zero and -3,4, while the homozygote resistant (RR) has a deviation lower than -3,4. In addition, there is a quantitative aspect where the highly resistant strains have a lower deviation value than moderately resistant sea lice. Most likely, genetically resistant strains that have not been exposed to organophosphates for some time has a lower deviation value than genetically resistant strains that been repeatedly and newly exposed to organophosphates.

The results from the Real-Time PCR SNP-analyses showed good correlation with results obtained by genome sequencing, and correlated with the known resistance status in sea lice populations (Table 6).

Also, analyses performed on a higher number of samples field strains showed good correlation with the known resistance status and as shown in Figure 11.

The Real-Time PCR-SNP-assay showed surprisingly good results, and we believe that it will serve as a practical tool in the differentiation between organophosphate sensitive and resistant sea lice.

Thus the reported SNP and the relevant Real-Time PCR SNP-assay represent an ideal tool for differentiating between organophosphate resistant and sensitive sea lice in the aquaculture industry. Also, the prevalence of sensitive versus resistant sea lice in a population, and the deviation value for individual lice or average deviation values for populations, can be used to predict the best possible outcome of a treatment using organophosphates in the population. Also, the technique can become an important tool for optimizing sea lice treatments using organophosphates, combination treatments using organophosphate in combination with other insecticides or other measures to reduce sea lice infection pressure, and to monitor the resistant status of populations of sea lice before treatment.

**Table 6. Conclusions regarding genotype based on the Real-Time PCR-assay showed good correlation with genotyping.** The figure shows that the Real-Time PCR-assay is suited for differentiation between genotypes related to resistance towards organophosphates.

| Lice population | Parallel | Deviation | Conclusion |
|---|---|---|---|
| **SS1** | 1 | 3 | SS |
| **SS1** | 2 | 3 | SS |
| **SS2** | 1 | 12,8 | SS |
| **SS2** | 2 | 11,2 | SS |
| **SS3** | 1 | 3,1 | SS |

| | | | |
|---|---|---|---|
| **SS3** | 2 | 2,2 | SS |
| **SS4** | 1 | 4,6 | SS |
| **SS4** | 2 | 4,1 | SS |
| **SS5** | 1 | 8,8 | SS |
| **SS5** | 2 | 4,7 | SS |
| **Average** | | **5,75** | |
| **RS1** | 1 | -0,5 | RS |
| **RS1** | 2 | -0,5 | RS |
| **RS2** | 1 | -1,1 | RS |
| **RS2** | 2 | -0,9 | RS |
| **RS3** | 1 | -0,8 | RS |
| **RS3** | 2 | -0,6 | RS |
| **RS4** | 1 | -1,2 | RS |
| **RS4** | 2 | -1,4 | RS |
| **RS5** | 1 | -1,2 | RS |
| **RS5** | 2 | -1,3 | RS |
| **RS6** | 1 | -1,7 | RS |
| **RS6** | 2 | -1,9 | RS |
| **Average** | | **-1,09** | |
| **RR1** | 1 | -4,7 | RR |
| **RR1** | 2 | -4,5 | RR |
| **RR2** | 1 | -4,4 | RR |
| **RR2** | 2 | -4,5 | RR |
| **RR4** | 1 | -4,7 | RR |
| **RR4** | 2 | -4,2 | RR |
| **RR5** | 1 | -4,9 | RR |
| **RR5** | 2 | -4,6 | RR |
| **Average** | | **-4,56** | |

**Table 7: Sequences related to the invention**

| SEQ ID No. | | Description of sequence |
|---|---|---|
| 1. | SEQID No. 1 | Acetylcholinesterase gene sequence (LS-ace1) isolated from organophosphate sensitive sea lice strain (LS-Alta) |
| 2. | SEQ ID No. 2 | Acetylcholinesterase gene sequence (LS-ace1) isolated from organophosphate restistant sea lice strain (LS-Hitra) |
| 3. | SEQ ID No. 3 | Acetylcholinesterase gene sequence (LS-ace2) isolated from organophosphate sensitive sea lice strain (LS-Alta) |
| 4. | SE ID No. 4 | Acetylcholinesterase gene sequence (LS-ace2) isolated from organophosphate restistant sea lice strain (LS-Hitra) |
| 5. | SEQ ID No. 5 | Acetylcholinesterase amino acid sequence encoded by the acetylcholinesterase gene sequence (LS-acel, SEQ ID No. 1) isolated from organophosphate sensitive sea lice strain (LS-Alta) |

(continued)

| SEQ ID No. | | Description of sequence |
|---|---|---|
| 6. | SEQ ID No. 6 | Acetylcholinesterase amino acid sequence encoded by the acetylcholinesterase gene sequence (LS-acel, SEQ ID No. 2) isolated from organophosphate sensitive sea lice strain (LS-Hitra) |
| 7. | SEQ ID No. 7 | Acetylcholinesterase amino acid sequence encoded by the acetylcholinesterase gene sequence (LS-ace2, SEQ ID No. 3) isolated from organophosphate sensitive sea lice strain (LS-Alta) |
| 8. | SEQ ID No. 8 | Acetylcholinesterase amino acid sequence encoded by the acetylcholinesterase gene sequence (LS-ace2, SEQ ID No. 4) isolated from organophosphate sensitive sea lice strain (LS-Hitra) |
| 9. | SEQ ID No.9 | forward primer used in example 3 |
| 10. | SEQ ID No.10 | reverse primer used in example 3 |
| 11. | SEQ ID No.11 | Forward primer used in example 5 |
| 12. | SEQ ID No.12 | Forward primer used in example 5 |
| 13. | SEQ ID No. 13 | Probe used in example 5 in accordance with IUPAC nucleotide code |
| 14. | SEQ ID No. 14 | Probe used in example 5 (sensitive) |
| 15. | SEQ ID No. 15 | Probe used in example 5 (resistant) |

**References**

Arnold et al. (2006). Bioinformatics 22,195-201

Boublik et al. (2002). Protein engeneering 15, 43-50

Costello M. J. (2009). Journal of Fish Diseases. 32. 115-118

Denholm et al. (2002). Pest Manag Sci 58, 528-536

Fallang et al. (2004). Pest Manag Sci, 60, 1163-1170

Harel et al. (2000). Protein Science 9, 1063-1072

Helgesen et al. (2013). J Fish Dis 36, 261-272

Jiang et al. (2009). Biochem Biophys Res Comm 378, 269-272

Kim et al. (2006). Insect Mol Biol 15, 513-522

Nabeshima et al. (2004). Biochem Biophys Res Comm 313, 794-801

Ordentlich et al. (1993). J Biol Chem 268, 17083-95

Pezzementi et al. (2003). Comp Biochem Physiol B 136, 813-832

Pike A., Wadsworth S. L., (2000), Academic Press. 44. 232-337).

Seong et al. (2012). Insect Mol Biol 21, 149-159

Sevatdal S., et al. (2005), Aquaculture 244. 19-27

Torrissen et al. (2013). J Fish Dis 36, 171-194

Vellom et al. (1993). Biochemistry 32, 12-17

Weill et al. (2002). Proc Roy Soc B Biol Sci 269, 2007-2016


## Claims

1. An in vitro method for detection of organophosphate resistance in one or more sea lice of the specie *L. salmonis* comprising the steps of

    a. analyzing the genomic material of the sea lice to be analyzed,
    b. detecting a single nucleotide polymorphism (SNP) associated with organophosphate resistance in the sea lice to be analyzed, wherein

    the SNP is at the position 1085 of the ace 1 gene, wherein the numbering of the position is in accordance with the nucleic acid sequence depicted in SEQ ID NO. 2, wherein said sea lice is resistant to organophosphate if comprising an acetylcholine esterase gene 1 (ace1 gene) encoding an acetylcholine esterase protein according to SEQ ID No. 6, wherein the amino acid in position 362 is tyrosine.

2. A method according to claim 1, comprising the steps of detecting a single nucleotide polymorphism (SNP) associated with organophosphate resistance in the one or more sea lice to be analyzed, wherein said sea lice is resistant to organophosphate if comprising a SL-ace1 gene wherein the nucleotide in position 1085 of said SL-ace1 is A in the one or more sea lice to be analyzed, and wherein the numbering of said positions is in accordance with the sequence depicted in SEQ ID No. 2.

3. A method according to any of the above claims, comprising the steps of:

    a) collecting sea lice from infested fish or water samples;
    b) isolating genomic material from the any life stage of collected sea lice;
    c) determining the nucleotide polymorphic site at the positions 1085 of the acel gene in the isolated genomic material, wherein the numbering of the position is in accordance with the nucleic acid sequence depicted in SEO ID No. 2.

d) determining that said sea lice is resistant to organophosphates if the nucleotide in said position is A, or a complementary oligonucleotide thereof.

**4.** A method according to claim 3, wherein step c) is performed using a primer selected from the group consisting of SEQ ID No. 9-12.

**5.** A method according to claim 3, wherein step c) is performed using at least one probe selected from the group consisting of SEQ ID No. 13-15.

**6.** A method according to claim 3, wherein step c) comprises nucleic acid amplification.

**7.** A method according to claim 6, wherein the nucleic acid amplification is performed using polymerase chain reaction.

**8.** A method according to any of the above claims 3-7, wherein step c) is performed by contacting the genomic material of the sea lice to be analyzed with a detection reagent, and determining which nucleotide is present in position 1085 in the acel gene.

**9.** A method according to claim 3-8, wherein step c) is performed using SNP specific probe hybridization, SNP specific primer extension, SPN specific amplification, sequencing, 5' nuclease digestion, molecular beacon assay, oligonucleotide ligation assay, size analysis, single-stranded conformation polymorphism analysis, denaturing gradient gel electrophoresis.

**10.** A method according to any of the above claims, wherein the organophosphate is azamethiphos.

**11.** Acetylcholinesterase protein SL-ace1 encoding nucleotide sequence comprising the sequence depicted in SEQ ID No. 2.

**12.** An isolated oligonucleotide sequence, wherein the oligonucleotide sequence is identical or has at least 80% sequence identity with a sequence selected from the group consisting of SEQ ID No. 9-12 or a fragment thereof, and complementary sequences of SEQ ID No. 9-12 or fragments thereof, wherein said oligonucleotide sequence is an oligonucleotide primer comprising at least 16 consecutive nucleotides of a target nucleic acid molecule of SEQ ID No. 2.

**13.** An isolated oligonucleotide sequence, wherein the oligonucleotide sequence is identical or has at least 80 % sequence identity with a sequence selected from the group consisting of SEQ ID No. 13-14 and complementary sequences of SEQ ID No. 13-14.

**14.** An isolated oligonucleotide sequence, wherein said sequence is SEQ ID No. 15 or the sequence complementary to SEQ ID No. 15.

**15.** A kit for detection of organophosphate resistance in sea lice comprising at least one oligonucleotide according to claim 12-14.

**16.** The use of an isolated oligonucleotide sequence according to claims 12-14 for the detection of organophosphate resistance in one or more sea lice, wherein said sequence is capable of detecting a codon encoding tyrosine in position 362 of the acetylcholine esterase protein according to SEQ ID No. 6.

**17.** The use according to claim 16, wherein the oligonucleotide sequence is capable of detecting the SNP T1085A in SEQ ID No. 2.

## Patentansprüche

**1.** In vitro-Verfahren zum Nachweis einer Organophosphatresistenz bei einer Seelaus oder mehreren Seeläusen der Spezies *L. salmonis,* die folgenden Schritte umfassend:

a. Analysieren des genomischen Materials der zu analysierenden Seeläuse,

b. Detektieren eines Einzelnukleotidpolymorphismus (SNP), der mit der Organophosphatresistenz bei den zu analysierenden Seeläusen zusammenhängt, wobei sich der SNP an der Position 1085 des ACE 1-Gens befindet, wobei die Nummerierung der Position mit der in SEQ ID Nr. 2 dargestellten Nukleinsäuresequenz übereinstimmt, wobei die Seeläuse resistent gegen Organophosphat sind, wenn sie ein Acetylcholinesterase-Gen 1 (ACE 1-Gen), das ein Acetylcholinesterase-Protein gemäß SEQ ID Nr. 6 kodiert, umfassen, wobei es sich bei der Aminosäure an Position 362 um Tyrosin handelt.

2. Verfahren nach Anspruch 1, die Schritte umfassend, einen Einzelnukleotidpolymorphismus (SNP) nachzuweisenen, der mit der Organophosphatresistenz bei der einen zu analysierenden Seelaus oder den mehreren zu analysierenden Seeläusen zusammenhängt, wobei die Seeläuse resistent gegen Organophosphat sind, wenn sie ein Gen SL-ACE 1 umfassen, wobei es sich bei dem Nukleotid an Position 1085 des SL-ACE 1 um A bei der einen zu analysierende Seelaus oder den mehreren zu analysierenden Seeläusen handelt, und wobei die Nummerierung der Positionen mit der in SEQ ID Nr. 2 dargestellten Sequenz übereinstimmt.

3. Verfahren nach einem der vorhergehenden Ansprüche, die folgenden Schritte umfassend:

a) Einsammeln von Seeläusen aus befallenen Fischen oder Wasserproben;
b) Isolieren von genomischem Material aus jedem Lebensstadium der eingesammelten Seeläuse;
c) Bestimmen der nukleotidpolymorphen Stelle an der Positionen 1085 des Gens ACE 1 in dem isolierten genomischen Material, wobei die Nummerierung der Position mit der in SEQ ID Nr. 2 dargestellten Sequenz übereinstimmt,
d) Bestimmen, dass die Seeläuse resistent gegen Organophosphate sind, wenn es sich bei dem Nukleotid an der Position um A oder ein komplementäres Oligonukleotid von diesem handelt.

4. Verfahren nach Anspruch 3, wobei Schritt c) unter Verwendung eines Primers erfolgt, der aus der Gruppe ausgewählt ist, die aus SEQ ID Nr. 9 - 12 besteht.

5. Verfahren nach Anspruch 3, wobei der Schritt c) unter Verwendung einer Sonde erfolgt, die aus der Gruppe ausgewählt ist, die aus SEQ ID Nr. 13 - 15 besteht.

6. Verfahren nach Anspruch 3, wobei der Schritt c) eine Nukleinsäure-Amplifikation umfasst.

7. Verfahren nach Anspruch 6, wobei die Nukleinsäure-Amplifikation unter Verwendung einer Polymerasekettenreaktion erfolgt.

8. Verfahren nach einem der vorhergehenden Ansprüche 3 bis 7, wobei Schritt c) erfolgt, indem das genomische Material der zu analysierenden Seeläuse mit einem Nachweisreagens in Kontakt gebracht und bestimmt wird, welches Nukleotid an der Position 1085 im Gen ACE 1 vorhanden ist.

9. Verfahren nach Anspruch 3 bis 8, wobei Schritt c) unter Verwendung einer SNP-spezifischen Sondenhybridisierung, einer SNP-spezifischen Primerextension, einer SNP-spezifischen Amplifikation, Sequenzierung, eines 5'-Nukleaseverdaus, eines Molecular Beacon Assays, Oligonukleotid-Assays, einer Größenanalyse, einer Einzelstrang-Konformationspolymorphismusanalyse, einer Denaturierungsgradienten-Gelelektrophorese erfolgt.

10. Verfahren nach einem der vorhergehenden Ansprüche, wobei es sich bei dem Organophosphat um Azamethiphos handelt.

11. Acetylcholinesteraseprotein-SL-ACE 1 kodierende Nukleotidsequenz, welche die in SEQ ID Nr. 2 dargestellte Sequenz umfasst.

12. Isolierte Oligonukleotidsequenz, wobei die Oligonukleotidsequenz identisch oder eine mindestens 80%-ige Sequenzidentität zu einer Sequenz ist bzw. hat, die aus der Gruppe ausgewählt ist, die aus SEQ ID Nr. 9 - 12 oder einem Fragment davon und komplementären Sequenzen von SEQ ID Nr. 9 - 12 oder Fragmenten davon besteht, wobei es sich bei der Oligonukleotidsequenz um einen Oligonukleotid-Primer handelt, der mindestens 16 aufeinanderfolgende Nukleotide eines Target-Nukleinsäuremoleküls von SEQ ID Nr. 2 umfasst.

13. Isolierte Oligonukleotidsequenz, wobei die Oligonukleotidsequenz identisch oder eine mindestens 80%-ige Sequenzidentität zu einer Sequenz ist bzw. hat, die aus der Gruppe ausgewählt ist, die aus SEQ ID Nr. 13 - 14 und komplementären Sequenzen von SEQ ID Nr. 13 - 14 besteht.

**14.** Isolierte Oligonukleotidsequenz, wobei es sich bei der Sequenz um SEQ ID Nr. 15 oder die zu SEQ ID Nr. 15 komplementäre Sequenz handelt.

**15.** Set zum Nachweis einer Organophosphatresistenz bei Seeläusen, mindestens ein Oligonukleotid nach den Ansprüchen 12 bis 14 umfassend.

**16.** Verwendung einer isolierten Oligonukleotidsequenz nach den Ansprüchen 12 bis 14 zum Nachweis einer Organophosphatresistenz bei einer Seelaus oder mehreren Seeläusen, wobei die Sequenz in der Lage ist, ein codonkodierendes Tyrosin an der Position 362 des Acetylcholinesterase-Proteins gemäß SEQ ID Nr. 6 nachzuweisen.

**17.** Verwendung nach Anspruch 16, wobei die Oligonukleotidsequenz in der Lage ist, den SNP T1085A in SEQ ID Nr. 2 nachzuweisen.

## Revendications

**1.** Procédé *in vitro* de détection d'une résistance aux organophosphates chez un ou plusieurs poux de poisson de l'espèce *L. salmonis* comprenant les étapes

a. d'analyse de la matière génomique du pou de poisson à analyser,
b. de détection d'un polymorphisme mononucléotidique (SNP) associé à une résistance aux organophosphates dans le pou de poisson à analyser, dans lequel le SNP est en position 1085 du gène ace 1, dans lequel la numérotation de la position est en conformité avec la séquence d'acides nucléiques schématisée dans SEQ ID NO:2,

dans lequel ledit pou de poisson est résistant aux organophosphates s'il comprend un gène 1 d'acétylcholine estérase (gène ace1) codant une protéine acétylcholine estérase selon SEQ ID NO:6, dans lequel l'acide aminé en position 362 est la tyrosine.

**2.** Procédé selon la revendication 1, comprenant les étapes de détection d'un polymorphisme mononucléotidique (SNP) associé à une résistance aux organophosphates dans le ou les plusieurs poux de poisson à analyser, dans lequel ledit pou de poisson est résistant aux organophosphates s'il comprend un gène SL-ace1 dans lequel le nucléotide en position 1085 dans ledit SL-ace1 est A dans le ou les plusieurs poux de poisson à analyser, et dans lequel la numérotation desdites positions est conforme à la séquence schématisée dans SEQ ID NO:2.

**3.** Procédé selon l'une quelconque des revendications précédentes, comprenant les étapes :

a) de collecte de poux de poisson à partir d'échantillons de poisson ou d'eau infestés ;
b) d'isolement de la matière génomique de tout stade de vie des poux de poisson collectés ;
c) de détermination du site polymorphique nucléotidique aux positions 1085 du gène ace1 dans la matière génomique isolée, dans lequel la numérotation de la position est conforme à la séquence d'acides nucléiques schématisée dans SEQ ID NO:2 ;
d) de détermination du fait que lesdits poux de poisson sont résistants aux organophosphates si le nucléotide dans ladite position est A, ou un oligonucléotide complémentaire de celui-ci.

**4.** Procédé selon la revendication 3, dans lequel l'étape c) est réalisée à l'aide d'une amorce choisie dans le groupe constitué par SEQ ID NO:9 à 12.

**5.** Procédé selon la revendication 3, dans lequel l'étape c) est réalisée à l'aide d'au moins une sonde choisie dans le groupe constitué par SEQ ID NO:13 à 15.

**6.** Procédé selon la revendication 3, dans lequel l'étape c) comprend une amplification d'acide nucléique.

**7.** Procédé selon la revendication 6, dans lequel l'amplification d'acide nucléique est réalisée à l'aide d'une amplification en chaîne par polymérase.

**8.** Procédé selon l'une quelconque des revendications 3 à 7 ci-dessus, dans lequel l'étape c) est réalisée par mise en contact de la matière génomique des poux de poisson à analyser avec un réactif de détection, et détermination de l'identité du nucléotide présent en position 1085 dans le gène ace1.

**9.** Procédé selon les revendications 3 à 8, dans lequel l'étape c) est réalisée à l'aide d'une hybridation de sonde spécifique de SNP, une extension d'amorce spécifique de SNP, une amplification spécifique de SNP, un séquençage, une digestion par une nucléase en 5', un dosage par balise moléculaire, un dosage par ligature d'oligonucléotides, une analyse de taille, une analyse de polymorphisme de conformation monobrin, une électrophorèse sur gradient de gel dénaturant.

**10.** Procédé selon l'une quelconque des revendications ci-dessus, dans lequel l'organophosphate est l'azaméthiphos.

**11.** Séquence nucléotidique codant la protéine acétylcholinestérase SL-ace1 comprenant la séquence schématisée dans SEQ ID NO:2.

**12.** Séquence oligonucléotidique isolée, dans laquelle la séquence oligonucléotidique est identique ou a au moins 80% d'identité de séquence avec une séquence choisie dans le groupe constitué par SEQ ID NO:9 à 12 ou un fragment de celles-ci, et
des séquences complémentaires de SEQ ID NO:9 à 12 ou des fragments de celles-ci, dans laquelle ladite séquence oligonucléotidique est une amorce oligonucléotidique comprenant au moins 16 nucléotides consécutifs d'une molécule d'acide nucléique cible de SEQ ID NO:2.

**13.** Séquence oligonucléotidique isolée, dans laquelle la séquence oligonucléotidique est identique ou a au moins 80% d'identité de séquence avec une séquence choisie dans le groupe constitué par SEQ ID NO:13 et 14 et des séquences complémentaires de SEQ ID NO:13 et 14.

**14.** Séquence oligonucléotidique isolée, dans laquelle ladite séquence est SEQ ID NO:15 ou la séquence complémentaire de SEQ ID NO:15.

**15.** Kit de détection d'une résistance aux organophosphates dans des poux de poisson comprenant au moins un oligonucléotide selon les revendications 12 à 14.

**16.** Utilisation d'une séquence oligonucléotidique isolée selon les revendications 12 à 14, pour la détection d'une résistance aux organophosphates dans un ou plusieurs poux de poisson, dans laquelle ladite séquence est capable de détecter un codon codant la tyrosine en position 362 de la protéine acétylcholine estérase selon SEQ ID NO:6.

**17.** Utilisation selon la revendication 16, dans laquelle la séquence oligonucléotidique est capable de détecter le SNP T1085A dans SEQ ID N0:2.

**Figure 1:**

```
                       -------------------------------------------------------00000
                       -------------------------------------------------------00000
                       -------------------------------------------------------12345
TC-ace1,               -------------------------------------------------------DDHSE
LS-ace1,               -------------------------MWIQVRKQNFGLYFNKILVYLLTLSWSVGAIVQDN
                       -------------------------00000000000000000000000000000000000
                       -------------------------00000000011111111112222222222333333
                       -------------------------12345678901234567890123456789012345

                       000000000000-00000000000000000000000000000000000000000000000
                       000011111111-11222222222233333333334444444444555555555566666
                       678901234567-89012345678901234567890123456789012345678901234
TC-ace1,             6 LLVNTKSGKVMG-TRVPVLSSHISAFLGIPFAEPPVGNMRFRRPEPKKPWSGVWNASTYP
LS-ace1,            36 LVITTKKGKIRGVTLKSATNREVDAWYGIPYAQPPVGNLRFRHPRHIDAWEGIKETTRPP
                       000000000000000000000000000000000000000000000000000000000000
                       333344444444445555555555666666666677777777778888888888999999
                       678901234567890123456789012345678901234567890123456789012345

                       000000000000000000000000000000000000111111111111111111111111
                       666667777777777888888888899999999990000000000111111111122222
                       567890123456789012345678901234567890123456789012345678901234
TC-ace1,            65 NNCQQYVDEQFPGFSGSEMWNPNREMSEDCLYLNIWVPSPRPKSTTVMVWIYGGGFYSGS
LS-ace1,            96 NSCIQVVDTLFPGFEGAEMWNVNTEPSEDCLYLSVHVPKPRPTGSAVLVWIYGGGFYSGT
                       000011111111111111111111111111111111111111111111111111111111
                       999900000000001111111111222222222233333333334444444444555555
                       678901234567890123456789012345678901234567890123456789012345

                       111111111111111111111111111111111111111111111111111111111111
                       222223333333333444444444455555555556666666666777777777788888
                       567890123456789012345678901234567890123456789012345678901234
TC-ace1,           125 STLDVYNGKYLAYTEEVVLVSLSYRVGAFGFLALHGSQEAPGNVGLLDQRMALQWVHDNI
LS-ace1,           156 STLEVYDPRVLVSEENIIFVAMQYRVASLGFL-FFDTEDVPGNAGLYDQMMALQWVKNNI
                       11111111111111111111111111111111-111111111111122222222222222
                       55556666666666777777777788888888-889999999999900000000001111
                       67890123456789012345678901234567-890123456789012345678901234

                       111111111111111222222222222222222222222222222222222222222222
                       888889999999999000000000011111111112222222222333333333344444
                       567890123456789012345678901234567890123456789012345678901234
TC-ace1,           185 QFFGGDPKTVTIFGESAGGASVGMHILSPGSRDLFRRAILQSGSPNCPWASVSVAEGRRR
LS-ace1,           215 EEFGGDPDKITIFGESAGGCSVALHLLSPLSRNLFSQAIMQSASALVPWGVITKKESIIR
                       222222222222222222222222222222222222222222222222222222222222
                       111112222222222333333333344444444445555555555666666666677777
                       567890123456789012345678901234567890123456789012345678901234

                       222222222222--222222222222222222222222222222222222222222222333
                       444445555555--55666666666677777777778888888888999999999999000
                       567890123456--7890123456789012345678901234567890123456789012
TC-ace1,           245 AVELGRNLNCNLN--SDEELIHCLREKKPQELIDVEWNVLPFDSIFRFSFVPVIDGEFFP
LS-ace1,           275 GRRLAEMMSCPYDEKNTKAMIECLRQKDATEMVNQEW-IGIISGIAEFPFVPIVDGSFLD
                       22222222222222222222222233333333333-333333333333333333333333
                       77777888888888899999999990000000000011-111111111222222222223333
                       56789012345678901234567890123456789011-2345678901234567890123

                       33333333333333333333333333333333333333333333-333333333333333
                       00000001111111111222222222233333333334444444-444555555555566
                       34567890123456789012345678901234567890123456-789012345678901
TC-ace1,           303 TSLESMLNSGNFKKTQILLGVNKDEGSFFLLYGAPGFSKDSESK-ISREDFMSGVKLSVP
LS-ace1,           334 ESPGKSLTTKNYKKTNILIGANKEEGNYFIMYYLTDLFKNTESVYYVDRTDFIRSVSELNH
                       333333333333333333333333333333333333333333333333333333333333
                       333333444444444455555555556666666666777777777788888888889999
                       456789012345678901234567890123456789012345678901234567890123

                       333333333333333333333333333333333333333444444444444444444444
                       666666667777777777888888888899999999990000000000111111111122
                       234567890123456789012345678901234567890123456789012345678901
TC-ace1,           362 HANDLGLDAVTLQYTDWMDDNNGIKNRDGLDDIVGDHNVICPLMHFVNKYTKFGNGTYLY
LS-ace1,           394 YVKKMGREAITFEYTDWLNPNDPIKNREAIDRMVGDYQFICPTADFARIYASTGNNVYMY
                       333333444444444444444444444444444444444444444444444444444444
                       999999000000000011111111112222222222333333333344444444445555
```

```
                         456789012345678901234567890123456789012345678901234567890123
                         444444444444444444444444444444444444444444444444444444444444
                         222222223333333333444444444455555555556666666666777777777788
                         234567890123456789012345678901234567890123456789012345678901
TC-ace1,             422 FFNHRASNLVWPEWMGVIHGYEIEFVFGLPLVKELNYTAEEEALSRRIMHYWATFAKTGN
LS-ace1,             454 YFTERSSTSPWPTWSGVLHGDEIAFVFGEALNKSKNYDKSEIALSKRMMGYWANFAKTGN
                         444444444444444444444444444444444444444444444445555555555555
                         555555666666666677777777778888888888999999999900000000001111
                         456789012345678901234567890123456789012345678901234567890123

                         44---4444444444444445555555555555555555555555555555555555555
                         88---8888889999999999000000000011111111112222222222333333333
                         23---4567890123456789012345678901234567890123456789012345678
TC-ace1,             482 PN---EPHSQESKWPLFTTKEQKFIDLNTEPMKVHQRLRVQMCVFWNQFLPKLLNATACD
LS-ace1,             514 PSLSADGTWSTNYWPLHTPTKQEVLELNANYSRVLEGLRVKKCAFWKKYLPKLLSLTSNN
                         555555555555555555555555555555555555555555555555555555555555
                         111111222222222233333333334444444444555555555566666666667777
                         456789012345678901234567890123456789012345678901234567890123

                         55555-------------------------------------------------------
                         34444-------------------------------------------------------
                         90123-------------------------------------------------------
TC-ace1,             539 GELSS-------------------------------------------------------
LS-ace1,             574 GDHQCQTSSTSSPSCCKDGTCCNSGTSFASTFFSVFIVTTSVHLFYNFKFSVIYSNINNG
                         555555555555555555555555556666666666666666666666666666666666
                         777777888888888899999999990000000000111111111122222222223333
                         456789012345678901234567890123456789012345678901234567890123

                         --
                         --
                         --
TC-ace1,                 --
LS-ace1,             634 KL
                         66
                         33
                         45
```

## Figure 2:

```
Bla_ger_ace1      ------------------------------------------------------------
Nep_cin_ace1      ------------------------------------------------------------
Lip_ent_ace1      ------------------------------------------------------------
Cim_lec_ace1      ------------------------------------------------------------
Bom_man_ace1      ------------------------------------------------------------
Bom_Mor_ace1      ------------------------------------------------------------
Chi_sup_ace1      ------------------------------------------------------------
Bem_tab_ace1      ------------------------------------------------------------
Api_mel_ace1      ------------------------------------------------------------
Cul_pip           ----------------------------------------------MEIRGLITRLLGPC
Cul_qui           ----------------------------------------------MEIRGLITRLLGPC
Aed_alb           ----------------------------------------------MEIRGLITRLLGPC
Ano_gam           ---------------------------------------MEIRGLLMGRLRLGRRMVPLG
Cte_fel_ace1      ------------------------------------------------------------
LS_ace1           ------------------------------------------------------------
LS_ace2           ------------------------------------------------------------
Tet_urt_ace1      ------------------------------------------------------------
Rhi_dec           ------------------------------------------------------------
Celeg_ace1        ------------------------------------------------------------
Cae_bri_ace1      ------------------------------------------------------------
Homosap           ------------------------------------------------------------
Tor_cal           ------------------------------------------------------------
Bom_man_ace2      ------------------------------------------------------------
Bom_Mor_ace2      ------------------------------------------------------------
Chi_sup_ace2      ------------------------------------------------------------
Lep_dec           ------------------------------------------------------------
Nep_cin_ace2      ------------------------------------------------------------
Cim_lec_ace2      ------------------------------------------------------------
Lip_ent_ace2      ------------------------------------------------------------
Bla_ger_ace2      --------------------------------------------------------MATQ
Api_mel_ace2      ------------------------------------------------------------
Bem_tab_ace2      ------------------------------------MATPATTRVVTQRQRREPCPRGTK
Dros              ------------------------------------------MAISCRQSRVLPMSLPLP
Mus_dom           MARSVRTPISPSSSSSSSRSSWSSPSSSFYSLLSSFKASLTRPSSSSSVAHHLAARNNDI
Cte_fel_ace2      ----------------------------------------------------------MY
Celeg_ace2        ------------------------------------------------------------
Cae_bri_ace2      ------------------------------------------------------------

Bla_ger_ace1      -------MDVGDLVGGLSVGSGDTDPLRDIHIREHVIKDHVHSHHGSG------------
Nep_cin_ace1      ------------------------------------------------------------
Lip_ent_ace1      ------------------------------------------------------------
Cim_lec_ace1      ------------------------------------------------------------
Bom_man_ace1      --MRVVLAALTALAARTLAGPHEHRARHHAPAP----------PQPYH------------
Bom_Mor_ace1      --MRVVLAALTALAARTLAGPHEHRARHHAPAP----------PQPYH------------
Chi_sup_ace1      --MRVVLAALTALAARALAGPHEHRARHHAPDHPLHFPAPANPAQPYR------------
Bem_tab_ace1      ------------------------------------------------------------
Api_mel_ace1      ------------------------------------------------------------
Cul_pip           HLRHLILCSLGLYSILVKSVHCRHHDIGSSVAHQLG--SKYSQ-----------------
Cul_qui           HLRHLILCSLGLYSILVQSVHCRHHDIGSSVAHQLG--SKYSQ-----------------
Aed_alb           HIRHLILCSLGIYSILVQSVHCRHHDIGSSTAHQLG--SKYAQ-----------------
Ano_gam           LLGVTALLLILPPSALVQGRHHELNNGAAIGSHQLS--AAAGVGLSSQSAQSGSLASGVM
Cte_fel_ace1      ---MQCGALLSLLAVVAQASRRREAPAAPAPAYADFDEQMG-------------------
LS_ace1           ------------------------------------------------------------
LS_ace2           ------------------------------------------------------------
Tet_urt_ace1      MVPMFNHNINHFNNVIVTTLTHHQYTNSRCNGNNNVIKSITN------------------
Rhi_dec           ------------------------------------------------------------
Celeg_ace1        ------------------------------------------------------------
Cae_bri_ace1      ------------------------------------------------------------
Homosap           ------------------------------------------------------------
Tor_cal           ------------------------------------------------------------
Bom_man_ace2      -MINYGKIVFTKLLLCVLISGTFA------------------------------------
Bom_Mor_ace2      -MINYGKIVFTKLLLCVLMSGTFA------------------------------------
Chi_sup_ace2      -MSRNIKIVFTKLLLCFFVSGAFG------------------------------------
Lep_dec           -----MGQLSILCLFVTVCASVCG------------------------------------
Nep_cin_ace2      -MARLRFSTLSLSLLVAVATQPQP------------------------------------
Cim_lec_ace2      -MSPWIGCVLAVVIGLAQGRPYSG------------------------------------
Lip_ent_ace2      ----MSRYSCTVIVISILVGHVWG------------------------------------
Bla_ger_ace2      CLLILAGCICTAMAIGAGSKPHFN------------------------------------
Api_mel_ace2      --MTTRILLLFFLLLSSCTRPSRGNA----------------------------------
Bem_tab_ace2      ELWPLALTLCAVLGLAAGHPSHRKHHG---------------------------------
Dros              LTIPLPLVLVLSLHLSGVCG----------------------------------------
Mus_dom           CRGLFATLVILLRMSALTSA----------------------------------------
```

```
Cte_fel_ace2     HLTSILVFSLLSISTRVDGS----------------------------------------
Celeg_ace2       ------------------------------------------------------------
Cae_bri_ace2     ------------------------------------------------------------

Bla_ger_ace1     --------------------GGDPSYMSRGDPSIVGRGEPMDMGRRDPPVDFGRRHSIDM
Nep_cin_ace1     --------------------------MVWLCPVYVLVCTMTCTCGFAATLRHARHQAAPP
Lip_ent_ace1     ----------------------------------MEYGERSSDILADGSLDLSRKDRVEF
Cim_lec_ace1     ------------------------------------------------------------
Bom_man_ace1     --------------------GHGEAVRYNPELDTILPRLEDHETSSKRASDAETSSKRTK
Bom_Mor_ace1     --------------------GHGEAVRYNPELDTILPRLEDHETSSKRASDAETSSKRTK
Chi_sup_ace1     --------------------GHGEAARYNPELDTILPRVEDHETSSKRAKLDDAETSSKR
Bem_tab_ace1     ------------------------MDFDHLPLRASPETDQLRNPRHGFGFRDGISDEGLN
Api_mel_ace1     -----------------------MPRNRNSLTKFEVLMVLSLTVLVQLDVCRCTPSHRSR
Cul_pip          ----------SSSLSSSSQSSSSLAEEATLNKDSDAFFTPYIGHGDSVRIVDAELGTLER
Cul_qui          ----------SSSLSSSSQSSSSLAEEATLNKDSDAFFTPYIGHGDSVRIVDAELGTLER
Aed_alb          ----------SSSLSSSSQSSSSLVEEPALNKDSDAFFTPYIGHGDSVRIVDAELGTLER
Ano_gam          SSVPAAGASSSSSSSLLSSSAEDDVARITLSKDADAFFTPYIGHGESVRIIDAELGTLEH
Cte_fel_ace1     ----------------------------PELPDEHGIYSPYMGHGESVRVLDPDLGTLEL
LS_ace1          --------------------------------------------------------MWIQ
LS_ace2          --------------------------------------------------------MWIQ
Tet_urt_ace1     --------------------SILKSVTVFTVKTLWNHLLVPIVVILLFQSSANVFSSALP
Rhi_dec          ----------------------------------------MLHENLASCHLTLLALLVCG
Celeg_ace1       ------------------------------------------------------------
Cae_bri_ace1     ------------------------------------------------------------
Homosap          -----------------------------------------------------MRPPQCL
Tor_cal          ------------------------------------------------------------
Bom_man_ace2     -------------------------------RSWANHHDTTTSTTQTTPTT---------
Bom_Mor_ace2     -------------------------------RSWANHHDTTTSTTQTTPTT---------
Chi_sup_ace2     -------------------------------RSWANHHDTTTSTTQTTPTT---------
Lep_dec          -------------------------------YSWP---------SDETTTK---------
Nep_cin_ace2     -------------------------------STPRTLHSNDHNHGFLNEHKHSHAHAYKS
Cim_lec_ace2     -------------------------------PTSERTHTPPR------------------
Lip_ent_ace2     -------------------------------APSWSRESLSLIN----------------
Bla_ger_ace2     -------------------------------SSSASSTPHSTIGGGSSPNT---------
Api_mel_ace2     ------------------------VPSSQRG-----------------------------
Bem_tab_ace2     -------------QTSHLNNHKNHNFDEDHYASFAAYQEPSNKTGTSNGDRKFKAGEEER
Dros             ------------------------------------------------------------
Mus_dom          ------------------------------------------------------------
Cte_fel_ace2     ------------------------------------------------------------
Celeg_ace2       -----------------------------------------------------MRAPVIG
Cae_bri_ace2     -----------------------------------------------------MRAPVNR

Bla_ger_ace1     DRVGHGGDPLDLAHESRMSEEGSGHGMQDDPLVIDTKKGKVRGITLTAATGKLVDAWLGI
Nep_cin_ace1     PDRLSPLDIPSRYEMDMTDDVVEDAPAPEDPLLIHTLKGKVRGQTMTAATGKLVDAWLGI
Lip_ent_ace1     GSRDKDIE---REREAKDDYHHEETKAEEDPLEIMTMKGKVRGTTLVAGNGKLVDAWLGI
Cim_lec_ace1     --------MRWLLVATVVLGAGASPPLDDDPLIIETDKGRVRGITIAASTGKLVDAWLGI
Bom_man_ace1     YEERFYSNHERAAELMADEPVSEKGDEED-PLVIRTRKGKVRGITLTSATGKKVDAWFGI
Bom_Mor_ace1     YEERFYSNHERAAELMADEPVSEKGDEED-PLVIRTRKGKVRGITLTSATGKKVDAWFGI
Chi_sup_ace1     DDDRFYSNHERIDDEGFLADEPQPGPEDDDPLIVRTRKGRVRGITLTAATGKKVDAWFGI
Bem_tab_ace1     FRHSEHEGERSKYKGAEAEEEMMADEGDNDPLVVQTTKGKVRGTTLTAATGKQVDAWLGI
Api_mel_ace1     HHADMSYEDTMSFRSGEELPRPAALNSNDDPLIVQTRKGKVRGKTMTATTGKEVDAWFGI
Cul_pip          EHIHSTTTRRRGLTRR----ESSSDATDSDPLVITTDKGKIRGTTLEAPSGKKVDAWMGI
Cul_qui          EHIHSTTTRRRGLTRR----ESSSDATDSDPLVITTDKGKIRGTTLEAPSGKKVDAWMGI
Aed_alb          EHVHSTTTRRRGLTRR----ESSSDANDNDPLLITTDKGKVRGLTLEAPSGKKVDAWLGI
Ano_gam          VHSG-ATPRRRGLTRR----ESNSDANDNDPLVVNTDKGRIRGITVDAPSGKKVDVWLGI
Cte_fel_ace1     GEEREAKRHREYRSSTGKTRRESSRHPEDDPLVVRTNKGLVRGTTLTSSTGKHVDAWLGI
LS_ace1          VRKQNFGLYFNKILVYLLTLSWSVGAIVQDNLVITTKKGKIRGVTLKSATNREVDAWYGI
LS_ace2          VRKHNLGLSFERILVYLLTLSWSLGSIVQEDLVITTRKGKIRGVTLKSATNKEVDAWYGI
Tet_urt_ace1     HSEINSLYADGPSFSSSFNSEHHHHHHHNDPLVVLTKKGYVRGRSVVSPTGKPVDAFLGI
Rhi_dec          GVVLRCLSIEPEEDAPNRVEDQDADEDHVETVVVQTAKGLVKGFIARSPLGKTVRVLYGI
Celeg_ace1       ------------MRNSLLFFIFLPSTILAVDLIHLHDGSPLFGEEVLSQTGKPLTRFQGI
Cae_bri_ace1     ------------MRYSLLFFIFLPCVITAVDLIHLHDGSPLFGEEVLSQTGKPLTRFLGI
Homosap          LHTPSLASPLLLLLLWLLGGGVGAEGREDAELLVTVRGGRLRGIRLKTPGG-PVSAFLGI
Tor_cal          --------------------------DDHSELLVNTKSGKVMGTRVPVLSS-HISAFLGI
Bom_man_ace2     --------------------SPVPKNIHNDPLIVETKSGLIKGY-AKTVMGREVHIFTGI
Bom_Mor_ace2     --------------------SPVPKNIHNDPLIVETKSGLIKGY-AKTVMGREVHIFTGI
Chi_sup_ace2     --------------------SPLPKNIHSDPLIVETKSGLIKGY-AKTVMGREVHIFTGI
Lep_dec          --------------------PSQFKDFHTDPLVVETTSGLVRGY-SKTVLGREVHVFTGI
Nep_cin_ace2     HDRAHNTHAQFAEATGPASTPSGGTPKHGDPLIVETTSGLVRGL-SKTVLGREVHVFTGI
Cim_lec_ace2     -------------------------AEDLDPMIVKTRSGLVRGM-AKSVLDREVHVFTGI
Lip_ent_ace2     --------------------TTASRDYHMDPLVVETTTGLVKGV-SKMVLDREVHVFYGI
Bla_ger_ace2     --------------------NPGKLDYHDDPLVVETQSGLVRGS-ARVVLGKEVHIFTGI
Api_mel_ace2     -------------------------NVHNDPLVVETTSGLVRGF-PRTVLDKEVHVFYGI
Bem_tab_ace2     KYIFDGQRRFKSLEHERHTHADYEESFVNDPLVVRTKSGLIRGV-EKQVMGHKVHVFTGI
Dros             ---------------------------VIDRLVVQTSSGPVRGR-SVTVQGREVHVYTGI
Mus_dom          ---------------------------MTDHLTVQTTSGPVRGR-SVTVQGRDVHVFTGI
```

```
Cte_fel_ace2      ---------------------------HQDPLTVTTTSGLIRGR-ARIVLGREVHVFTGI
Celeg_ace2        RHLTYHVFCQFALVTLFIVRRIEPRSIVRGDHVVHTPLGTIRGV-GQTFDGAKVSAFLGV
Cae_bri_ace2      RHLIYHVFYRFLLLAALFTKYVETRSIVRGDHVVHTPLGTIRGV-GQTFDGAKVSAFLGV

                                                       67  70          84
Bla_ger_ace1      PYAQKPLGPLRFRHPRPVDRWDKHGEIYNATKMPNSCVQIIDTVFGDFPGALIWNPNTPL
Nep_cin_ace1      PYAQKPLGPLRFKHPRPPDRWD---YVYNATKQPNSCVQIFDTVFGDFSGAMMWNPNTQI
Lip_ent_ace1      PYAQKPLGNLRFRHPRPVERWEG---VLNTTKLPNSCMQILDTVFGDFPGATMWNPNTPL
Cim_lec_ace1      PYAQKPIGKLRFRHPRPLDKWN---HILNATRLPNTCVQIIDTVFGDFPGAIIWNPNTQL
Bom_man_ace1      PYAQKPMGDLRFRHPRPVEDWG--DEILNTTTLPHSCVQIVDTVFGDFPGAMMWNPNTDM
Bom_Mor_ace1      PYAQKPMGDLRFRHPRPVEDWG--DEILNTTTLPHSCVQIVDTVFGDFPGAMMWNPNTDM
Chi_sup_ace1      PYAQKPIGDLRFRHPRPAESWG--EEILNTTTLPHPCVQIIDTVFGDFPGAMMWNPNTDM
Bem_tab_ace1      PYAQKPIGALRFRHPRPIDKWEG---ILNATKMPNSCTQIVDTVFGDFAGSAMWNPNTPM
Api_mel_ace1      PYAQKPLESLRFRHPRPAERWSG---ILNATTLPNSCVQILDTVFGEFAGATMWNPNTPL
Cul_pip           PYAQPPLGPLRFRHPRPAERWT---GVLNATKPPNSCVQIVDTVFGDFPGATMWNPNTPL
Cul_qui           PYAQPPLGPLRFRHPRPAERWT---DRG--------------HVFGDFPGATMWNRNTPL
Aed_alb           PYAQPPLGPLRFRHPRPVEKWT---GVLNATTPPNSCVQIVDTVFGDFPGATMWNPNTPL
Ano_gam           PYAQPPVGPLRFRHPRPAEKWT---GVLNTTTPPNSCVQIVDTVFGDFPGATMWNPNTPL
Cte_fel_ace1      PYGRNPSGALRFRHPRPADPWQ---GVLNATSPPNTCVQIVDTLFGDFPGATMWNPNTPI
LS_ace1           PYAQPPVGNLRFRHPRHIDAWEG---IKETTRPPNSCIQVVDTLFPGFEGAEMWNVNTEP
LS_ace2           PYAQPPVGNLRFRHPKDINAWDG---MKETTKHPNSCIQVVDTFFPGFEGSEMWNTNTEQ
Tet_urt_ace1      RYAKPPTGKFRFRRPKPIDSWQG---IFNATSFPGACYQVNDTFFGNFMGATEWNPNVPL
Rhi_dec           PYAKPPTGKRRFDRAESIEEPWT--DVFDATVKPNSCFQVLDTLYGNFSGSTMWNANTEM
Celeg_ace1        PFAEPPVGNLRFKKPKPKQPWRIP---LNATTPPNSCIQSEDTYFGDFYGSTMWNANTKL
Cae_bri_ace1      PFAEPPIGNLRFRKPKPKQPWRIP---FNATTPPNSCIQSEDTYFGDFYGSTMWNPNTKL
Homosap           PFAEPPMGPRRFLPPEPKQPWSG---VVDATTFQSVCYQYVDTLYPGFEGTEMWNPNREL
Tor_cal           PFAEPPVGNMRFRRPEPKKPWSG---VWNASTYPNNCQQYVDEQFPGFSGSEMWNPNREM
Bom_man_ace2      PFAKPPLGPLRFRKPVPIEPWHG---VLEANLMPNSCYQERYEYFPGFEGEEMWNPNTNI
Bom_Mor_ace2      PFAKPPLGPLRFRKPVPIEPWHG---VLEANLMPNSCYQERYEYFPGFEGEEMWNPNTNI
Chi_sup_ace2      PFAKPPLGPLRFRKPVPIDPWHG---VLEATAMPNSCYQERYEYFPGFEGEEMWNPNTNI
Lep_dec           PFAKPPIEQLRFKKPVPIDPWHG---ILDATKQPNSCFQERYEYFPGFEGEEMWNPNTNI
Nep_cin_ace2      PFAKPPVGPLRFRRPVPVDPWHG---VYDATTLSNSCYQERYEYFPGFEGEEMWNPNTNI
Cim_lec_ace2      PFAKPPVGPLRFRKPVPVENWHG---ILDATTLPNSCHQQRYEYFPGFEGEEMWNPNTNM
Lip_ent_ace2      PFAKPPAGPLRFRRPVPIDPWHG---VFDATTLPNSCYQERYEYFPGFEGEEMWNPNTNI
Bla_ger_ace2      PFAKPPVGPLRFRRPVPVEPWHG---VLDATALPNSCYQERYEYFKGFEGEEMWNPNTNV
Api_mel_ace2      PFAKPPIGPLRFRKPLPIEPWHG---VLNATVLPNSCYQERYEYFPGFPGEEMWNPNTNI
Bem_tab_ace2      PFAKPPVGMLRFRKPVEIDPWRG---VLNATSLPNSCYQERLEYFPGFQGEEMWNPNTNI
Dros              PYAKPPVEDLRFRKPVPAEPWHG---VLDATGLSATCVQERYEYFPGFSGEEIWNPNTNV
Mus_dom           PYAKPPVDDLRFRKPVPAEPWHG---VLDATRLPATCVQERYEYFPGFSGEEIWNPNTNV
Cte_fel_ace2      PYAKPPVGPLRFRKPVPAEPWHG---VLDATHPPNCCVQERYEYFQGFQGEELWNPNTNI
Celeg_ace2        PYAKPPIGSRRFKMAEMIDRWSG---ELEARTLAKTCYLTIDSAFPQFPGAEMWNPPGAI
Cae_bri_ace2      PYAKPPVGSRRFKMAEMIDRWSG---ELEARTLAKTCYLTIDSAFPQFPGAEMWNPPGAI

                     94                                                       114
Bla_ger_ace1      SEDCLYINVVVPKPRPKNA---------------------------------AVMVWIFG
Nep_cin_ace1      SEDCLYLNVVAPKPRPSNA---------------------------------AVMVWVFG
Lip_ent_ace1      SEDCLYINVVAPKPRPKKA---------------------------------AVMVWIFG
Cim_lec_ace1      SEDCLYLNVVVPKPRPTNA---------------------------------AVMVWIFG
Bom_man_ace1      QEDCLYINIVTPRPRPKNA---------------------------------AVMLWVFG
Bom_Mor_ace1      QEDCLYINIVTPRPRPKNA---------------------------------AVMLWVFG
Chi_sup_ace1      QEDCLYINIVSPRPRPKNA---------------------------------AVMLWVFG
Bem_tab_ace1      SEDCLYINVITPKPRPRNA---------------------------------AVMVWIFG
Api_mel_ace1      SEDCLYVNVVVPRPRPTNA---------------------------------AVMVWIFG
Cul_pip           SEDCLYINVVVPRPRPKNA---------------------------------AVMLWIFG
Cul_qui           SEDCLYINVFVPRPRPKNA---------------------------------AVMLWIFG
Aed_alb           SEDCLYINVVVPHPRPKNS---------------------------------AVMLWIFG
Ano_gam           SEDCLYINVVAPRPRPKNA---------------------------------AVMLWIFG
Cte_fel_ace1      SEDCLYVNVVAPKPRPTNG---------------------------------PVMLWIFG
LS_ace1           SEDCLYLSVHVPKPRPTGS---------------------------------AVLVWIYG
LS_ace2           SEDCLYLSVHAPKPRPTKS---------------------------------AVLVWIYG
Tet_urt_ace1      DEDCLSVNIWVPRPRPKSA---------------------------------AVLLWIYG
Rhi_dec           SEDCLKLNVWTPGPSASSGGRP----------------------------LAVLVWIYG
Celeg_ace1        SEDCLYLNVYVPGKVDPNKK------------------------------LAVMVWVYG
Cae_bri_ace1      SEDCLYLNVYVPGKVDPNKK------------------------------LAVMIWVYG
Homosap           SEDCLYLNVWTPYPRPTSP-------------------------------TPVLVWIYG
Tor_cal           SEDCLYLNIWVPSPRPKS--------------------------------TTVMVWIYG
Bom_man_ace2      SEDCLYLNIWVPQHLRVRHHQ-------------------DKPLAERPKVPILVWIYG
Bom_Mor_ace2      SEDCLYLNIWVPQHLRVRHHQ-------------------DKPLAERPKVPILVWIYG
Chi_sup_ace2      SEDCLYLNIWVPQHLRVRHHQ-------------------KKPLTEKPKVPILVWIYG
Lep_dec           SEDCLYLNIWVPQRLRIRHHA-------------------DKPTIDRPKVPVLIWIYG
Nep_cin_ace2      SEDCLYLNIWVPQRLRIRHKSS------------------SEENTYRQKVPVLIWIYG
Cim_lec_ace2      SEDCLYLNIWVPQKIRLRHHN-------------------AQDRYNKPKVPVLIWIYG
Lip_ent_ace2      SEDCLYLNIWVPQRVRLRHHGGS-----------------QQDERHPHKVPMLIWIYG
Bla_ger_ace2      SEDCLYLNIWVPQRVRLRHSG-------------------------ERPKVPILVWIYG
Api_mel_ace2      SEDCLYLNIWVPQKYRLRHKGDGSPG----------------GNGGPRNGLLPLLVWIYG
Bem_tab_ace2      SEDCLYLNLWVPQKMRLRHRR------------------------HIHQKAPVLIWIYG
```

```
Dros             SEDCLYINVWAPAKARLRHGRGANGGEHPNGKQADTDHLIHNGNPQNTTNGLPILIWIYG
Mus_dom          SEDCLFMNIWAPAKARLRHGRGTNGGEHSS--KTDQDHLIHSATPQNTTNGLPILIWIYG
Cte_fel_ace2     SEDCLYLNVFAPARLKGGGQEGQ---------------------------GLPMLIWIYG
Celeg_ace2       SEDCLNMNIWVPEDHDGS-----------------------------------VMVWIYG
Cae_bri_ace2     SEDCLNMNIWVPEDHDGS-----------------------------------VMVWIYG

                    121      130
Bla_ger_ace1     GGFYSGSATLDVYDHKTLVSEENVIIVSMQYRVASLGFLFFD--------TIDVPGNAGL
Nep_cin_ace1     GGFYSGSATLDVYDPRILVSEENVIYVSMQYRVASLGFLFFD--------TPEVPGNAGL
Lip_ent_ace1     GGFYSGTATLDVYDPKTLVSEEKVIVVSMQYRIASLGFLFFD--------TPDVPGNAGL
Cim_lec_ace1     GGFYSGSATLDVYDHKTLVSEENVILVSMQYRVASLGFLYLD--------TADVPGNAGL
Bom_man_ace1     GGFYSGTATLDVYDPKILVSEEKVVYVSMQYRVASLGFLFFD--------TADVPGNAGL
Bom_Mor_ace1     GGFYSGTATLDVYDPKILVSEEKVVYVSMQYRVASLGFLFFD--------TADVPGNAGL
Chi_sup_ace1     GGFYSGTATLDVYDPKIMVSEEKIVYVSMQYRVASLGFLFFD--------TPDVPGNAGM
Bem_tab_ace1     GGFYTGTATLDIYDYKILASEENVILVSMQYRITCLGFLYFD--------TQDVPGNAGL
Api_mel_ace1     GGFYSGSATLDVYDHKTLVSEEKVILVSMQYRVASLGFLYFG--------TPDVPGNAGL
Cul_pip          GGFYSGTATLDVYDHRTLASEENVIVVSLQYRVASLGFLFLG--------TPEAPGNAGL
Cul_qui          GGFYSGTATLDVYDHRTLASEENVIVVSLQYRVASLGFLFLG--------TPEAPGNAGL
Aed_alb          GGFYSGTATLDVYDHRTLASEENVIVVSLQYRVASLGFLFLG--------TPEAPGNAGL
Ano_gam          GGFYSGTATLDVYDHRALASEENVIVVSLQYRVASLGFLFLG--------TPEAPGNAGL
Cte_fel_ace1     GGFYSGSSTLDVYDPKTLAAEEGVLVVSMQYRVASLGFLFLG--------TPDAPGNAGL
LS_ace1          GGFYSGTSTLEVYDPRVLVSEENIIFVAMQYRVASLGFLFFD--------TEDVPGNAGL
LS_ace2          GGFYSGTSTLELYDPRVLVSEENIIFVGIQYRVASLGFLFFD--------TEDVPGNAGL
Tet_urt_ace1     GGFWSGSSSLDFYDGSVLAGEESIIFVSINYRVASLGFIFFD--------TSDAPGNAGL
Rhi_dec          GGFYSGTSTLDVYDARTLVSEENVVVVAMNYRVASLGFLSFG--------NETLPGNAGL
Celeg_ace1       GGFWSGTATLDVYDGRILTVEENVILVAMNYRVSIFGFLYMN--------RPEAPGNMGM
Cae_bri_ace1     GGFWSGTSTLDVYDGRILTVEENVILVAMNYRVSIFGFLYMN--------RSEAPGNMGM
Homosap          GGFYSGASSLDVYDGRFLVQAERTVLVSMNYRVGAFGFLALP-------GSREAPGNVGL
Tor_cal          GGFYSGSSTLDVYNGKYLAYTEEVVLVSLSYRVGAFGFLALH-------GSQEAPGNVGL
Bom_man_ace2     GGYMSGTATLDLYKADIMASTSDVIVASMQYRVGAFGFLYLNKYFS-PG-SEEAPGNMGL
Bom_Mor_ace2     GGYMSGTATLDLYKADIMASTSDVIVASMQYRVGAFGFLYLNKYFS-PG-SEEAPGNMGL
Chi_sup_ace2     GGYMSGTATLDLYKADIMASSSDVIVASMQYRVGAFGFLYLNKYFS-AG-SEEAPGNMGL
Lep_dec          GGYMSGTATLDVYDADIIAATSDVIVASMQYRLGSFGFLYLNRYFP-RG-SDETPGNMGL
Nep_cin_ace2     GGYMSGTATLDIYDADMVAATSDVIVASMQYRVGAFGFLYLSPELP-PG-SEEAPGNLGL
Cim_lec_ace2     GGYMSGTATLDVYDGLMVAATSDVIVASMQYRIGAFGFLYLEPLVK-TG-SNDAPGNMGL
Lip_ent_ace2     GGFMSGTSTLDVYDADIVAATSDVIVASMQYRIGAFGFLYLAPYSKNKD-NDEAAGNMGL
Bla_ger_ace2     GGYMSGTSTLDVYDADIVVATSDIIVASMQYRVGSFGFLYLKPFFG-PD-SEEAPGNMGM
Api_mel_ace2     GGFMSGTATLDVYNADIMAATSNVIIASMQYRVGAFGFLYLNKHFT--N-SEEAPGNMGL
Bem_tab_ace2     GGYMTGTSTLELYDADIVAGVCNVIVASLQYRVGSFGFLYLKPLLP-EG-IEEAPGNMGL
Dros             GGFMTGSATLDIYNADIMAAVGNVIVASFQYRVGAFGFLHLAPEMPSEF-AEEAPGNVGL
Mus_dom          GGFMTGSATLDIYNAEIMSAVGNVIVASFQYRVGAFGFLHLSPVMP-GF-EEEAPGNVGL
Cte_fel_ace2     GGYMSGSAALDIYNAEILSSTGNVIVAAMQYRVGAFGFLYLAPHFR-NG-ASEAPGNMGL
Celeg_ace2       GGFFSGTPSLDLYSGSVFAAKEHTIVVNVNYRLGPFGFLYFGD-------DSPIQGNMGL
Cae_bri_ace2     GGFFSGTPSLDLYSGSVFAAKEHTIVVNVNYRLGPFGFLYFGD-------DSPIQGNMGL

                                              200
                                               ↓
Bla_ger_ace1     FDQLMALQWVHDNIQAFGGNPNNVTLFGESAGAVSVSLHLLSPLSRNLFSQAIMESGSPT
Nep_cin_ace1     FDQLMALQWVHDNIHFFGGNPHNVTLFGESAGAVSVSLHLLSPLSRNLFSQAIMESGSAT
Lip_ent_ace1     FDQLMALQWVHDNIHAFGGNPHNVTLFGESAGAVSVSTHLLSPLSRNLFSQAIMESGSPT
Cim_lec_ace1     YDQRMALQWVHDNIHLFGGDPQKVTLFGESAGAVSVSLHLLSPLSHKLFNQAIMESGSAV
Bom_man_ace1     FDQLMALQWVKDNIGYFGGNPHNITLFGESAGAVSVSLHLLSPLSRNLFSQAIMQSGAAT
Bom_Mor_ace1     FDQLMALQWVKDNIGYFGGNPHNITLFGESAGAVSVSLHLLSPLSRNLFSQAIMQSGAAT
Chi_sup_ace1     FDQLMALQWVKDNIAYFGGNPHNVTLFGESAGAVSVSLHLLSPLSRNLFSQAIMQSGAAT
Bem_tab_ace1     FDQLMALQWIRNNIHAFGGNPHNITLFGESAGAVSVSMHLLSPLSRNLFSQAIMESGSAT
Api_mel_ace1     FDQVMALEWVRDNIAAFGGNPDNVTLFGESAGAVSVSMHLLSPLSRHLFNQAIMQSGSPT
Cul_pip          FDQNLALRWVRDNIHRFGGDPSRVTLFGESAGAVSVSLHLLSALSRDLFQRAILQSGSPT
Cul_qui          FDQNLALRWVRDNIHRFGGDPSRVTLFGESAGAVSVSLHLLSALSRDLFQRAILQSGSPT
Aed_alb          FDQNLALRWVRDNIHKFGGDPSRVTLFGESAGAVSVSLHLLSALSRDLFQRAILQSGSPT
Ano_gam          FDQNLALRWVRDNIHRFGGDPSRVTLFGESAGAVSVSLHLLSALSRDLFQRAILQSGSPT
Cte_fel_ace1     FDQLLALRWVRDNVRAFGGDPDRVTLFGESAGAVSVSMHLLSPLSKDLFARAILESGSPT
LS_ace1          YDQMMALQWVKNNIEEFGGDPDKITIFGESAGGCSVALHLLSPLSRNLFSQAIMQSASAL
LS_ace2          YDQMMALQWVKNNIEAFGGDPDKITIFGESAGGCSVALHLLSPLSRNLFSQAIMQSSSAL
Tet_urt_ace1     FDQLMAMEWIRENIAAFGGNPANITIFGESAGAVSAALHLLSPLSRNVFSQAILQSGSAT
Rhi_dec          YDQYMALKWVQENVAAFGGDPDRVTLFGESAGAVSVGLHVLSPLSESLFHRVILQSGSPG
Celeg_ace1       WDQLLAMKWVHKNIDLFGGDLSRITLFGESAGAASVSIHMLSPKSAPYFHRAIIQSGSAT
Cae_bri_ace1     WDQLLAMKWVHKNIDLFGGDTSRITLFGESAGAASVSIHMLSQKSAPYFHRAIIQSGSAT
Homosap          LDQRLALQWVQENVAAFGGDPTSVTLFGESAGAASVGMHLLSPPSRGLFHRAVLQSGAPN
Tor_cal          LDQRMALQWVHDNIQFFGGDPKTVTIFGESAGGASVGMHILSPGSRDLFRRAILQSGSPN
Bom_man_ace2     WDQQLAIRWIKENARAFGGDPELITLFGESAGGGSVSLHMLSPEMKGLFKRGILQSGTLN
Bom_Mor_ace2     WDQQLAIRWIKENARAFGGDPELITLFGESAGGGSVSLHMLSPEMKGLFKRGILQSGTLN
Chi_sup_ace2     WDQQLAIRWIKENARAFGGDPELITLFGESAGGGSVSLHMLSPEMKGLFKRGILQSGTLN
Lep_dec          WDQILAIRWIKDNAAAFGGDPDLITLFGESAGGGSISIHLISPVTKGLVRRGIMQSGTMN
Nep_cin_ace2     WDQALAIQWIKANIANFGGDPELCTLFGESAGGGSVSLHLVSPVTRGLVRRGIMQSGTLN
Cim_lec_ace2     WDQAMAIRWIKDNIENFGGDPELITLFGESAGGGSVSIHLISPVTRGLARRGIMQSGTIN
Lip_ent_ace2     WDQAMAIRWLKDNAEAFGGDPELLTLFGESAGGGSVSLHLMSPVTKGLVRRGILQSGTLN
Bla_ger_ace2     WDQALAIRWLKDNAAAFGGDPDLITLFGESAGGGSVSLHLMSPITKGLVRRGIMQSGTLN
Api_mel_ace2     WDQALALRWLRDNAEAFGGDPELITIFGESAGGSSVSLHLISPVTRGLVRRGILQSGTLN
```

```
Bem_tab_ace2     WDQAMAIKWIKDNIAAFGGDPDMLTLFGESAGGSSVNIHLISPVTKGLARRGILQSGTLN
Dros             WDQALAIRWLKDNAHAFGGNPEWMTLFGESAGSSSVNAQLMSPVTRGLVKRGMMQSGTMN
Mus_dom          WDQALALRWLKENARAFGGNPEWMTLFGESAGSSSVNAQLMSPVTRGLVKRGMMQSGTMN
Cte_fel_ace2     WDQALAIRWLKDNAAAFGGDPERLTLFGESAGAGSVSLHLLSPATRGLFARALLQSGTIN
Celeg_ace2       MDQQLALRWVHENIGAFGGDRSRVTLFGESAGSASTTAHLFAPNSHKYFRNIIAKSGSII
Cae_bri_ace2     MDQQLALKWVHENIGAFGGDRSRVTLFGESAGSTSATAHLFAPNSHKYFRNIIAKSGSII

                  233                 254          265            279
Bla_ger_ace1     APWAIISREESILRGLRLAEAVGCPRSRS---DIRAVIDCLRKKNATDLVNNEWG----T
Nep_cin_ace1     APWAIISRDESFVRGLRLAEAVGCPHTRA---EIHEAIDCLRKKNASELVENEWG----T
Lip_ent_ace1     APWAIISREESILRGLRLAEAVNCPHDKN---QIKSVIECLRNTNASVLVDNEWG----T
Cim_lec_ace1     APWAIISREESMLRGLRLAEAVGCPHSKH---ELRAVIDCLRNTNATDLVSNEWG----T
Bom_man_ace1     APWAIISREESILRGIRLAEAVHCPHSRS---DLAPMIECLRKKNADELVNNEWG----T
Bom_Mor_ace1     APWAIISREESILRGIRLAEAVHCPHSRS---DLAPMIECLRKKNADELVNNEWG----T
Chi_sup_ace1     APWAIISREESILRGTRLAEAVHCPHSLK---DMGPMIECLRKKSADELVNNEWG----T
Bem_tab_ace1     APWAIISRQESIIRGLRLAEAVGCPHTRA---QIPEAIECLRKVNASVLVENESG----T
Api_mel_ace1     APWAIISREESIVRGIRLAEAVGCPHDRD---NLQEVIDCLRVKDPVELVKNEWG----T
Cul_pip          APWALVSREEATLRALRLAEAVNCPHDAT---KLSDAVECLRTKDPNELVDNEWG----T
Cul_qui          APWALVSREEATLRALRLAEAVNCPHDAT---KLSDAVECLRTKDPNELVDNEWG----T
Aed_alb          APWALVSREEATLRALRLAEAVNCPHDAS---KLTDTVECLRTKDPNVLVDNEWG----T
Ano_gam          APWALVSREEATLRALRLAEAVGCPHEPS---KLSDAVECLRGKDPHVLVNNEWG----T
Cte_fel_ace1     APWALRSRQEALNRSLLLAKTVGCPHSPD---DLAATAECLRQKDSRDLVNNEWN----D
LS_ace1          VPWGVITKKESIIRGRRLAEMMSCPYDEK---NTKAMIECLRQKDATEMVNQEWIGI--I
LS_ace2          VPWGVISKKESIRRGRRLAEEMRCPYGEN---NTNAMIECLLQKDATELVNQEWSGT--V
Tet_urt_ace1     CPWAISDRKKAYQRSLALAQAVGCGSTSTR--SVHAIIECMQSIPASELVAQEET----T
Rhi_dec          VPWGFQDRDKARQSAKRLATALRAPDSLD-----QETLDSLRCERPEDIVNNETN----S
Celeg_ace1       SPWAIEPRDVALARAVILYNAMKCGNMSLINPDYDRILDCFQRADADALRENEWAP---V
Cae_bri_ace1     SPWAIEPRDVALARAVILYNAMKCGNMSLISPDYDRILDCFQRADADALRENEWAP---V
Homosap          GPWATVGMGEARRRATQLAHLVGCPPGGT-GGNDTELVACLRTRPAQVLVNHEWHVL-PQ
Tor_cal          CPWASVSVAEGRRRAVELGRNLNCN-----LNSDEELIHCLREKKPQELIDVEWNVL-PF
Bom_man_ace2     APWSWMTGERAQDIGKVLIDDCNCNSSLLA-KDPSLVMDCMRGVDAKTISVQQWNS---Y
Bom_Mor_ace2     APWSWMTGERAQDIGKVLIDDCNCNSSLLA-KDPSLVMDCMRGVDAKTISVQQWNS---Y
Chi_sup_ace2     APWSWMTGERAQDIGKVLVDDCNCNSSLLT-ADPSLVMDCMRGVDAKTISVQQWNS---Y
Lep_dec          APWSYMSGERAEQIGKILIQDCGCNVSLLE-NSPRKVMDCMRAVDAKTISLQQWNS---Y
Nep_cin_ace2     APWSYMTGERAVEIAKTLIDDCGCNASMLI-ESPSRVMSCMRAVDAKTISVQQWNS---Y
Cim_lec_ace2     APWSFMTGERALEIGKILVEDCGCNVSQLA-ESPSRVMACLRAVDAKSISVHQWDS---Y
Lip_ent_ace2     APWSYMEAPKAVDIAKQLIDDCGCNSSILA-DFPHEVMTCMRNVEPKLISVQQWNS---Y
Bla_ger_ace2     APWSYMTGEKAADIGRVLVEDVGCNSTQLS-EAPSKVMACLRSVDSKAISVKQWNS---Y
Api_mel_ace2     APWSYMSGEKANEVATILVDDCGCNSTMLN-ENPARVMACMRSVDAKTISVQQWNS---Y
Bem_tab_ace2     MPWSYMEAEKAMQIGKILVDDCNCNSSQLE-ENPTKVFACMRAVDAKIVSSQQWSS---Y
Dros             APWSHMTSEKAVEIGKALINDCNCNASMLK-TNPAHVMSCMRSVDAKTISVQQWNS---Y
Mus_dom          APWSHMTSEKAVEIGKALVNDCNCNASLLP-ENPQAVMACMRQVDAKTISVQQWNS---Y
Cte_fel_ace2     APWSHMTAQDAVRVAEALVEDCGCNATLLR-DSPSMVLACMRSVDAKTISVQQWNS---Y
Celeg_ace2       NSWASATPPTMLDLSFRLAKKVNCSSPDMN-----AIVKCLRSVPAHLVQAEADNISGDI
Cae_bri_ace2     NSWASAPPPTMLDLSFRLAKKVNCSSTDMN-----VVAKCLRSVPAHLVQAEADNISGDI

                                                        327
                    288  290                                330 331  334
Bla_ger_ace1     LGICEFPFVPI-IDGTILDGPPQRSLAEKNFKK-TNILMGSNTEEGYYFIIYYLTELFRK
Nep_cin_ace1     LGICEFPFVPI-VDGAFLDDLPVRSLATKNFKK-TNILMGSNTEEGYYFIIYYLTELFRK
Lip_ent_ace1     LGICEFPFVPV-IDGSFLDETPQKSLANKNFKK-TNILMGSNTEEGYYFIIYYLTELLRK
Cim_lec_ace1     LGICEFPFVPI-VDGTFVDDHPKRNLAARNFKK-TNILMGSNTEEGYYFIIYYLTELFRK
Bom_man_ace1     LGICEFPFVPI-IDGSFLDEMPVRSLAHQNFKK-TNILMGSNTEEGYYFILYYLTELFPK
Bom_Mor_ace1     LGICEFPFVPI-IDGSFLDEMPVRSLAHQNFKK-TNILMGSNTEEGYYFILYYLTELFPK
Chi_sup_ace1     LGICEFPFVPI-IDGSFLDEMPIRSLAHQNFKK-TNILMGSNTEEGYYFILYYLTELFPK
Bem_tab_ace1     LGICDFPFVPV-VDGSFLDEMPSKSLATKNFKK-TNILMGSNTEEGNYFIMYYLTDLFRK
Api_mel_ace1     LGICEFPFVPV-IDGAFLDETPQRSLATSSFKK-ANIMMGSNTEEGFYFIIYYLTELFHI
Cul_pip          LGICEFPFVPV-VDGAFLDETPQRSLASGRFKK-TDILTGSNTEEGYYFIIYYLTELLRK
Cul_qui          LGICEFPFVPV-VDGAFLDETPQRSLASGRFKK-TDILTGSNTEEGYYFIIYYLTELLRK
Aed_alb          LGICEFPFVPV-VDGAFLDETPQRSLASGRFKK-TDILTGSNTEEGYYFIIYYLTELLRK
Ano_gam          LGICEFPFVPV-VDGAFLDETPQRSLASGRFKK-TEILTGSNTEEGYYFIIYYLTELLRK
Cte_fel_ace1     LGICEFPFVPV-VDGAFLDESPQRALKRGNFKK-TDILTGSNTEEGYYFIIYYLTELFKK
LS_ace1          SGIAEFPFVPI-VDGSFLDESPGKSLTTKNYKK-TNILIGANKEEGNYFIMYYLTDLFKN
LS_ace2          FGISEFPFVPI-VDGKFMDKTPEKSLKEKDYKK-TNILMGVNKDEGNFFIMYYLPELFKK
Tet_urt_ace1     TGVVEFAFIPI-VDGSFLDEDPEVSLRTKNFKH-TPILTGSNRDEGTYFLVYHSPHIFNL
Rhi_dec          GGVVDFPFVPV-VDGVFLPDTPQTLMDKGSFARNISVMLGSNANEGSWFLQYFFG--FPV
Celeg_ace1       REFGDFPWVPV-VDGDFLLENAQTSLKQGNFKK-TQLLAGSNRDESIYFLTYQLPDIFPV
Cae_bri_ace1     REFGDFPWVPV-VDGDFLLENAQTSLKQGNFKK-TQLLAGSNRDESIYFLTYQLPDIFPV
Homosap          ESVFRFSFVPV-VDGDFLSDTPEALINAGDFHG-LQVLVGVVKDEGSYFLVYGAPGFSKD
Tor_cal          DSIFRFSFVPV-IDGEFFPTSLESMLNSGNFKK-TQILLGVNKDEGSFFLLYGAPGFSKD
Bom_man_ace2     TGILGFPSAPT-VDGIFLPKDPDTMMKEGNFHN-SEVLLGSNQDEGTYFLLYDFLDYFEK
Bom_Mor_ace2     TGILGFPSAPT-VDGIFLPKDPDTMMKEGNFHN-SEVLLGSNQDEGTYFLLYDFLDYFEK
Chi_sup_ace2     TGILGFPSAPT-VDGVFLPKDPDTMMKEGNFHN-TEVLLGSNQDEGTYFLLYDFLDYFEK
Lep_dec          SGILGFPSTPT-IEGVLLPKHPMDMLAEGDYED-MEILLGSNHDEGTYFLLYDFIDFFEK
Nep_cin_ace2     FGILGFPSAPT-IDGVFLPKHPLDLLKEGDFQD-TEILIGSNQDEGTYFILYDFIDYFEK
Cim_lec_ace2     FSILNFPSAPT-IDGNFLPKHPLELLAEGDFPE-TEIIIGSNLDEGTYFMLYDFIDYFEK
Lip_ent_ace2     WGILGFPSAPT-IDGVFLPEHRLALLKKGDFPE-TEIMIGSNLDEGTYFILYDFIDYFEK
Bla_ger_ace2     WGILGFPSAPT-IDGVFLPKHPMDLIKELDWED-TEILIGSNQDEGTYFILYDFMDYFEK
```

```
Api_mel_ace2     WGILGFPSAPT-IDGIFLPKHPLDLLREADFKD-TEILIGNNENEGTYFILYDFNDIFEK
Bem_tab_ace2     FGILGYPSAPT-IDGEFLPKHPLELMKDQNFED-IELLIGSNRDEGTYFLLYDFLEFFEK
Dros             SGILSFPSAPT-IDGAFLPADPMTLMKTADLKD-YDILMGNVRDEGTYFLLYDFIDYFDK
Mus_dom          SGILSFPSAPT-IDGAFLPADPMTLLKTADLSG-YDILIGNVKDEGTYFLLYDFIDYFDK
Cte_fel_ace2     SGILGFPSAPT-IDGVFMTGDPMQMLRNADLQG-VDVMIGSNKDEGTYFILYDFIDYFEK
Celeg_ace2       GPPMTFAYVPVSSDANFFQGDVFQKLANKQFKKDVNIIFGSVKDEGTYWLPYYMSLPKYG
Cae_bri_ace2     GPPMTFAYVPVSSDANFFQGDVIQKLNNKQFKKDVNIIFGSVKDEGTYWLPYYMSLPKYG

Bla_ger_ace1     E------------ENVYVNREEFLRSVQELN---PYVNNVARQAIVFEYTDWLNPDDPIR
Nep_cin_ace1     E------------ENVYVBRDEFLHAVHELN---PYVNNVARQAIVFEYTDWLNPDDPIR
Lip_ent_ace1     E------------ENVYVNRDEFLQAVRELN---PYINKVARQAIIFEYTDWLNPDDPVR
Cim_lec_ace1     E------------ENVYINREEFLRAVVELN---PYVNNIARQAIIFEYTDWQNPEDPIK
Bom_man_ace1     E------------ENVGISREQFLQAVRELN---PYVNDVARQAIIYEYTDWLNPEDPVK
Bom_Mor_ace1     E------------ENVGISREQFLQAVRELN---PYVNDVARQAIIYEYTDWLNPEDPVK
Chi_sup_ace1     E------------ENVGITREQYLQAVRELN---PYVSDVGRQAIVFEYTDWLNPEDPVR
Bem_tab_ace1     E------------ENIHVSRDQFIQAVSELN---PYN-FIVRRAIIFEYTDWLNPDDPVK
Api_mel_ace1     DG-----------SEVKVSREQFISAVSELN---PYVNQFGRRAIIYEYTDWLRPDDPHA
Cul_pip          E------------EGVTVTREEFLQAVRELN---PYVNGAARQAIVFEYTDWIEPDNPNS
Cul_qui          E------------EGVTVTREEFLQAVRELN---PYVNGAARQAIVFEYTDWIEPDNPNS
Aed_alb          E------------EGVTVSREEFLQAVRELN---PYVNGAARQAIVFEYTDWTEPENPNS
Ano_gam          E------------EGVTVTREEFLQAVRELN---PYVNGAARQAIVFEYTDWTEPDNPNS
Cte_fel_ace1     E------------EGINVTREQFLQAVKDLN---PRVGPIGTQAIVFEYTDWLDPEDPLG
LS_ace1          T------------ESVYVDRTDFIRSVSELN---HYVKKMGREAITFEYTDWLNPNDPIK
LS_ace2          N------------ENVYINRTDFIRSVSDLN---IYVNNAGREAITFEYTDWLNPNDPIK
Tet_urt_ace1     S------------EGIYISRSEFQSLIRIIY---PHLSPLAQEAVIQEYTHWINPDDQIE
Rhi_dec          SD-----------ETPEVTKENFTAVLEALD---PSLEHTPIAEIMKTYTAGEIPSTAAD
Celeg_ace1       ADF-------FTKTDFIKDRQLWIKGVKDLLPRQILKCQLTLAAVLHEYEPQDLPVTPRD
Cae_bri_ace1     ADF-------FSKSEFIKDRQTWIKGVKDLLPRQILKCQLTLAAVLHEYEPQDLPISAQN
Homosap          N-------------ESLISRAEFLAGVRVGV---PQVSDLAAEAVVLHYTDWLHPEDPAR
Tor_cal          S-------------ESKISREDFMSGVKLSV---PHANDLGLDAVTLQYTDWMDDNNGIK
Bom_man_ace2     D------------GPSFLQREKFLEIVDTIFK---DFSKIKREAIVFQYTDWEEITDGYL
Bom_Mor_ace2     D------------GPSFLQREKFLEIVDTIFK---DFSKIKREAIVFQYTDWEEITDGYL
Chi_sup_ace2     D------------GPSFLQREKFLEIVDTIFK---EFSKIKREAIVFQYTDWEEITDGYL
Lep_dec          D------------GPSFLQREKYHDIIDTIFK---NMSRLERDAIVFQYTNWEHVHDGYL
Nep_cin_ace2     D------------GPSFLQRDKFLDIINTIFK---NFTRLERDAIIFQYTDWEHANDGYL
Cim_lec_ace2     D------------GPIFLQRDKYLDIVNTIFK---NMITLERDAIIFQYTDWEKVNDEHL
Lip_ent_ace2     D------------GPSFLQRDKFLEIINTIFK---NFSRIEREAIVFQYTDWDQSNDGFL
Bla_ger_ace2     D------------SPTFLQRDKYLDIVNLIFK---NMTRLERDAIIFQYTDWEHLADGYK
Api_mel_ace2     D------------QASFLERERFLGIINNIFK---NMSQIEREAITFQYTDWEEVYNGYI
Bem_tab_ace2     D------------GPSLLQRDKFLDIIHTIFK---NFSPLEKEAIIFQYTDWENLGDGYT
Dros             D------------DATALPRDKYLEIMNNIFG---KATQAEREAIIFQYTSWE-GNPGYQ
Mus_dom          D------------DATSLPRDKYLEIMNNIFQ---KASQAEREAIIFQYTSWE-GNPGYQ
Cte_fel_ace2     D------------GPSILQRDKFLEIMSTIFV---KASPAERQAIDFQYTDWENPTDGSL
Celeg_ace2       FAFNHTISAEDPHNRALITRDHYEESMRAFMPYFAGSKLVLNAFMNSYEHVSTSNVPEER
Cae_bri_ace2     FAFNHTISAEDPHNRALITREHYEESMKAFMPYFAGSKLVLNAFMNSYEHVSTSNVPEER

                                                                    440
                              402                            432        442
Bla_ger_ace1     NRDALDKMVGDYHFTCNVNEFAHRYAETGN-NVYMYYFKHRSVGNPWPSWTGVMHGDEIN
Nep_cin_ace1     NRDALDKMVGDYHFTCNVNEFAHRYAETGN-NVYMYYFKYRSIGNPWPSWTGVMHADEIN
Lip_ent_ace1     SRDALDKMVGDYHFTCNVNEFAHRYAETGN-NVYMYYFKHRSAANPWPSWTGVMHGDEIN
Cim_lec_ace1     NRDALDKMVGDYQFTCTVNEFAHTYAESGN-VVYMYLFSHRSIGNPWPSWTGVMHGDEIN
Bom_man_ace1     NRNALDKMVGDYHFTCGVNEFAHRYAETGN-NVYTYYYKHRSKNNPWPSWTGVMHADEIN
Bom_Mor_ace1     NRNALDKMVGDYHFTCGVNEFAHRYAETGN-NVYTYYYKHRSKNNPWPSWTGVMHADEIN
Chi_sup_ace1     NRNALDKMVGDYHFTCGVNEFAHRYAETGN-NVYTYYYKHRSKNNPWPSWTGVMHADEIN
Bem_tab_ace1     NRDALDKIVGDFHFTCNVNEFAYRYAETGN-TVYMYYFKHRSTSSPWPTWSGALHGDEIN
Api_mel_ace1     NRDALDKIVGDYQFTCNVNEFAGRYTDTGN-TVYMYYYKHRSMNNPWPRWTGVMHADEIS
Cul_pip          NRDALDKMVGDYHFTCNVNEFAQRYAEEGN-NVFMYLYTHRSKGNPWPRWTGVMHGDEIN
Cul_qui          NRDALDKMVGDYHFTCNVNEFAQRYAEEGN-NVFMYLYTHRSKGNPWPRWTGVMHGDEIN
Aed_alb          NRDALDKMVGDYHFTCNVNEFAQRYAEEGN-NVYMYLYTHRSKGNPWPRWTGVMHGDEIN
Ano_gam          NRDALDKMVGDYHFTCNVNEFAQRYAEEGN-NVYMYLYTHRSKGNPWPRWTGVMHGDEIN
Cte_fel_ace1     NRDALDKIVGDYQFTCNVNEFAHRYASEGL-NVYMYLFSHRSRINPWPRWTGVMHGDEIS
LS_ace1          NREAIDRMVGDYQFICPTADFARIYASTGN-NVYMYYFTERSSTSPWPTWSGVLHGDEIA
LS_ace2          NREAIDRMVGDYQFTCPTADFARIYASTGN-NIYMYYFTERSSTSPWPTWSGVLHGDEIA
Tet_urt_ace1     NREATDKFVGDYHFTCPVNEMSYRYALYGN-DVWTYHFTHRSSKSFWPSWMGVIHGEEIK
Rhi_dec          ILKALDSIVGDYHFTCPAVRWADTFARAGI-PVYQYVFARRSSRNPWPQWTGVMHGEEVP
Celeg_ace1       WINAMDKMLGDYHFTCSVNEMALAHTKHGG-DTYYYYFTHRASQQTWPEWMGVLHGYEIN
Cae_bri_ace1     WLNAMDKMLGDYHFTCSVNEMALAHTKHGG-DTFYYYFTHRATQQTWPEWMGVLHGYEIN
Homosap          LREALSDVVGDHNVVCPVAQLAGRLAAQGA-RVYAYVFEHRASTLSWPLWMGVPHGYEIE
Tor_cal          NRDGLDDIVGDHNVICPLMHFVNKYTKFGN-GTYLYFFNHRASNLVWPEWMGVIHGYEIE
Bom_man_ace2     NQKMIADVVGDYFFVCPTNYFAEILADAGV-DVYYYYFTHRTSTSLWGEWMGVMHGDEME
Bom_Mor_ace2     NQKMIADVVGDYFFVCPTNYFAEILADAGV-GVYYYYFTHRTSTSLWGEWMGVMHGDEME
Chi_sup_ace2     NQKMIADVVGDYFFVCPTNYFAEILADAGV-DVYYYYFTHRTSTSLWGEWMGVMHGDEME
Lep_dec          NQKMIGDVVGDYFFVCPTNNFAEVAADRGM-KVFYYYFTHRTSTSLWGEWMGVIHGDEVE
Nep_cin_ace2     NQKMIGDVVGDYFFICPTNLFAQAFADHGL-KVFYYFFTQRTSTSLWGEWMGVMHGDEIE
Cim_lec_ace2     NQKMVGDIIGDYFFICPTNQFAQMFAEHGL-KVYYYFFTQRPSTSPWGEWMGVMHGDEVG
Lip_ent_ace2     NQKMIADVGGDYYFVCPSNLFAEMFADVGM-KVYYYYFTQRTSTDLWGEWMGVMHGDEIE
```

```
Bla_ger_ace2      NQRMIADVVGDYFFICPTNVFAEQFAEHGT-KVYYYYFTQRTSLNLWGQWMGVMHGDEIE
Api_mel_ace2      YQKMVADVVGDYFFICPSIHFAQLFADRGM-KVYYYFFTQRTSTNLWGEWMGVLHGDEVE
Bem_tab_ace2      NQKMIGEIVGDYFFICPSNYFAQVMSDNGA-KIYYYYFTQRTSTNLWGEWMGVMHGDEVE
Dros              NQQQIGRAVGDHFFTCPTNEYAQALAERGA-SVHYYYFTHRTSTSLWGEWMGVLHGDEIE
Mus_dom           NQQQIGRAVGDHFFTCPTNEYAQALAERGA-SVHYYYFTHRTSTSLWGEWMGVLHGDEIE
Cte_fel_ace2      NQDQVGRAVGDHFFVCPSNLFAEGLAERGA-NVRYYYFTHRTSTSVWGEWMGVMHGDEIE
Celeg_ace2        YRDGVARFLGDLFFTCSLIDFADLISDNIFGNVYMYYFTYRSSANPWPKWMGVMHGYEIE
Cae_bri_ace2      YRDGVARFLGDLFFTCSLIDFADLISDNIFGNVYMYYFTYRSSANPWPRWMGVMHGYEIE

Bla_ger_ace1      YVFGEPLNPAKN----YQPQEIELSRRMMRYWANFAKTGNP--SMSEDGTWTATYWPVHT
Nep_cin_ace1      YIFGEPLNPILN----YHPQEVELSRRMMRYWANFAKTGNP--SMSEDGTWTATYWPVHT
Lip_ent_ace1      YVFGEPLNPKKN----YQPQEKILSKRMMRYWANFAKTGNP--SMSEDGTWTDVYWPVHT
Cim_lec_ace1      YVFGEPLNPTKN----YLPSEAELSRRMMNYWANFAKTGNP--NLKGNNSWTSTYWPQHT
Bom_man_ace1      YVFGEPLNPGKN----YSPEEVEFSKRLMRYWANFARSGNP--SLNPNGEMTKIHWPVHT
Bom_Mor_ace1      YVFGEPLNPGKN----YSPEEVEFSKRLMRYWANFARSGNP--SLNPNGEMTKIHWPVHT
Chi_sup_ace1      YVFGEPLNPGKN----YSPEEVEFSKRIMRYWANFARSGNP--SLNPNGDMTKVHWPVHT
Bem_tab_ace1      YIFGEPLNPTKK----YQPAEVELAKRMMRYWANFAKTGNP--SLSSDGSWASVYWPQHT
Api_mel_ace1      YVFGEPLDPTKG----YTPEEVNLSKKMMRYWANFAKTGDP--NVGDVDVWTQAYWPPHT
Cul_pip           YVFGEPLNSALG----YQDDEKDFSRKIMRYWSNFAKTGNP--NPSTPSVDL-PEWPKHT
Cul_qui           YVFGEPLNSALG----YQDDEKDFSRKIMRYWSNFAKTGNP--NPSTPSVDL-PEWPKHT
Aed_alb           YVFGEPLNSDLG----YMEDEKDFSRKIMRYWSNFAKTGNP--NPSPPNSDF-TEWPKHT
Ano_gam           YVFGEPLNPTLG----YTEDEKDFSRKIMRYWSNFAKTGNP--NPNTASSEF-PEWPKHT
Cte_fel_ace1      YVFGEPQDSSRG----YTHAEAALSKRMMRYWANFAKTGDP--NPG-PGNEP-IYWPKHT
LS_ace1           FVFGEALNKSKN----YDKSEIALSKRMMGYWANFAKTGNP--SLSADGTWSTNYWPLHT
LS_ace2           FVFGEPLNTSKN----YDDSEIALSKRIMSYWANFAKTGNP--NVLANGNYSNKIWPLHT
Tet_urt_ace1      FVLGEPLDPVHG----YTPAEVQLSKRIMRYWANFARTGNPNKQFPDGDDTESIVWPEYT
Rhi_dec           FVFGEPLNDTHC----YSEEDKTLSRRIMRYWANFAKTGNPN-LTEDGSFGSTIHWPERT
Celeg_ace1        FIFGEPLNQKRFN---YTDEERELSNRFMRYWANFAKTGDP--NKNEDGSFTQDVWPKYN
Cae_bri_ace1      FIFGEPFNQKRFN---YTDEERELSNRFMRYWANFAKTGDP--NKNEDGSFTQDIWPKYN
Homosap           FIFGIPLDPSRN----YTAEEKIFAQRLMRYWANFARTGDP----NEPRDPKAPQWPPYT
Tor_cal           FVFGLPLVKELN----YTAEEEALSRRIMHYWATFAKTGNP----NEPHSQES-KWPLFT
Bom_man_ace2      YVFGHPLNMSLQ----YHSRERDLAAHIMQSFTQFALTGKP--------HKPDEKWPLYS
Bom_Mor_ace2      YVFGHPLNMSLQ----YHSRERDLAAHIMQSFTQFALTGKP--------HEPDEKWPLYS
Chi_sup_ace2      YVFGHPLNMSLQ----YHTRERDLAAHIMQSFTRFALTGKP--------HKPDEKWPLYS
Lep_dec           YVFGHPLNMSLQ----FNSRERELSLKIMQAFARFATTGKP--------VTDDVNWPLYT
Nep_cin_ace2      YVFGHPLNMSLQ----YNARERDLSLRIMQAYSRFALTGKP--------VSDDINWPIYS
Cim_lec_ace2      YVFGRPLNMSLS----YSARERDLSLRIIEAFSTFALTGKP--------VPEDVNWPPYT
Lip_ent_ace2      YVFGHPLNMSIQ----YNKKERALSKRIMDTFTRFALTGKP--------MPEEREWPPYT
Bla_ger_ace2      YVFGHPLNMSID----YNDNERDLSLRIMEIYSRFALTGKP--------IANEADWPTYT
Api_mel_ace2      YVFGHPLNKSLK----YSDKERDLSLRMILYFSEFAYLGKP--------TKEDSEWPSYS
Bem_tab_ace2      YVFGHPLNMSLQ----YNARERDLSNRIMEAFSKFAMTGKP--------TGEDVTWPQYT
Dros              YFFGQPLNNSLQ----YRPVERELGKRMLSAVIEFAKTGNP--------AQDGEEWPNFS
Mus_dom           YFFGQPLNNSLQ----YRPVERELGKRMLNSVIEFAKSGNP--------AVDGEEWPNFS
Cte_fel_ace2      YIFGQPLNESLQ----YRDRERELSAMMVQSVADFARTGDP--------TPAGETWPLYS
Celeg_ace2        YAFGQPYWRPHLYDQTHLEDEKRLSSIIMQIWANFANTGRT-----------DSFWPQYN
Cae_bri_ace2      YAFGQPYWRPHLYDQKQLEDEKRLSSIIMQVWANFANTGRT-----------DSFWPQYN
```

521

```
Bla_ger_ace1      AYGREYLTLDVN----------------STETGRGPRLKQCAFWKKYLPQLIAVTSNLNQ
Nep_cin_ace1      AYGREYLTLDVN----------------STATGRGPRLKQCAFWKKYLPQLIAATEKLQA
Lip_ent_ace1      PFGREFLTLAVN----------------NTSTGRGPRLKQCAFWKKYLPQLVAVTANLNS
Cim_lec_ace1      AYGREFLTLGINQ--------------SSSTTGRGPRVKQCAFWKKYLPQLIASTETRTN
Bom_man_ace1      AFGREYLSLAVN----------------SSSIGRGLRVKQCAFWQKHLPQLMAATNKPEP
Bom_Mor_ace1      AFGREYLSLAVN----------------SSSVGRGLRVKQCAFWQKHLPQLMAATNKPEP
Chi_sup_ace1      AFGGEYLSLAVN----------------SSAVGHGLKVKQCAFWQKYLPQLMAATTKPEP
Bem_tab_ace1      TYGREFLTLDIN----------------STAIGQGPRLRQCAFWKKYLPQLISTTSYLVP
Api_mel_ace1      AAKKEYMTLDTN----------------SSEIGNGPRVRQCIFWKNYLPQLVAGTSKLEP
Cul_pip           AHGRHYLELGLN----------------TTFVGRGPRLRQCAFWKKYLPQLVAATSNLQV
Cul_qui           AHGRHYLELGLN----------------TTFVGRGPRLRQCAFWKKYLPQLVAATSNLQV
Aed_alb           AHGRHYLELGLN----------------TTYVGRGPRLRQCAFWKKYLPQLVAATSNLQA
Ano_gam           AHGRHYLELGLN----------------TSFVGRGPRLRQCAFWKKYLPQLVAATSNLPG
Cte_fel_ace1      VEHKEYLTLGVN----------------DSELGKGPRLRQCAFWKQFLPQLIIATKNTTA
LS_ace1           PTKQEVLELNAN----------------YSRVLEGLRVKKCAFWKKYLPKLLSLTSNNGD
LS_ace2           PIKQEVLELNAN----------------YSRVFEGLRVRKCAFWKTYLPKLLSLTSNNTK
Tet_urt_ace1      AHEKEYLVISTN----------------DSSIGRGLRAKQCAFWKNFLPKLINALENRHN
Rhi_dec           DSLKRHLVLDVN-----------------ESVGWAHRQTYCDFWENVRRNRTPPVPSC--
Celeg_ace1        SVSMEYMNMTVESS-----------YPSMKRIGHGPRRKECAFWKAYLPNLMAAVADVGD
Cae_bri_ace1      SVSMEYMNMTVESS-----------YPGQNRIGHGPRRKECAFWKAYLPNLMAAVADVGD
Homosap           AGAQQYVSLDLRP----------------LEVRRGLRAQACAFWNRFLPKLLSATDTLDE
Tor_cal           TKEQKFIDLNTEP----------------MKVHQRLRVQMCVFWNQFLPKLLNATACDGE
Bom_man_ace2      RSSPHYYTYTAV-------------GPSGPAGPRGPRASACAFWNDFLNKLN---ELERV
Bom_Mor_ace2      RSSPHYYTYTAV-------------GPSGPAGPRGPRASACAFWNDFLNKLN---ELERA
Chi_sup_ace2      RSSPHYYTYTAD-------------GTSGPAGPRGPRASACAFWNDFLNKLN---ELEHV
Lep_dec           KDQPQYFIFNAD-------------KNG---IGKGPRATACAFWNDFLPKLRDNSGSEEA
Nep_cin_ace2      REQPQYYIFNAE-------------KSG---IGKGPRATACAFWNEFLPRLRGQPDPECL
Cim_lec_ace2      KEQPYYYVFNAE-------------DMG---IGHGPRSTACAFWNDFFPKLKETPGENCG
```

```
Lip_ent_ace2    KEEPKYYIYNAE-------------SMG---TGKGPRSNPCAFWNDFLPKLQGNPRFEDQ
Bla_ger_ace2    KDHPKYYILNSD-------------TYG---TGKGPRTTNCAFWNDFLPKLANHPDPDYA
Api_mel_ace2    RDEPKYFIFDAE-------------KTG---LGKGPRTTYCAFWNEFLPKLKGIPDPXPN
Bem_tab_ace2    RSNPQYFIFHAT-------------TSG---LGSGPRLTACQFWNEFLPKLRNVSENISS
Dros            KEDPVYYIFST--------------DDKIEKLARGPLAARCSFWNDYLPKVRSWAGTCDG
Mus_dom         KEDPVYYVFST--------------DEKIEKLQRGPLAKRCSFWNDYLPKVRSWIGSECE
Cte_fel_ace2    REKPIYYEFNAEGPQPEPSLDIDGSDSDLEHTGRGPRATACAFWNEFLPKLRALSETPPS
Celeg_ace2      KIERKAIELGETTLQG------------KHRIISDVHGGFCRMIDEAKAFVKQKNANDCR
Cae_bri_ace2    KIERKAIELGESTLHG------------NHRIIADVHGSYCRMIDEAKAFVKQKNASDCR

Bla_ger_ace1    QPESCPTDTSGAEMNKESSLLLICLVMKIILLWGPRGWV---------------------
Nep_cin_ace1    PVVETCTGAAGEARVAWSLIAMAFALCVV-------------------------------
Lip_ent_ace1    NNPQPCASSSHKTFDVISFHVFTLIIISKLTHLWILQ-----------------------
Cim_lec_ace1    PVQECTNGVNSLSLSKELLPAAVAFFAALTVYV---------------------------
Bom_man_ace1    PKNCTNSVSSLWPSRKALSFNVIATAALTGTSLFKYTI----------------------
Bom_Mor_ace1    PKNCTNSVPSLWPSRNTLGFNVIATAALTGTALFKYTI----------------------
Chi_sup_ace1    IQNCTSNSGTRHYYGVTSLSLVTVFGFLQPTILKYIII----------------------
Bem_tab_ace1    ANSSNCTSAAPSSLLQHGFNKLSSHSSPFILLLAVFQFLLLLLSNSAIL-----------
Api_mel_ace1    KETCSGTRVNDGGRLLLVLSLVVIGTLFSHRIATRAYDELPEIFDPKGFV----------
Cul_pip         TPAPSVPCESSSTSYRSTLLLIVTLLLVTRFKI---------------------------
Cul_qui         TPAPSVPCESSSTSYRSTLLLIVTLLLVTRFKI---------------------------
Aed_alb         TPAPSEPCGSSAALYRPLLFLIVSLVLVTRFKI---------------------------
Ano_gam         PAPPSEPCESSAFFYRPDLIVLLVSLLTATVRFIQ-------------------------
Cte_fel_ace1    SVRSECVSGVPAPSACGAVIALFLLLCAILHRSPL-------------------------
LS_ace1         HQCQTSSTSSPSCCKDGTCCNSGTSFASTFFSVFIVTTSVHLFYNFKFSVIYSNINNGKL
LS_ace2         SEVVTNPS----------------------------------------------------
Tet_urt_ace1    STCTSHSNQIGSSNWSLAISLISLIMCFLPSLR---------------------------
Rhi_dec         ------------------------------------------------------------
Celeg_ace1      PYLVWKQQMDKWQNEYITDWQYHFEQYKRYQTYRQSDSETCGG-----------------
Cae_bri_ace1    PYLVWKQQMDKWQNEYITDWQYHFEQYKRYQTYRQSDSETCGG-----------------
Homosap         AERQWKAEFHRWSSYMVHWKNQFDHYSKQDRCSDL-------------------------
Tor_cal         LSS---------------------------------------------------------
Bom_man_ace2    PCDGAVTGPYSSVAGTALPVTLLTTLAITIAL----------------------------
Bom_Mor_ace2    PCDGAVTGPYSSVAGTALPVTLLTTLAITIAL----------------------------
Chi_sup_ace2    PCDRAVTGPYSSVAGTTLPILLLTALATSVAL----------------------------
Lep_dec         PCVNTYLSKIRSSSNELLPPSTSLVLIWIMTLLNAL------------------------
Nep_cin_ace2    ADVAEVETSSPLVDNVSDNSTSTTFKPCTVITVLGLLLLTI-------------------
Cim_lec_ace2    CDKSEPILDVELVQNMSMGIMLNRGNSESPWISLVFGLIVALAAV---------------
Lip_ent_ace2    GCNGKAEETFSNPMSRSSTLSKGIWLILLSSLAVILSL----------------------
Bla_ger_ace2    CASGVAETANLGVNSATSCLLVGPKHLLYFVTIVLARLLLSL------------------
Api_mel_ace2    TCKVIASSVSAGEEGLGNSLAITALSSILVLSRVI-------------------------
Bem_tab_ace2    TTDIKSCCSQEMSLGDCNNTALRVSDSGGRAAPEMGFLLLALFPVLLTSIR---------
Dros            DSGSASISPRLQLLGIAALIYICAALRTKRVF----------------------------
Mus_dom         NKSSTSASAAIYEMKMQQLTLLAVAIILTMVNSIFQ------------------------
Cte_fel_ace2    PCEITERIIQIASGCVNFKYLWTWSLLLNLMVINLV------------------------
Celeg_ace2      TTRKSASTEDLTSSSSTTYLFSIIVYLSILISYISL------------------------
Cae_bri_ace2    STRKSDSSSTETSSTANSLYLFIIISLSLLISCISL------------------------
```

Figure 3:

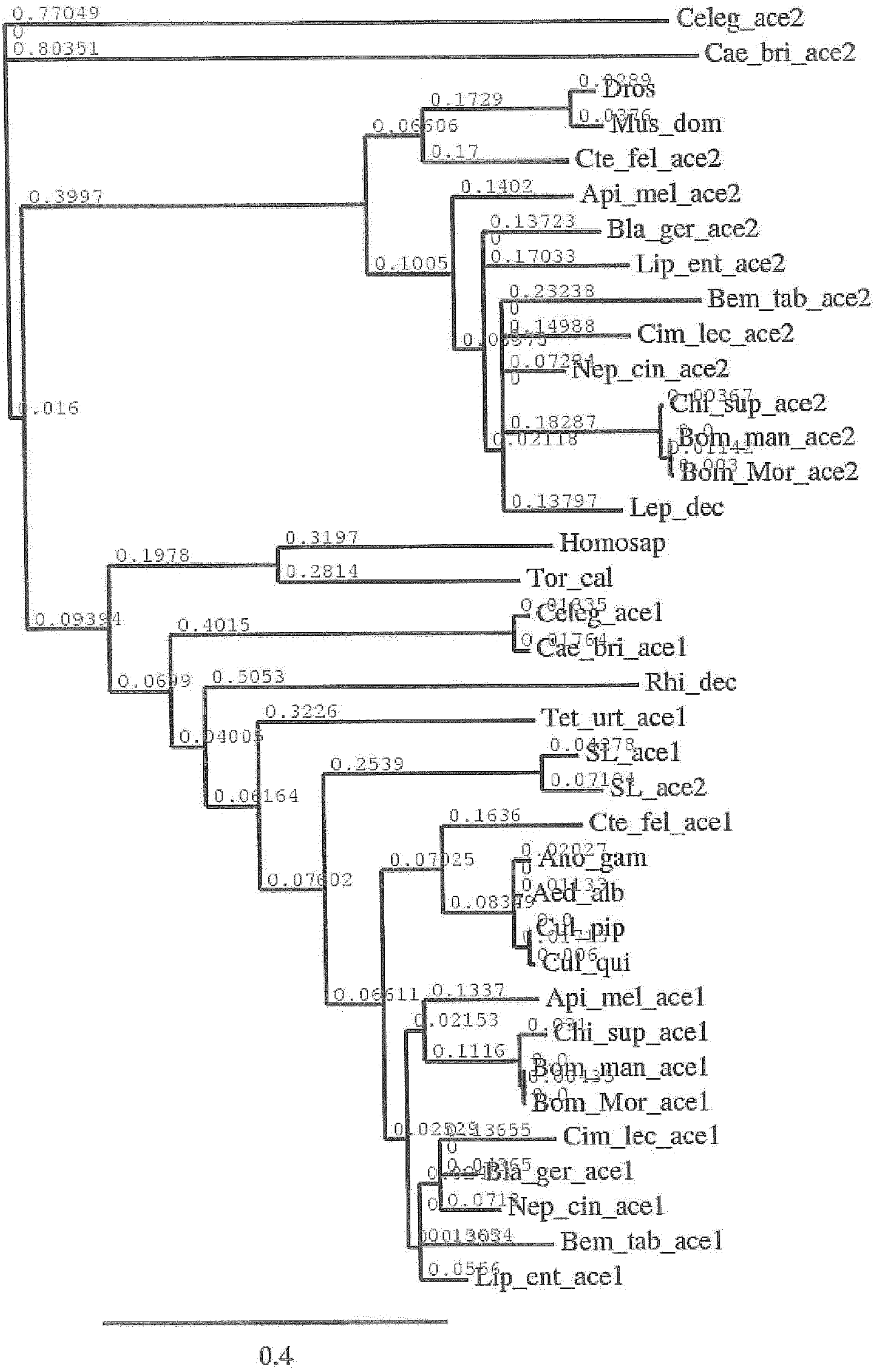
Celeg_ace2
Cae_bri_ace2
Dros
Mus_dom
Cte_fel_ace2
Api_mel_ace2
Bla_ger_ace2
Lip_ent_ace2
Bem_tab_ace2
Cim_lec_ace2
Nep_cin_ace2
Chi_sup_ace2
Bom_man_ace2
Bom_Mor_ace2
Lep_dec
Homosap
Tor_cal
Celeg_ace1
Cae_bri_ace1
Rhi_dec
Tet_urt_ace1
SL_ace1
SL_ace2
Cte_fel_ace1
Ano_gam
Aed_alb
Cul_pip
Cul_qui
Api_mel_ace1
Chi_sup_ace1
Bom_man_ace1
Bom_Mor_ace1
Cim_lec_ace1
Bla_ger_ace1
Nep_cin_ace1
Bem_tab_ace1
Lip_ent_ace1
0.4

## Figure 4:

```
LSAlta     ATGTGGATTCAAGTCAGAAAACAAAACTTTGGTCTTTACTTTAATAAGATATTAGTGTAT
LSHitra    ATGTGGATTCAAGTCAGAAAACAAAACTTTGGTCTTTACTTTAATAAGATATTAGTGTAT

LSAlta     TTGCTCACTTTGTCATGGAGCGTGGGTGCTATCGTACAAGACAATTTGGTGATCACCACA
LSHitra    TTGCTCACTTTGTCATGGAGCGTGGGTGCTATCGTACAAGACAATTTGGTGATCACCACA

LSAlta     AAAAAAGGAAAGATCCGAGGTGTTACTCTTAAATCTGCAACAAATAGGGAAGTAGATGCA
LSHitra    AAAAAAGGAAAGATCCGAGGTGTTACTCTTAAATCTGCAACAAATAGGGAAGTAGATGCA
                                                     Arg80Arg
LSAlta     TGGTATGGGATTCCATACGCACAACCTCCAGTGGGTAATCTTCGATTTCGTCACCCTAGA
LSHitra    TGGTATGGGATTCCATACGCACAACCTCCAGTGGGTAATCTTCGATTTCGTCACCCTAGG

LSAlta     CACATTGATGCCTGGGAGGGGATTAAAGAAACGACCCGACCTCCAAATTCTTGTATTCAA
LSHitra    CACATTGATGCCTGGGAGGGGATTAAAGAAACGACCCGACCTCCAAATTCTTGTATTCAA

LSAlta     GTAGTTGATACATTATTTCCAGGCTTTGAAGGCGCAGAAATGTGGAATGTGAATACAGAG
LSHitra    GTAGTTGATACATTATTTCCAGGCTTTGAAGGCGCAGAAATGTGGAATGTGAATACAGAG

LSAlta     CCCAGCGAGGACTGTCTTTATTTAAGTGTCCATGTCCCTAAACCCCGTCCTACAGGATCA
LSHitra    CCCAGCGAGGACTGTCTTTATTTAAGTGTCCATGTCCCTAAACCCCGTCCTACAGGATCA

LSAlta     GCTGTTCTGGTATGGATCTACGGTGGAGGATTTTATTCTGGAACTTCTACTCTGGAAGTC
LSHitra    GCTGTTCTGGTATGGATCTACGGTGGAGGATTTTATTCTGGAACTTCTACTCTGGAAGTC

LSAlta     TATGATCCACGAGTTCTTGTGTCAGAAGAAAACATAATCTTCGTTGCCATGCAATATCGT
LSHitra    TATGATCCACGAGTTCTTGTGTCAGAAGAAAACATAATCTTCGTTGCCATGCAATATCGT

LSAlta     GTTGCAAGTTTAGGGTTCCTTTTCTTTGATACGGAGGATGTTCCTGGAAATGCGGGATTG
LSHitra    GTTGCAAGTTTAGGGTTCCTTTTCTTTGATACGGAGGATGTTCCTGGAAATGCGGGATTG

LSAlta     TATGATCAAATGATGGCTCTCCAATGGGTAAAGAACAATATAGAGGAATTTGGTGGTGAT
LSHitra    TATGATCAAATGATGGCTCTCCAATGGGTAAAGAACAATATAGAGGAATTTGGTGGTGAT
                                                         Ser235Ser
LSAlta     CCTGATAAAATCACCATTTTCGGAGAGTCCGCCGGTGGTTGCTCCGTAGCCCTTCACCTC
LSHitra    CCTGATAAAATCACCATTTTCGGAGAGTCCGCCGGTGGTTGCTCGGTAGCCCTTCACCTC

LSAlta     CTTTCCCCACTCTCAAGGAACCTGTTTTCTCAAGCCATCATGCAGAGTGCTTCAGCTCTT
LSHitra    CTTTCCCCACTCTCAAGGAACCTGTTTTCTCAAGCCATCATGCAGAGTGCTTCAGCTCTT

LSAlta     GTTCCATGGGGAGTCATCACAAAAAAAGAAAGTATTATTCGTGGTCGGAGGCTTGCGGAA
LSHitra    GTTCCATGGGGAGTCATCACAAAAAAAGAAAGTATTATTCGTGGTCGGAGGCTTGCGGAA

LSAlta     ATGATGAGCTGTCCTTACGATGAAAAAAATACAAAGGCCATGATTGAATGCCTGCGGCAA
LSHitra    ATGATGAGCTGTCCTTACGATGAAAAAAATACAAAGGCCATGATTGAATGCCTGCGGCAA

LSAlta     AAGGATGCAACAGAGATGGTTAATCAGGAGTGGATTGGTATCATTTCTGGGATTGCAGAG
LSHitra    AAGGATGCAACAGAGATGGTTAATCAGGAGTGGATTGGTATCATTTCTGGGATTGCAGAG

LSAlta     TTCCCTTTTGTTCCTATTGTGGATGGAAGTTTCTTGGATGAGAGCCCTGGAAAATCTCTT
```

```
LSHitra        TTCCCTTTTGTTCCTATTGTGGATGGAAGTTTCTTGGATGAGAGCCCTGGAAAATCTCTT

LSAlta         ACAACAAAGAACTATAAAAAAACCAACATTTTAATTGGAGCGAATAAGGAAGAAGGGAAT
LSHitra        ACAACAAAGAACTATAAAAAAACCAACATTTTAATTGGAGCGAATAAGGAAGAAGGGAAT

                    Phe362Tyr
LSAlta         TATTTCATCATGTACTATCTTACAGATCTCTTTAAAAATACGGAGAGCGTCTATGTTGAT
LSHitra        TATTACATCATGTACTATCTTACAGATCTCTTTAAAAATACGGAGAGCGTCTATGTTGAT

LSAlta         CGAACAGATTTCATTCGAAGTGTTTCAGAATTGAACCACTATGTAAAAAAAATGGGAAGA
LSHitra        CGAACAGATTTCATTCGAAGTGTTTCAGAATTGAACCACTATGTAAAAAAAATGGGAAGA

LSAlta         GAGGCAATAACTTTTGAGTACACAGATTGGCTCAATCCAAACGATCCCATAAAAAATAGA
LSHitra        GAGGCAATAACTTTTGAGTACACAGATTGGCTCAATCCAAACGATCCCATAAAAAATAGA

LSAlta         GAAGCAATTGATCGCATGGTCGGTGACTATCAATTCATTTGCCCAACTGCTGACTTTGCT
LSHitra        GAAGCAATTGATCGCATGGTCGGTGACTATCAATTCATTTGCCCAACTGCTGACTTTGCT

LSAlta         CGTATTTATGCTAGTACAGGAAATAATGTATACATGTACTATTTCACTGAGCGATCTTCC
LSHitra        CGTATTTATGCTAGTACAGGAAATAATGTATACATGTACTATTTCACTGAGCGATCTTCC

LSAlta         ACTAGCCCATGGCCAACATGGTCTGGTGTACTTCATGGCGATGAAATTGCTTTTGTTTTT
LSHitra        ACTAGCCCATGGCCAACATGGTCTGGTGTACTTCATGGCGATGAAATTGCTTTTGTTTTT

LSAlta         GGAGAGGCCCTTAATAAGTCAAAGAATTATGATAAGTCAGAAATTGCCCTATCAAAAAGA
LSHitra        GGAGAGGCCCTTAATAAGTCAAAGAATTATGATAAGTCAGAAATTGCCCTATCAAAAAGA

LSAlta         ATGATGGGATATTGGGCTAATTTTGCTAAAACTGGGAATCCGAGTCTTTCTGCTGATGGA
LSHitra        ATGATGGGATATTGGGCTAATTTTGCTAAAACTGGGAATCCGAGTCTTTCTGCTGATGGA

LSAlta         ACTTGGAGCACAAATTATTGGCCTTTACATACGCCGACAAAACAGGAGGTACTTGAATTA
LSHitra        ACTTGGAGCACAAATTATTGGCCTTTACATACGCCGACAAAACAGGAGGTACTTGAATTA

LSAlta         AACGCCAATTATTCTCGAGTTCTCGAGGGTCTTCGAGTTAAAAAATGTGCCTTTTGGAAA
LSHitra        AACGCCAATTATTCTCGAGTTCTCGAGGGTCTTCGAGTTAAAAAATGTGCCTTTTGGAAA

LSAlta         AAATATCTACCTAAACTTTTATCATTAACTTCAAACAATGGAGACCATCAGTGTCAAACA
LSHitra        AAATATCTACCTAAACTTTTATCATTAACTTCAAACAATGGAGACCATCAGTGTCAAACA

LSAlta         TCCTCGACATCATCTCCCTCATGCTGTAAAGATGGAACATGCTGCAACAGTGGGACGTCC
LSHitra        TCCTCGACATCATCTCCCTCATGCTGTAAAGATGGAACATGCTGCAACAGTGGGACGTCC

LSAlta         TTTGCATCCACGTTCTTTTCAGTATTCATTGTAACAACGTCGGTGCATCTATTTTATAAT
LSHitra        TTTGCATCCACGTTCTTTTCAGTATTCATTGTAACAACGTCGGTGCATCTATTTTATAAT

LSAlta         TTTAAATTTAGTGTAATTTACTCAAATATTAATAATGGAAAACTGTGA
LSHitra        TTTAAATTTAGTGTAATTTACTCAAATATTAATAATGGAAAACTGTGA
```

**Figure 5:**

```
LSAlta     ATGTGGATTCAAGTCCGAAAACACAACTTAGGTCTTTCCTTTGAAAGGATATTAGTGTAT
LSHitra    ATGTGGATTCAAGTCCGAAAACACAACTTAGGTCTTTCCTTTGAAAGGATATTAGTGTAT

LSAlta     TTACTCACATTGTCATGGAGTCTGGGATCCATCGTACAAGAAGATTTGGTGATCACCACA
LSHitra    TTACTCACATTGTCATGGAGTCTGGGATCCATCGTACAAGAAGATTTGGTGATCACCACA

LSAlta     AGAAAAGGAAAGATCCGAGGTGTTACTCTGAAATCTGCAACAAATAAGGAAGTAGATGCA
LSHitra    AGAAAAGGAAAGATCCGAGGTGTTACTCTGAAATCTGCAACAAATAAGGAAGTAGATGCA

LSAlta     TGGTATGGGATACCATACGCACAACCTCCCGTGGGTAATCTTCGATTTCGTCACCCCAAA
LSHitra    TGGTATGGGATACCATACGCACAACCTCCCGTGGGTAATCTTCGATTTCGTCACCCCAAA

LSAlta     GACATTAATGCCTGGGATGGGATGAAAGAAACGACCAAACATCCAAATTCTTGTATTCAA
LSHitra    GACATTAATGCCTGGGATGGGATGAAAGAAACGACCAAACATCCAAATTCTTGTATTCAA

LSAlta     GTAGTTGATACATTTTTTCCGGGCTTTGAAGGCTCAGAGATGTGGAATACAAATACTGAG
LSHitra    GTAGTTGATACATTTTTTCCGGGCTTTGAAGGCTCAGAGATGTGGAATACAAATACTGAG

LSAlta     CAAAGCGAGGACTGCCTTTACTTAAGTGTTCATGCCCCTAAACCCCGTCCTACAAAATCA
LSHitra    CAAAGCGAGGACTGCCTTTACTTAAGTGTTCATGCCCCTAAACCCCGTCCTACAAAATCA

LSAlta     GCTGTTCTGGTATGGATCTACGGTGGAGGATTTTATTCTGGAACTTCAACTCTGGAACTC
LSHitra    GCTGTTCTGGTATGGATCTACGGTGGAGGATTTTATTCTGGAACTTCAACTCTGGAACTC

LSAlta     TATGATCCACGAGTTCTTGTGTCAGAAGAAAACATAATCTTCGTCGGCATACAATATCGT
LSHitra    TATGATCCACGAGTTCTTGTGTCAGAAGAAAACATAATCTTCGTCGGCATACAATATCGT

LSAlta     GTTGCAAGTTTAGGATTCTTATTCTTTGATACGGAGGATGTTCCTGGAAATGCGGGATTG
LSHitra    GTTGCAAGTTTAGGATTCTTATTCTTTGATACGGAGGATGTTCCTGGAAATGCGGGATTG

LSAlta     TATGATCAAATGATGGCTCTCCAATGGGTAAAGAACAATATAGAGGCATTTGGTGGTGAT
LSHitra    TATGATCAAATGATGGCTCTCCAATGGGTAAAGAACAATATAGAGGCATTTGGTGGTGAT

LSAlta     CCTGATAAAATCACCATTTTTGGAGAGTCCGCCGGTGGTTGCTCCGTAGCCCTTCATCTC
LSHitra    CCTGATAAAATCACCATTTTTGGAGAGTCCGCCGGTGGTTGCTCCGTAGCCCTTCATCTC

LSAlta     CTTTCTCCACTCTCAAGGAACCTATTCTCTCAAGCCATTATGCAAAGTTCTTCAGCTCTT
LSHitra    CTTTCTCCACTCTCAAGGAACCTATTCTCTCAAGCCATTATGCAAAGTTCTTCAGCTCTT

LSAlta     GTTCCATGGGGAGTCATATCAAAAAAAGAAAGTATCCGTCGTGGTCGAAGACTTGCAGAA
LSHitra    GTTCCATGGGGAGTCATATCAAAAAAAGAAAGTATCCGTCGTGGTCGAAGACTTGCAGAA

LSAlta     GAGATGCGTTGTCCTTATGGTGAAAATAATACCAATGCTATGATTGAATGCCTGCTGCAA
LSHitra    GAGATGCGTTGTCCTTATGGTGAAAATAATACCAATGCTATGATTGAATGCCTGCTGCAA

LSAlta     AAGGACGCAACAGAGTTGGTCAACCAGGAGTGGAGTGGTACCGTCTTTGGGATTTCAGAG
LSHitra    AAGGACGCAACAGAGTTGGTCAACCAGGAGTGGAGTGGTACCGTCTTTGGGATTTCAGAG

LSAlta     TTCCCATTTGTTCCAATTGTGGATGGAAAATTCATGGATAAAACCCCTGAAAAATCTCTT
LSHitra    TTCCCATTTGTTCCAATTGTGGATGGAAAATTCATGGATAAAACCCCTGAAAAATCTCTT
```

```
LSAlta      AAAGAAAAGGACTATAAAAAAACCAACATTTTAATGGGAGTCAATAAGGACGAAGGGAAC
LSHitra     AAAGAAAAGGACTATAAAAAAACCAACATTTTAATGGGAGTCAATAAGGACGAAGGGAAC

LSAlta      TTTTTCATCATGTATTATCTTCCAGAACTCTTCAAAAAAAACGAAAACGTTTATATTAAC
LSHitra     TTTTTCATCATGTATTATCTTCCAGAACTCTTCAAAAAAAACGAAAACGTTTATATTAAC

LSAlta      CGAACAGATTTCATCCGCAGTGTTTCAGATTTGAACATCTATGTGAACAATGCAGGAAGA
LSHitra     CGAACAGATTTCATCCGCAGTGTTTCAGATTTGAACATCTATGTGAACAATGCAGGAAGA

LSAlta      GAGGCAATAACTTTTGAATACACAGATTGGCTCAATCCAAACGATCCCATAAAAAATAGA
LSHitra     GAGGCAATAACTTTTGAATACACAGATTGGCTCAATCCAAACGATCCCATAAAAAATAGA

                                                          Iso433Thr
LSAlta      GAAGCAATTGATCGCATGGTCGGTGACTATCAATTCATTTGCCCAACTGCTGACTTTGCT
LSHitra     GAAGCAATTGATCGCATGGTCGGTGACTATCAATTCACTTGCCCAACTGCTGACTTTGCT

LSAlta      CGTATTTATGCTAGTACAGGAAATAATATATACATGTACTATTTCACTGAGCGATCTTCC
LSHitra     CGTATTTATGCTAGTACAGGAAATAATATATACATGTACTATTTCACTGAGCGATCTTCC

LSAlta      ACTAGCCCATGGCCAACATGGTCTGGTGTACTTCATGGCGATGAAATTGCTTTTGTTTTT
LSHitra     ACTAGCCCATGGCCAACATGGTCTGGTGTACTTCATGGCGATGAAATTGCTTTTGTTTTT

LSAlta      GGAGAGCCCCTAAATACGTCAAAAAATTATGATGATTCAGAAATTGCCTTATCAAAAAGA
LSHitra     GGAGAGCCCCTAAATACGTCAAAAAATTATGATGATTCAGAAATTGCCTTATCAAAAAGA

LSAlta      ATAATGAGCTATTGGGCTAATTTTGCAAAAACTGGGAACCCGAATGTTTTGGCTAATGGA
LSHitra     ATAATGAGCTATTGGGCTAATTTTGCAAAAACTGGGAACCCGAATGTTTTGGCTAATGGA

LSAlta      AACTACAGCAACAAAATCTGGCCCTTACATACACCAATAAAACAGGAGGTACTTGAACTA
LSHitra     AACTACAGCAACAAAATCTGGCCCTTACATACACCAATAAAACAGGAGGTACTTGAACTA

LSAlta      AATGCAAATTATTCTCGAGTTTTTGAGGGGCTTCGAGTTAGAAAATGTGCTTTTTGGAAA
LSHitra     AATGCAAATTATTCTCGAGTTTTTGAGGGGCTTCGAGTTAGAAAATGTGCTTTTTGGAAA

LSAlta      ACATATCTTCCTAAGCTTTTATCATTAACTTCAAACAATACAAAGTCTGAAGTTGTAACC
LSHitra     ACATATCTTCCTAAGCTTTTATCATTAACTTCAAACAATACAAAGTCTGAAGTTGTAACC

LSAlta      AATCCGTCATAA
LSHitra     AATCCGTCATAA
```

Figure 6:

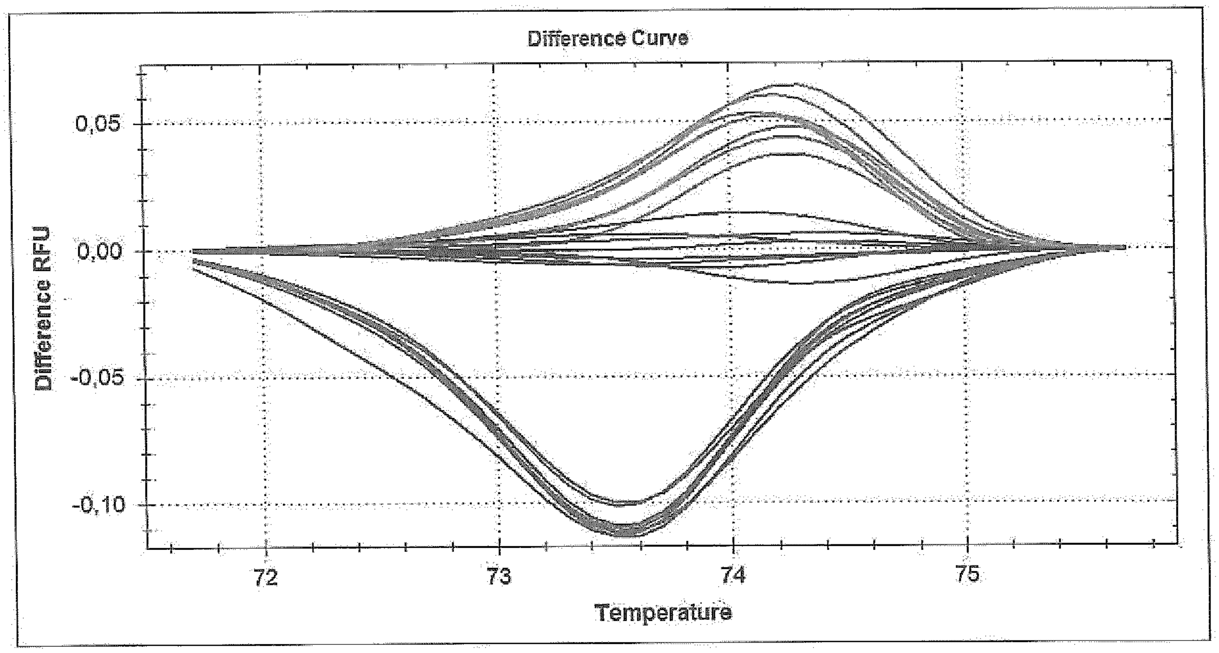
Difference Curve
0,05
0,00
-0,05
-0,10
Difference RFU
72
73
74
75
Temperature

Figure 7:

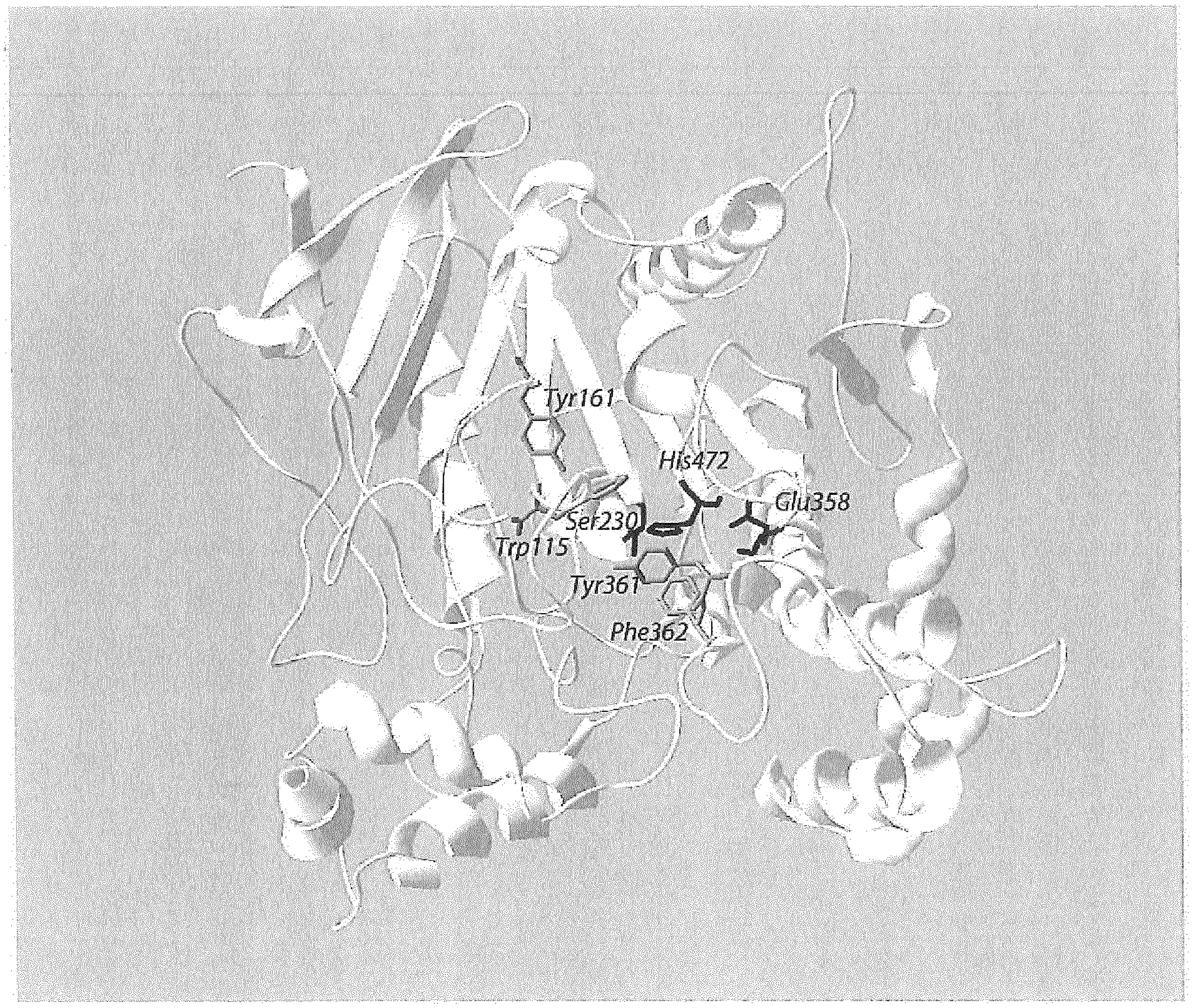

Figure 8:

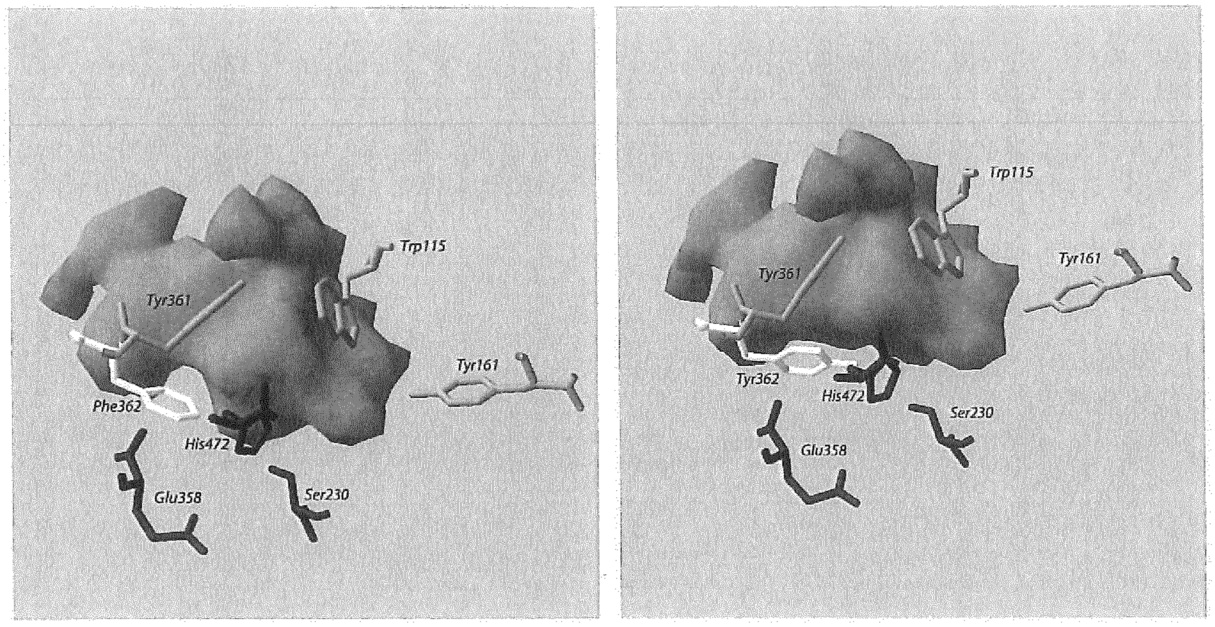


Figure 9:

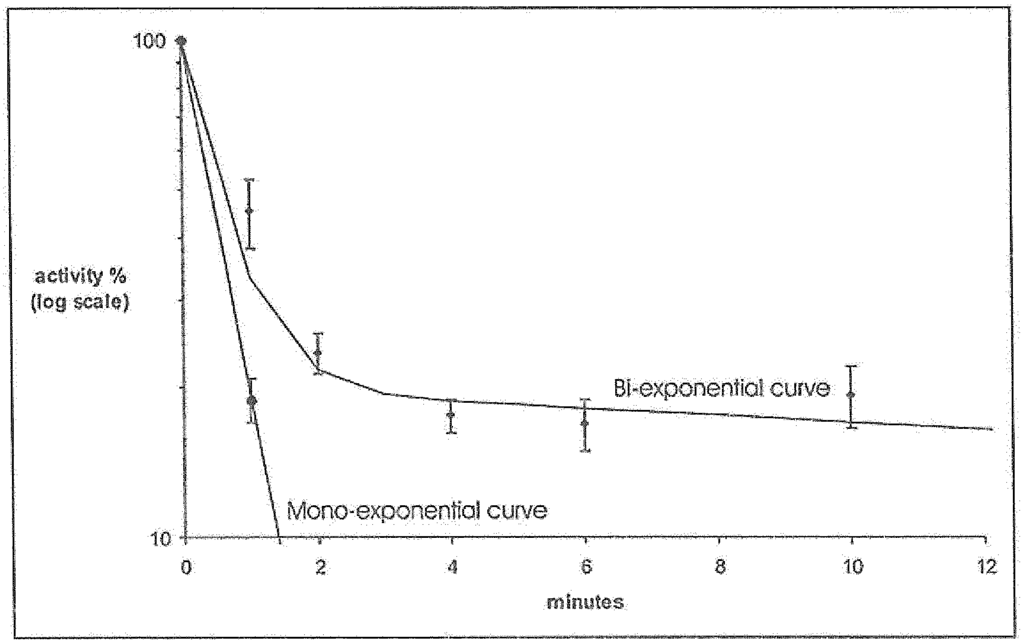

Figure 10

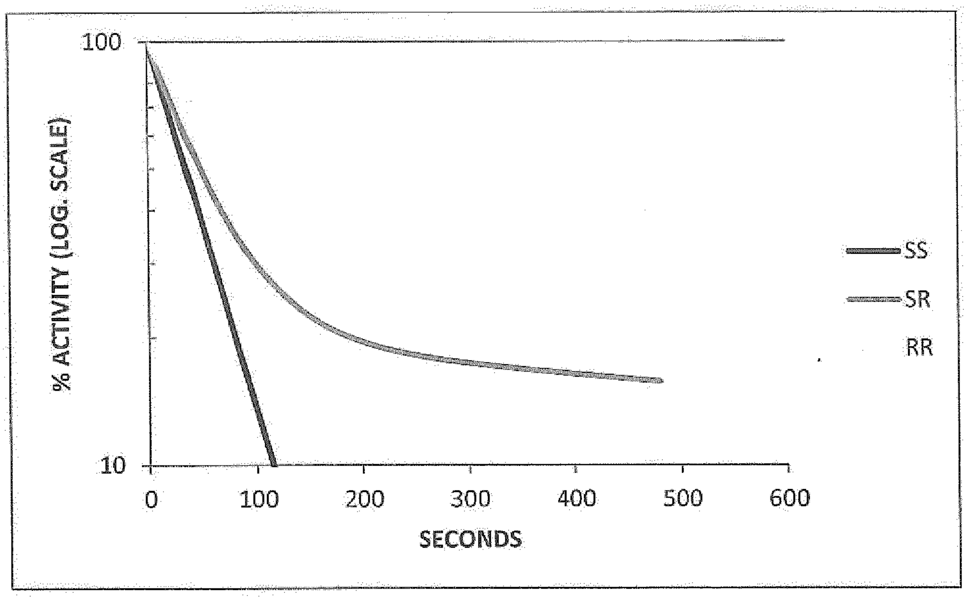


Figure 11

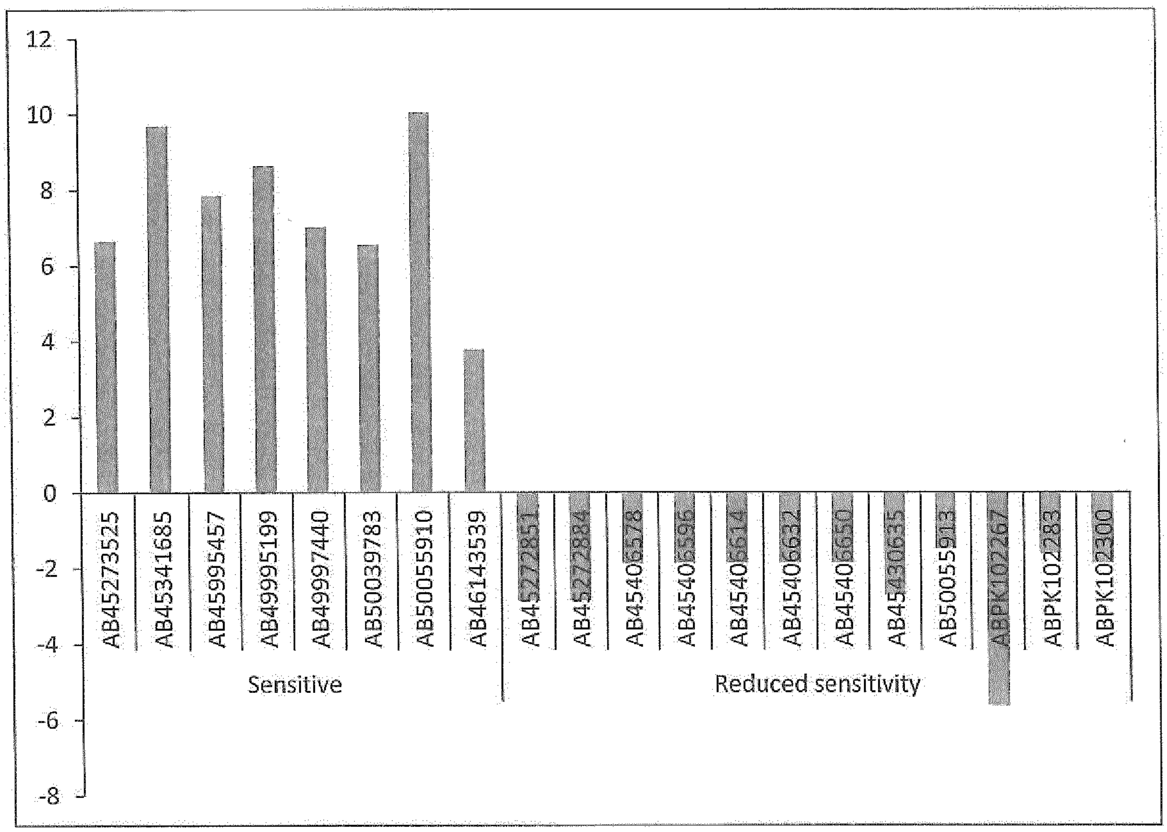

**Figure 12:**

| | |
|---|---|
| 0 - 60 | ATGTGGATTCAAGTCAGAAAACAAAACTTTGGTCTTTACTTTAATAAGATATTAGTGTAT |
| 61-120 | TTGCTCACTTTGTCATGGAGCGTGGGTGCTATCGTACAAGACAATTTGGTGATCACCACA |
| 121-180 | AAAAAAGGAAAGATCCGAGGTGTTACTCTTAAATCTGCAACAAATAGGGAAGTAGATGCA |
| 181-240 | TGGTATGGGATTCCATACGCACAACCTCCAGTGGGTAATCTTCGATTTCGTCACCCTAGA |
| 241-300 | CACATTGATGCCTGGGAGGGGATTAAAGAAACGACCCGACCTCCAAATTCTTGTATTCAA |
| 301-360 | GTAGTTGATACATTATTTCCAGGCTTTGAAGGCGCAGAAATGTGGAATGTGAATACAGAG |
| 361-420 | CCCAGCGAGGACTGTCTTTATTTAAGTGTCCATGTCCCTAAACCCCGTCCTACAGGATCA |
| 421-480 | GCTGTTCTGGTATGGATCTACGGTGGAGGATTTTATTCTGGAACTTCTACTCTGGAAGTC |
| 481-540 | TATGATCCACGAGTTCTTGTGTCAGAAGAAAACATAATCTTCGTTGCCATGCAATATCGT |
| 541-600 | GTTGCAAGTTTAGGGTTCCTTTTCTTTGATACGGAGGATGTTCCTGGAAATGCGGGATTG |
| 601-661 | TATGATCAAATGATGGCTCTCCAATGGGTAAAGAACAATATAGAGGAATTTGGTGGTGAT |
| 661-720 | CCTGATAAAATCACCATTTTCGGAGAGTCCGCCGGTGGTTGCTCCGTAGCCCTTCACCTC |
| 721-780 | CTTTCCCCACTCTCAAGGAACCTGTTTTCTCAAGCCATCATGCAGAGTGCTTCAGCTCTT |
| 781-840 | GTTCCATGGGGAGTCATCACAAAAAAAGAAAGTATTATTCGTGGTCGGAGGCTTGCGGAA |
| 841-900 | ATGATGAGCTGTCCTTACGATGAAAAAAATACAAAGGCCATGATTGAATGCCTGCGGCAA |
| 901-960 | AAGGATGCAACAGAGATGGTTAATCAGGAGTGGATTGGTATCATTTCTGGGATTGCAGAG |
| 961-1020 | TTCCCTTTTGTTCCTATTGTGGATGGAAGTTTCTTGGATGAGAGCCCTGGAAAATCTCTT |
| 1021-1080 | ACAACAAAGAACTATAAAAAAACCAAC**ATTTTAATTGGAGCGAATAAGGAA**G**AAGGGAAT** |
| 1081-1140 | ***TATTTCATCATG***TACTATCTTAC**AGATCTCTTTAAAAATACGGAGAGCG**TCTATGTTGAT |
| 1141-1200 | CGAACAGATTTCATTCGAAGTGTTTCAGAATTGAACCACTATGTAAAAAAAATGGGAAGA |
| 1201-1260 | GAGGCAATAACTTTTGAGTACACAGATTGGCTCAATCCAAACGATCCCATAAAAAATAGA |
| 1261-1320 | GAAGCAATTGATCGCATGGTCGGTGACTATCAATTCATTTGCCCAACTGCTGACTTTGCT |
| 1321-1380 | CGTATTTATGCTAGTACAGGAAATAATGTATACATGTACTATTTCACTGAGCGATCTTCC |
| 1381-1440 | ACTAGCCCATGGCCAACATGGTCTGGTGTACTTCATGGCGATGAAATTGCTTTTGTTTTT |
| 1441-1500 | GGAGAGGCCCTTAATAAGTCAAAGAATTATGATAAGTCAGAAATTGCCCTATCAAAAAGA |
| 1501-1560 | ATGATGGGATATTGGGCTAATTTTGCTAAAACTGGGAATCCGAGTCTTTCTGCTGATGGA |
| 1561-1620 | ACTTGGAGCACAAATTATTGGCCTTTACATACGCCGACAAAACAGGAGGTACTTGAATTA |
| 1621-1680 | AACGCCAATTATTCTCGAGTTCTCGAGGGTCTTCGAGTTAAAAAATGTGCCTTTTGGAAA |
| 1681-1740 | AAATATCTACCTAAACTTTTATCATTAACTTCAAACAATGGAGACCATCAGTGTCAAACA |
| 1741-1800 | TCCTCGACATCATCTCCCTCATGCTGTAAAGATGGAACATGCTGCAACAGTGGGACGTCC |
| 1801-1860 | TTTGCATCCACGTTCTTTTCAGTATTCATTGTAACAACGTCGGTGCATCTATTTTATAAT |
| 1861-1908 | TTTAAATTTAGTGTAATTTACTCAAATATTAATAATGGAAAACTGTGA |

## Figure 13

| | |
|---|---|
| 0 - 60 | ATGTGGATTCAAGTCAGAAAACAAAACTTTGGTCTTTACTTTAATAAGATATTAGTGTAT |
| 61-120 | TTGCTCACTTTGTCATGGAGCGTGGGTGCTATCGTACAAGACAATTTGGTGATCACCACA |
| 121-180 | AAAAAAGGAAAGATCCGAGGTGTTACTCTTAAATCTGCAACAAATAGGGAAGTAGATGCA |
| 181-240 | TGGTATGGGATTCCATACGCACAACCTCCAGTGGGTAATCTTCGATTTCGTCACCCTAGG |
| 241-300 | CACATTGATGCCTGGGAGGGGATTAAAGAAACGACCCGACCTCCAAATTCTTGTATTCAA |
| 301-360 | GTAGTTGATACATTATTTCCAGGCTTTGAAGGCGCAGAAATGTGGAATGTGAATACAGAG |
| 361-420 | CCCAGCGAGGACTGTCTTTATTTAAGTGTCCATGTCCCTAAACCCCGTCCTACAGGATCA |
| 421-480 | GCTGTTCTGGTATGGATCTACGGTGGAGGATTTTATTCTGGAACTTCTACTCTGGAAGTC |
| 481-540 | TATGATCCACGAGTTCTTGTGTCAGAAGAAAACATAATCTTCGTTGCCATGCAATATCGT |
| 541-600 | GTTGCAAGTTTAGGGTTCCTTTTCTTTGATACGGAGGATGTTCCTGGAAATGCGGGATTG |
| 601-661 | TATGATCAAATGATGGCTCTCCAATGGGTAAAGAACAATATAGAGGAATTTGGTGGTGAT |
| 661-720 | CCTGATAAAATCACCATTTTCGGAGAGTCCGCCGGTGGTTGCTCGGTAGCCCTTCACCTC |
| 721-780 | CTTTCCCCACTCTCAAGGAACCTGTTTTCTCAAGCCATCATGCAGAGTGCTTCAGCTCTT |
| 781-840 | GTTCCATGGGGAGTCATCACAAAAAAAGAAAGTATTATTCGTGGTCGGAGGCTTGCGGAA |
| 841-900 | ATGATGAGCTGTCCTTACGATGAAAAAAATACAAAGGCCATGATTGAATGCCTGCGGCAA |
| 901-960 | AAGGATGCAACAGAGATGGTTAATCAGGAGTGGATTGGTATCATTTCTGGGATTGCAGAG |
| 961-1020 | TTCCCTTTTGTTCCTATTGTGGATGGAAGTTTCTTGGATGAGAGCCCTGGAAAATCTCTT |
| 1021-1080 | ACAACAAAGAACTATAAAAAAACCAAC**ATTTTAATTGGAGCGAATAAGGAA**G**AAGGGAAT** |
| 1081-1140 | ***TATTACATCATG***TACTATCTTAC**AGATCTCTTTAAAAATACGGAGAGCG**TCTATGTTGAT |
| 1141-1200 | CGAACAGATTTCATTCGAAGTGTTTCAGAATTGAACCACTATGTAAAAAAAATGGGAAGA |
| 1201-1260 | GAGGCAATAACTTTTGAGTACACAGATTGGCTCAATCCAAACGATCCCATAAAAAATAGA |
| 1261-1320 | GAAGCAATTGATCGCATGGTCGGTGACTATCAATTCATTTGCCCAACTGCTGACTTTGCT |
| 1321-1380 | CGTATTTATGCTAGTACAGGAAATAATGTATACATGTACTATTTCACTGAGCGATCTTCC |
| 1381-1440 | ACTAGCCCATGGCCAACATGGTCTGGTGTACTTCATGGCGATGAAATTGCTTTTGTTTTT |
| 1441-1500 | GGAGAGGCCCTTAATAAGTCAAAGAATTATGATAAGTCAGAAATTGCCCTATCAAAAAGA |
| 1501-1560 | ATGATGGGATATTGGGCTAATTTTGCTAAAACTGGGAATCCGAGTCTTTCTGCTGATGGA |
| 1561-1620 | ACTTGGAGCACAAATTATTGGCCTTTACATACGCCGACAAAACAGGAGGTACTTGAATTA |
| 1621-1680 | AACGCCAATTATTCTCGAGTTCTCGAGGGTCTTCGAGTTAAAAAATGTGCCTTTTGGAAA |
| 1681-1740 | AAATATCTACCTAAACTTTTATCATTAACTTCAAACAATGGAGACCATCAGTGTCAAACA |
| 1741-1800 | TCCTCGACATCATCTCCCTCATGCTGTAAAGATGGAACATGCTGCAACAGTGGGACGTCC |
| 1801-1860 | TTTGCATCCACGTTCTTTTCAGTATTCATTGTAACAACGTCGGTGCATCTATTTTATAAT |
| 1861-1908 | TTTAAATTTAGTGTAATTTACTCAAATATTAATAATGGAAAACTGTGA |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Journal of Fish Diseases,* vol. 32, 59-73
- **FOURNIER D et al.** Drosophila acetylcholinesterase: Mechanisms of resistance to organophosphates. *CHEMICO-BIOLOGICAL INTEGRATIONS,* 1993, vol. 87 (1-3), 233-238
- **SAMBROOK et al.** Molecular Cloning: A laboratory Manual. CSHL Press, 2001
- **YOU Y. ; MOREIRA B.G. ; BEHLKE M.A. ; OWCZARZY R.** Design of LNA probes that improve mismatch discrimination. *Nucleic Acids Res.,* 2006, vol. 34 (8), e60
- **ALTSCHUL et al.** *J. of Molec. Biol.,* 1990, vol. 215, 403-410
- **ALTSCHUL et al.** *Nuc. Acids Res.,* 1997, vol. 25, 3389-3402
- **KARLIN ; ALTSCHUL.** *Proc. Nat'l Acad. Sci. USA,* 1990, vol. 87, 2264-68
- *Proc. Nat'l Acad. Sci. USA,* 1993, vol. 90, 5873-77
- **OLIVER ; 2005.** *Mutat. Res.,* vol. 573 (1-2), 103-110
- *CHEMICAL ABSTRACTS,* 35575-96-3
- **SEVATDAL S ; FALLANG A ; INGEBRIGTSEN K ; HORSBERG TE.** Monooxygenase mediated organophosphate detoxification in sea lice (Lepeophtheirus salmonis). *Pest Manag Sci.,* August 2005, vol. 61 (8), 772-8
- **DU Y ; NOMURA Y ; SATAR G ; HU Z ; NAUEN R ; HE SY ; ZHOROV BS ; DONG K.** Molecular evidence for dual organophosphate-receptor sites on a mosquito sodium channel. *Proc Natl Acad Sci USA.,* 02 July 2013
- **ZHU F ; GUJAR H ; GORDON JR ; HAYNES KF ; POTTER MF ; PALLI SR.** Bed bugs evolved unique adaptive strategy to resist organophosphate insecticides. *Sci Rep.,* 2013, vol. 3, 1456
- **ARNOLD et al.** *Bioinformatics,* 2006, vol. 22, 195-201
- **BOUBLIK et al.** *Protein engeneering,* 2002, vol. 15, 43-50
- **COSTELLO M. J.** *Journal of Fish Diseases,* 2009, vol. 32, 115-118
- **DENHOLM et al.** *Pest Manag Sci,* 2002, vol. 58, 528-536
- **FALLANG et al.** *Pest Manag Sci,* 2004, vol. 60, 1163-1170
- **HAREL et al.** *Protein Science,* 2000, vol. 9, 1063-1072
- **HELGESEN et al.** *J Fish Dis,* 2013, vol. 36, 261-272
- **JIANG et al.** *Biochem Biophys Res Comm,* 2009, vol. 378, 269-272
- **KIM et al.** *Insect Mol Biol,* 2006, vol. 15, 513-522
- **NABESHIMA et al.** *Biochem Biophys Res Comm,* 2004, vol. 313, 794-801
- **ORDENTLICH et al.** *J Biol Chem,* 1993, vol. 268, 17083-95
- **PEZZEMENTI et al.** *Comp Biochem Physiol B,* 2003, vol. 136, 813-832
- **PIKE A. ; WADSWORTH S. L.** *Academic Press,* 2000, vol. 44, 232-337
- **SEONG et al.** *Insect Mol Biol,* 2012, vol. 21, 149-159
- **SEVATDAL S. et al.** *Aquaculture,* 2005, vol. 244, 19-27
- **TORRISSEN et al.** *J Fish Dis,* 2013, vol. 36, 171-194
- **VELLOM et al.** *Biochemistry,* 1993, vol. 32, 12-17
- **WEILL et al.** *Proc Roy Soc B Biol Sci,* 2002, vol. 269, 2007-2016